(19) Europäisches Patentamt
European Patent Office
Office européen des brevets

(11) EP 2 215 471 B1

(12) **EUROPEAN PATENT SPECIFICATION**

(45) Date of publication and mention of the grant of the patent:
**08.02.2012 Bulletin 2012/06**

(21) Application number: **08845473.1**

(22) Date of filing: **29.10.2008**

(51) Int Cl.:
***G01N 33/50*** *(2006.01)* ***C12Q 1/68*** *(2006.01)*

(86) International application number:
**PCT/US2008/081646**

(87) International publication number:
**WO 2009/058908 (07.05.2009 Gazette 2009/19)**

(54) **METHODS FOR PROGNOSING THE ABILITY OF A ZEARALENONE ANALOG COMPOUND TO TREAT CANCER**

VERFAHREN ZUR VORHERSAGE DER FÄHIGKEIT EINER ZEARALENON-ANALOGVERBINDUNG FÜR DIE KREBSBEHANDLUNG

PROCÉDÉS DE PRONOSTIC DE L'APTITUDE À TRAITER LE CANCER D'UN COMPOSÉ ANALOGUE DE LA ZÉARALÉNONE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.10.2007 US 796 P**

(43) Date of publication of application:
**11.08.2010 Bulletin 2010/32**

(73) Proprietor: **Eisai R&D Management Co., Ltd.**
**Tokyo 112-8088 (JP)**

(72) Inventors:
- **WANG, John, (yuan)**
  **Andover, MA 01810 (US)**
- **AGOULNIK, Sergei**
  **Andover, MA 01810 (US)**
- **NOMOTO, Kenichi**
  **Belmont, MA 02478 (US)**

(74) Representative: **Cornish, Kristina Victoria Joy et al**
**Kilburn & Strode LLP**
**20 Red Lion Street**
**London**
**WC1R 4PJ (GB)**

(56) References cited:
**WO-A-03/076424 WO-A-2006/036941**

- **SCHIRMER ANDREAS ET AL: "Targeted covalent inactivation of protein kinases by resorcylic acid lactone polyketides" PROCEEDINGS OF THE NATIONAL ACADEMY OF SCIENCES OF THE UNITED STATES OF AMERICA 14 MAR 2006,, vol. 103, no. 11, 14 March 2006 (2006-03-14), pages 4234-4239, XP002508855**
- **VRANIC ET AL: "PIK3CA and PTEN mutations in adenoid cystic carcinoma of the breast metastatic to kidney" HUMAN PATHOLOGY, SAUNDERS, PHILADELPHIA, PA, US, vol. 38, no. 9, 15 August 2007 (2007-08-15), pages 1425-1431, XP022271252 ISSN: 0046-8177**
- **VANESSA C GRAY-SCHOPFER ET AL: "The role of B-RAF in melanoma" CANCER AND METASTASIS REVIEWS, KLUWER ACADEMIC PUBLISHERS, DO, vol. 24, no. 1, 1 January 2005 (2005-01-01), pages 165-183, XP019205175 ISSN: 1573-7233**
- **ROBERTS P J ET AL: "Targeting the Raf-MEK-ERK mitogen-activated protein kinase cascade for the treatment of cancer" ONCOGENE,, vol. 26, no. 22, 1 May 2007 (2007-05-01), pages 3291-3310, XP002508857**
- **DAVIES H ET AL: "MUTATIONS OF THE BRAF GENE IN HUMAN CANCER" NATURE, NATURE PUBLISHING GROUP, LONDON, UK, vol. 417, no. 6892, 27 June 2002 (2002-06-27), pages 949-954, XP001188240 ISSN: 0028-0836 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 215 471 B1

- ANDERSSON P ET AL: "PIK3CA, HRAS and KRAS Gene Mutations in Human Penile Cancer" JOURNAL OF UROLOGY, BALTIMORE, MD, US, vol. 179, no. 5, 1 May 2008 (2008-05-01), pages 2030-2034, XP022594517 ISSN: 0022-5347 [retrieved on 2008-03-19]
- WANG JOHN (YUAN) ET AL: "Recent advances of MEK inhibitors and their clinical progress" CURRENT TOPICS IN MEDICINAL CHEMISTRY, BENTHAM SCIENCE PUBLISHERS, HILVERSUM, NL, vol. 7, no. 14, 1 July 2007 (2007-07-01), pages 1364-1378, XP009109971 ISSN: 1568-0266
- RAWLINS P ET AL: "Inhibition of endotoxin-induced TNF-alpha production in macrophages by 5Z-7-oxo-zeaenol and other fungal resorcylic acid lactones." INTERNATIONAL JOURNAL OF IMMUNOPHARMACOLOGY DEC 1999, vol. 21, no. 12, December 1999 (1999-12), pages 799-814, XP002524065 ISSN: 0192-0561
- FERNANDES DARREN J ET AL: "Contribution of the p38MAPK signalling pathway to proliferation in human cultured airway smooth muscle cells is mitogen-specific." BRITISH JOURNAL OF PHARMACOLOGY AUG 2004, vol. 142, no. 7, August 2004 (2004-08), pages 1182-1190, XP002524066 ISSN: 0007-1188

## Description

### RELATED APPLICATIONS

This application claims priority to U.S. Provisional Application No. 61/000,796, filed on October 29, 2007, titled "Methods for Prognosing the Ability of a Zearalenone Analog Compound to Treat Cancer".

### BACKGROUND OF THE INVENTION

The increased number of cancer cases reported in the United States, and, indeed, around the world, is a major concern. Currently there are only a handful of treatments available for specific types of cancer, and these provide no absolute guarantee of success. In order to be most effective, these treatments require not only an early detection of the cancer, but a reliable assessment of whether the cancer can be effectively treated with known compounds, and a reliable determination of whether a cancer in a subject is sensitive to treatment.

It is known that mutations in the RAS/RAF/MEK/ERK MAPK signaling pathway are often observed in transformed cell lines and frequently linked with human cancer. Davies et al. (Nature 417:949-954, 2002), for example, identified *BRAF* (encoding BRAF protein, an isoform of RAF) somatic missense mutations in 67% of malignant melanomas and in 12% of colorectal cancers. Mutations in this pathway have also been associated with thyroid cancer (*e.g.*, papillary thyroid carcinoma), pancreatic cancer, brain tumors (*e.g.*, primary brain tumors), ovarian cancer, leukemia (*e.g.*, chronic myeloid leukemia and/or acute lymphoblastic leukemia (ALL)), breast cancer, neural cancer (*e.g.*, glioma, neuroblastoma or retinoblastoma), multiple myeloma, melanoma (*e.g.*, metastatic melanoma), colorectal cancer (*e.g.*, colorectal carcinoma), and B-cell lymphoma.

Recently, it was discovered that zearalenone analog compounds have unique multikinase inhibition profiles, which may be useful against specific cancers associated with mutations in the MAPK signaling pathway (see, *e.g.*, U.S. Serial No. 60/951,906, filed on July 25, 2007, and U.S. Serial No. 12/180,408, filed on July 25, 2008. Often, however, certain cancer cells are resistant to treatment with chemotherapeutic drugs. Known chemotherapeutic drugs, including zearalenone analog compounds, may not be effective for treating all known cancers. Roberts and Der, 2007 (Oncogene Vol. 26, 3291-3310) disclose a method to determine whether a cancer patient will respond to inhibitors of the MAPK signaling pathway by measuring BRAF, before and after treatment. Thus, a need exists in the art for a method of prognosing the ability of a chemotherapeutic compound such as a zearalenone analog compound to treat a cancer in a subject. Additionally, certain cancer cells may become resistant to treatment with chemotherapeutic compounds over time. Therefore, a need also exists in the art for methods of determining whether a cancer in a subject is sensitive to treatment with a chemotherapeutic compound, *e.g.*, a zearalenone analog compound.

### SUMMARY OF THE INVENTION

In its broadest sense, the invention concerns subject-matter as defined in the appended claims.

The present invention provides methods for prognosing the ability of a zearalenone analog compound of Formula I to treat, e.g., inhibit the growth, of a cancer in a subject. The present invention is based, at least in part, on the discovery that cancer cell lines with activated MAPK signaling or wild-type PI3K signaling, or activated MAPK signaling and wild-type PI3K signaling are sensitive to treatment with zearalenone analog compounds. The description describes that the level of a cytokine, *e.g.*, IL-8, or certain other response markers, *e.g.*, phospho-ERK, Cyclin D, phospho-pRB, and p27, can be used to determine whether a cancer in a subject is sensitive to treatment with a zearalenone analog compound.

The description describes the *BRAF* mutation (*e.g.*, V600E) and *PTEN* mutation status (optionally readable via phospho-AKT levels or AKT expression) as useful markers for patient selection. Patients with mutated *BRAF,* or low to moderate phospho-AKT levels, or mutated *BRAF* and low to moderate phospho-AKT levels are predicted to respond to zearalenone analog compounds such as compound 106. Once treated, early pharmacodynamic indications of response to drug treatment can be determined by measuring decreases in the level of a cytokine, such as a decrease in plasma IL-6 or IL-8 levels.

Decreases in pharmacodynamic markers such as phospho-ERK, cyclin D1 and/or phospho-pRB, or increases in CDK inhibitor, p27 provide additional surrogate markers for response to treatment with a zearalenone analog compound. Overall, these findings allow us to generate a biomarker program for zearalenone analog compounds which provides patient enrichment strategies, as well as modalities for follow-up to treatment with early assessment of drug response via pharmacodynamic monitoring.

In one aspect, the invention provides methods of prognosing the ability of a zearalenone analog compound of Formula I to treat a cancer in a subject, the method comprising a) determining whether a sample derived from the subject exhibits activated MAPK signaling as compared to a control sample; and b) determining whether the sample exhibits wild-type PI3K signaling as compared to a control sample, wherein activated MAPK signaling and wild-type PI3K signaling

as determined in steps a) and b) indicates that the zearalenone analog compound has the ability to treat the cancer in the subject, thereby prognosing the ability of the zearalenone analog compound to treat the cancer in the subject.

In another aspect, the invention provides methods for prognosing the ability of a zearalenone analog compound of Formula I to treat a cancer in a subject, the method comprising a) determining whether a sample derived from the subject exhibits a mutation in the *BRAF* gene which increases BRAF activity; and b) determining the level of phosphorylated AKT protein in the sample as compared to the total level of AKT protein in the sample or as compared to a control sample, wherein the presence of a mutation in the *BRAF* gene is an indication of activated MAPK signaling and a low to moderate level of phosphorylated AKT protein as determined in step b) is an indication of a wild-type PI3K signaling.

In another aspect, the invention provides methods for prognosing the ability of a zearalenone analog compound of Formula I to treat a cancer in a subject, the method comprising a) determining whether a sample derived from the subject exhibits a mutation in the *BRAF* gene which activates BRAF; and b) determining the expression level of AKT protein in the sample as compared to a control sample, wherein the presence of a mutation in the *BRAF* gene is an indication of activated MAPK signaling and a low to moderate level of expression of AKT protein as determined in step b) is an indication of a wild-type PI3K signaling.

In yet another aspect, the invention provides methods of prognosing the ability of a zearalenone analog compound of Formula I to treat a cancer in a subject, the method comprising a) determining whether a sample derived from the subject exhibits a mutation in the *BRAF* gene which increases BRAF activity; and b) determining whether the sample exhibits a wild-type *PTEN* sequence, wherein the presence of a mutation in the *BRAF* gene is an indication of activated MAPK signaling and a wild-type *PTEN* sequence in the sample, is an indication of a wild-type PI3K signaling.

In one embodiment, the method further comprises measuring the activity of AKT protein in a sample from the subject, wherein a low to moderate level of activity of AKT protein in said sample as compared to a control sample indicates that the zearalenone analog compound has the ability to treat the cancer in the subject, thereby prognosing the ability of the zearalenone analog compound to treat the cancer in the subject.

In another embodiment, the method further comprises determining the level of phosphorylated AKT protein in a sample from said subject as compared to the total level of AKT protein in the sample or as compared to a control sample, wherein a low to moderate level of phosphorylated AKT protein in the sample as compared to the total level of AKT protein in the sample or as compared to the control sample indicates that the zearalenone analog compound has the ability to treat the cancer in the subject, thereby prognosing the ability of the zearalenone analog compound to treat the cancer in the subject.

In another aspect, the invention provides methods of prognosing the ability of a zearalenone analog compound of Formula I to treat a cancer in a subject, the method comprising a) determining whether a sample derived from the subject exhibits a V600E mutation in the *BRAF* gene; wherein the presence of a V600E mutation in the *BRAF* gene is an indication of activated MAPK signaling.

In another aspect, the invention provides methods of prognosing the ability of a zearalenone analog compound of Formula I to treat a cancer in a subject, the method comprising a) determining whether a sample derived from the subject exhibits a V600E mutation in the *BRAF gene;* and b) determining the level of phosphorylated AKT protein in the sample as compared to a control sample, wherein the presence of a V600E mutation in the *BRAF* gene and a low to moderate level of phosphorylated AKT protein in the sample is from about level 1 to about level 4 as compared to the total level of AKT protein in the sample.

The description describes methods of prognosing the ability of a zearalenone analog compound to treat a cancer in a subject, the method comprising determining whether a sample derived from the subject exhibits a mutation in the *BRAF* gene, wherein the presence of a mutation in the *BRAF* gene in the sample as compared to a control sample indicates that a zearalenone analog compound has the ability to treat the cancer in the subject, thereby prognosing the ability of a zearalenone analog compound to treat the cancer in the subject.

The description describes methods of prognosing the ability of a zearalenone analog compound to treat a cancer in a subject, the method comprising determining the level of phosphorylated AKT protein in a sample from the subject as compared to the total level of AKT protein in the sample or as compared to a control sample, wherein a low to moderate level of phosphorylated AKT protein in the sample as compared to the total level of AKT protein in the sample or as compared to the control sample indicates that a zearalenone analog compound has the ability to treat the cancer in the subject, thereby prognosing the ability of a zearalenone analog compound to treat the cancer in the subject.

The description describes methods of prognosing the ability of a zearalenone analog compound to treat a cancer in a subject, the method comprising determining the level of expression of AKT protein in a sample from the subject as compared to a control sample, wherein a low to moderate level of expression of AKT protein indicates that a zearalenone analog compound has the ability to treat the cancer in the subject, thereby prognosing the ability of a zearalenone analog compound to treat the cancer in the subject. The description describes that, the level of expression is determined by measuring the level of mRNA. The description describes that the level of expression is determined by measuring the level of AKT at the protein level.

The description describes methods of prognosing the ability of a zearalenone analog compound to treat a

cancer in a subject, the method comprising measuring the activity of AKT protein in a sample from the subject, wherein a low to moderate level of activity of AKT protein in the sample as compared to a control sample indicates that a zearalenone analog compound has the ability to treat the cancer in the subject, thereby prognosing the ability of a zearalenone analog compound to treat the cancer in the subject.

The description describes methods of prognosing the ability of a zearalenone analog compound to treat a cancer in a subj ect, the method comprising determining the mutational status of *PTEN* in a sample from the subject, wherein the lack of a mutation in *PTEN* in the sample as compared to a control sample indicates that a zearalenone analog compound has the ability to treat the cancer in the subject, thereby prognosing the ability of a zearalenone analog compound to treat the cancer in the subject.

In one embodiment, the cancer is a *BRAF* mutated cancer. In another embodiment, the *BRAF* mutated cancer is selected from the group consisting of metastatic melanoma, papillary thyroid carcinoma, colorectal carcinoma, and a primary brain tumor.

In another embodiment, the cancer is selected from the group of solid tumors and hematological malignancies, including leukemias, lymphomas, and myelomas. For example, the cancer can be a cancer such as breast cancer, melanoma, ovarian cancer, thyroid cancer, pancreatic cancer, colorectal cancer, brain tumors, neural cancer, neuroblastoma, retinoblastoma, glioma, such as astrocytoma, glioblastoma multiforme or other CNS tumors, chronic lymphocytic leukemia (CLL), acute myeloid leukemia (AML), B-cell lymphomas (*e.g.*, non-Hodgkin's B-cell lymphomas), or multiple myeloma.

In one embodiment, the zearalenone analog compound is the compound:

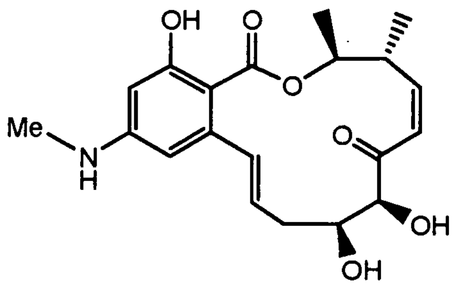

(Compound 091), or a pharmaceutically acceptable salt or ester thereof.

In another embodiment, the zearalenone analog compound is the compound:

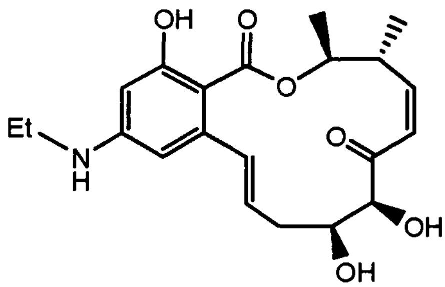

(Compound 106), or a pharmaceutically acceptable salt or ester thereof.

In one embodiment, the zearalenone analog is a compound of Formula I, or a pharmaceutically acceptable salt or ester thereof and wherein the $R_3$ is an unsubstituted $C_{1-3}$ alkyl-amino or wherein $R_3$ is a group selected from the group consisting of methylamino and ethylamino.

The *BRAF* gene encodes BRAF, a cytoplasmic serine/threonine kinase. Somatic mutation in the *BRAF* gene is common in human cancers. Many such mutations affect the kinase domain of the encoded protein (kinase domain mutations), and lead to elevated kinase activity of the encoded mutant BRAF protein. These mutations can lead to activation of the RAS/RAF/MEK/ERK MAPK signal transduction pathway.

In one embodiment, the mutation in the *BRAF* gene can be in a kinase domain of BRAF which may lead to elevated kinase activity of BRAF. In another embodiment, the mutation in the *BRAF* gene is V600E. In yet another embodiment, the mutation in the *BRAF* gene is selected from the group consisting of V600E, G464E, G464V, G466A, G466E, G466V, G469A, G469E, E586K, F595L, G596R, L597V, L597R, L597S and V600D.

In one embodiment, determining whether the sample exhibits a mutation in the *BRAF* gene is accomplished

using a technique selected from the group consisting of polymerase chain reaction (PCR) amplification reaction, reverse-transcriptase PCR analysis, single-strand conformation polymorphism analysis (SSCP), mismatch cleavage detection, heteroduplex analysis, Southern blot analysis, Western blot analysis, and deoxyribonucleic acid sequencing of the sample.

In another embodiment, determining whether the sample exhibits a mutation in the *BRAF* gene comprises measuring BRAF activity in the sample (*e.g.*, protein kinase activity of BRAF), wherein an increase in BRAF activity in the sample as compared to the control sample is an indication of a mutation in the *BRAF* gene.

In another embodiment, the level of AKT phosphorylation is determined by Western blot, immunohistochemistry (IHC) or fluorescent in situ hybridization (FISH). In another embodiment, the low to moderate level of phosphorylated AKT protein in the sample is from about level 1 to about level 4 as compared to the total level of AKT protein in the sample.

In one embodiment, determining whether the sample exhibits a lack of mutation in *PTEN* is accomplished using a technique selected from the group consisting of polymerase chain reaction (PCR) amplification reaction, reverse-transcriptase PCR analysis, single-strand conformation polymorphism analysis (SSCP), mismatch cleavage detection, heteroduplex analysis, Southern blot analysis, Western blot analysis, and deoxyribonucleic acid sequencing of the sample.

In one embodiment, the sample derived from the subject is a tumor biopsy.

In another embodiment, determining whether the sample exhibits activated MAPK signaling comprises identifying a mutation in the *BRAF* gene in the sample which increases BRAF activity, wherein the presence of a mutation in the *BRAF* gene in the sample is an indication of activated MAPK signaling. Mutations in the *BRAF* gene which are indicative of activated MAPK signaling include gain of function mutations, such as kinase domain mutations which increase the kinase activity of the encoded protein. In yet another embodiment, the mutation in the *BRAF* gene is selected from the group consisting of V600E, G464E, G464V, G466A, G466E, G466V, G469A, G469E, E586K, F595L, G596R, L597V, L597R, L597S and V600D.

In another embodiment, determining whether the sample exhibits activated MAPK signaling comprises measuring BRAF activity in the sample, wherein an increase in BRAF activity in the sample as compared to a control sample is an indication of activated MAPK signaling.

In another embodiment, determining whether the sample exhibits activated MAPK signaling comprises measuring the activity of one or more proteins selected from the group consisting of MEK1, MEK2, ERK1 and ERK2 in the sample, wherein an increase in the activity of the protein(s) in the sample (*e.g.*, protein kinase activity) as compared to a control sample is an indication of activated MAPK signaling.

In another embodiment, determining whether the sample exhibits wild-type PI3K signaling comprises determining the mutation status of the *PTEN* gene in the sample, wherein the lack of a loss of function mutation in the *PTEN* gene in the sample is an indication of wild-type PI3K signaling. The *PTEN* gene (also referred to as phosphatase and tensin homolog deleted on chromosome ten) encodes a protein which has protein phosphatase activity (serine/threonine/tyrosine phosphatase) and lipid phosphatase activity. *PTEN* is a tumor suppressor gene, which is a negative regulator of PI3K activity. Somatic mutation of the *PTEN gene* has been found in various human cancers. Loss of function mutations in the *PTEN gene* have been identified. For example, exons 7 and 8 of the *PTEN gene* contain $(A)_6$ repeats, and such sequences are targets of mutation (*e.g.*, frameshift). A 1 base pair deletion in the $(A)_6$ repeat of exon 7 or a 1 base pair deletion in the $(A)_6$ repeat of exon 8 have been observed in human cancers. Frameshifts in these regions have also been observed in human cancer (Guanti et al., Human Mol. Gen., 9(2): 283-287 (2000). Loss of function mutations in the *PTEN* gene (*e.g.*, deletions, insertions, point mutations) can decrease the protein and/or lipid phosphatase activity of PTEN, resulting in deregulation of PI3K and subsequent activation of AKT. Accordingly, the absence of a loss of function mutation in the *PTEN* gene or a wild-type *PTEN* sequence, can be used to determine whether a sample exhibits wild-type PI3K signaling. Conversely, the detection of a loss of function mutation in *PTEN* would not be indicative of wild-type PI3K signaling.

The description describes, determining whether the sample exhibits wild-type PI3K signaling comprises detecting DNA hypermethylation of the *PTEN* promoter in a sample as compared to a control. PTEN activity can be lost by promoter methylation silencing in many primary and metastatic human cancers, a phenomenon recognized as an alternative mechanism for tumor suppressor gene inactivation (Carnero et al., Curr. Cancer Drug Targets, 8(3):187-98 (2008); see also, Mirmohammadsadegh et al., Cancer Res., 66(13):6546-52 (2006)). Any suitable method can be used to detect DNA hypermethylation or CpG island hypermethylation, such as methylation-specific polymerase chain reaction (Hou et al., Cancer., 113(9):2440-7 (2008)) or quantitative positional methylation analysis (pyrosequencing) (Mirmohammadsadegh et al., Cancer Res., 66(13):6546-52 (2006)).

In one embodiment, determining whether the sample exhibits wild-type PI3K signaling comprises determining the level of phosphorylated AKT protein in the sample as compared to the total level of AKT protein in the sample, wherein a low to moderate level of phosphorylated AKT protein in the sample is an indication of wild-type PI3K signaling. In another embodiment, determining whether the sample exhibits wild-type PI3K signaling comprises determining the level of phosphorylated AKT protein in the sample as compared to a control sample, wherein a low to moderate level of

phosphorylated AKT protein in the sample is an indication of wild-type PI3K signaling. In some embodiments, the level of AKT phosphorylation is determined by Western blotting, immunohistochemistry (IHC) or fluorescent in situ hybridization (FISH).

The description describes, determining whether the sample exhibits wild-type PI3K signaling comprises determining the level of expression of AKT protein in a sample from the subject as compared to a control sample, wherein a low to moderate level of expression of AKT protein in the sample as compared to a control sample is an indication of wild-type PI3K signaling.

The description describes, determining whether the sample exhibits wild-type PI3K signaling comprises measuring the activity of AKT protein in the sample, wherein a low to moderate level of activity of AKT protein in the sample as compared to a control sample is an indication of wild-type PI3K signaling.

In another embodiment, combinations of these method can be used to determine whether the sample exhibits wild-type PI3K signaling. For example, determining whether the sample exhibits wild-type PI3K signaling can comprise (a) determining the mutation status of the *PTEN* gene in the sample and/or detecting DNA hypermethylation of the PTEN promoter; and (b) determining the level of phosphorylated AKT protein in the sample as compared to the total level of AKT protein in the sample; determining the level of phosphorylated AKT protein in the sample as compared to a control sample; measuring the activity of AKT protein in the sample as compared to a control sample; and/or determining the level of expression of AKT protein in a sample from the subject as compared to a control sample.

In another aspect, the invention provides a method of prognosing the ability of a zearalenone analog compound of Formula I to inhibit the growth of a cancer in a subject. The method includes: a) determining whether a sample derived from the subject exhibits activated MAPK signaling as compared to a control sample; b) determining whether a sample derived from the subject exhibits wild-type PI3K signaling as compared to a control sample, wherein activated MAPK signaling and wild-type PI3K signaling in the sample as compared to a control sample indicates that the zearalenone analog compound has the ability to inhibit the growth of a cancer in the subject, thereby prognosing the ability of the zearalenone analog compound to inhibit the growth of the cancer in the subject.

The description describes, the invention provides a method of prognosing the ability of a zearalenone analog compound of Formula I to promote the activation of apoptosis of a cancer in a subject. The method includes: a) determining whether a sample derived from the subject exhibits activated MAPK signaling as compared to a control sample; b) determining whether a sample derived from the subject exhibits wild-type PI3K signaling as compared to a control sample, wherein activated MAPK signaling and wild-type PI3K signaling in the sample as compared to a control sample indicates that a zearalenone analog compound has the ability to promote the activation of apoptosis of a cancer in the subject, thereby prognosing the ability of the zearalenone analog compound to promote the activation of apoptosis of the cancer in the subject.

The present invention also provides a composition comprising a therapeutically effective amount of a zearalenone analog compound of Formula I, or a pharmaceutically acceptable salt or ester thereof for use in treating cancer in a subject based on an evaluation the results of an assessment of a sample derived from the subject for activated MAPK signaling as compared to a control sample and for wild-type PI3K signaling as compared to a control sample, wherein the evaluation indicates that the sample has activated MAPK signaling and wild-type PI3K signaling.

The description describes use of a reagent for assessing the ability of a zearalenone analog compound to treat cancer in a subject, the use comprising: a) determining whether a sample derived from the subject exhibits activated MAPK signaling as compared to a control sample; and b) determining whether the sample exhibits wild-type PI3K signaling as compared to a control sample, wherein activated MAPK signaling and wild-type PI3K signaling in the sample as determined in steps a) and b) indicates that a zearalenone analog compound has the ability to treat the cancer in the subject.

The description describes the use of a reagent for assessing the ability of a zearalenone analog compound to treat cancer in a subject, the use comprising: a) determining whether a sample derived from the subject exhibits a mutation in the *BRAF* gene; and b) determining the level of phosphorylated AKT protein in the sample as compared to the total level of AKT protein in the sample or as compared to a control sample, wherein the presence of a mutation in the *BRAF* gene and a low to moderate level of phosphorylated AKT protein in the sample as determined in

step b), indicates that a zearalenone analog compound has the ability to treat the cancer in the subject.

The description describes the use of a reagent for assessing the ability of a zearalenone analog compound to treat cancer in a subject, the use comprising: a) determining whether a sample derived from the subject exhibits a mutation in the *BRAF* gene; and b) determining whether the sample exhibits a wild-type *PTEN* sequence, wherein the presence of a mutation in the *BRAF* gene and a wild-type *PTEN* sequence in the sample indicates that a zearalenone analog compound has the ability to treat the cancer in the subject.

The description describes the use of a reagent for assessing the ability of a zearalenone analog compound to treat cancer in a subject, the use comprising: a) determining whether a sample derived from the subject exhibits a V600E mutation in the *BRAF* gene; and b) determining the level of phosphorylated AKT protein in the sample as compared to the total level of AKT protein in the sample or as compared to a control sample, wherein the presence of a V600E

mutation in the *BRAF* gene and a low to moderate level of phosphorylated AKT as determined in step b) indicates that a zearalenone analog compound has the ability to treat the cancer in the subject.

The description describes methods of treating a cancer in a subject comprises a) evaluating the results of an assessment of a sample derived from the subject for the presence of a mutation in the *BRAF* gene; and b) administering a therapeutically effective amount of a composition comprising a zearalenone analog compound to the subject, if the results of the assessment indicate that the sample exhibits a mutation in the *BRAF* gene (*e.g.,* a V600E mutation in the *BRAF* gene).

The description describes methods of treating a cancer in a subject comprising a) evaluating the results of an assessment of a sample derived from the subject for the level of phosphorylated AKT protein in the sample as compared to the total level of AKT protein in the sample or as compared to a control sample; and b) administering a therapeutically effective amount of a composition comprising a zearalenone analog compound to the subject, if the results of the assessment indicate that the sample exhibits a low to moderate level of phosphorylated AKT protein.

The description describes methods of treating a cancer in a subject comprising a) evaluating the results of an assessment of a sample derived from the subject for the activity of AKT protein in the sample as compared to the activity of AKT protein in the sample or as compared to a control sample; and b) administering a therapeutically effective amount of a composition comprising a zearalenone analog compound to the subject, if the results of the assessment indicate that the sample exhibits a low to moderate level of activity of AKT protein.

The description describes, a method of treating a cancer in a subject comprising a) evaluating the results of an assessment of a sample derived from the subject for the mutational status of the *PTEN* gene; and b) administering a therapeutically effective amount of a composition comprising a zearalenone analog compound to the subject, if the results of the assessment indicate that the sample exhibits wild-type *PTEN* sequence.

In one embodiment, the cancer is a *BRAF* mutated cancer. In another embodiment, the *BRAF* mutated cancer is selected from the group consisting of metastatic melanoma, papillary thyroid carcinoma, colorectal carcinoma, and a primary brain tumor.

In another embodiment, the cancer is selected from the group of solid tumors and hematological malignancies, including leukemias, lymphomas, and myelomas. For example, the cancer can be a cancer such as breast cancer, melanoma, ovarian cancer, thyroid cancer, pancreatic cancer, colorectal cancer, brain tumors, neural cancer, neuroblastoma, retinoblastoma, glioma, such as astrocytoma, glioblastoma multiforme or other CNS tumors, chronic lymphocytic leukemia (CLL), acute myeloid leukemia (AML), B-cell lymphomas (e.g., non-Hodgkin's B-cell lymphomas), or multiple myeloma.

In one embodiment, the zearalenone analog compound is the compound:

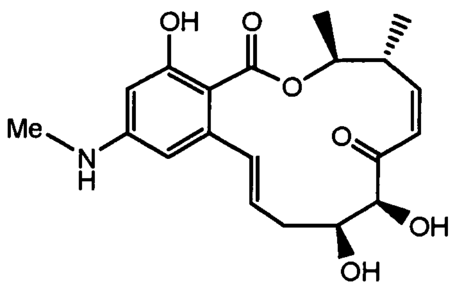

(Compound 091), or a pharmaceutically acceptable salt or ester thereof.

In another embodiment, the zearalenone analog compound is the compound:

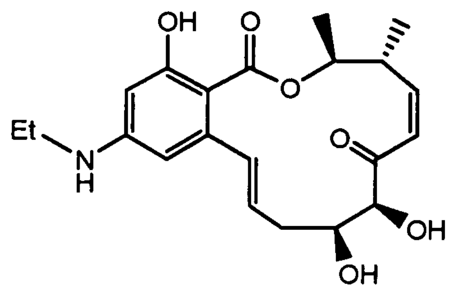

(Compound 106), or a pharmaceutically acceptable salt or ester thereof.

In one embodiment, the zearalenone analog compound of Formula I, or a pharmaceutically acceptable salt or ester thereof and wherein the $R_3$ is an unsubstituted $C_1$-$_3$ alkyl-amino or wherein $R_3$ is a group selected from the group consisting of methylamino and ethylamino.

The description describes a kit for prognosing the ability of a zearalenone analog compound to treat a cancer in a subject. The kit comprises a reagent, *e.g.*, a probe or an antibody, for determining whether a sample exhibits activated MAPK signaling; and a reagent, *e.g.*, a probe or an antibody, for determining whether the sample exhibits wild-type PI3K signaling. The description describes that the reagent for determining whether the sample exhibits activated MAPK signaling is a probe for identifying a *BRAF* mutation. The description describes that the reagent for determining whether the sample exhibits wild-type PI3K signaling is a probe for identifying a wild-type *PTEN* sequence.

Other features and advantages of the invention will be apparent from the following detailed description and claims.

## BRIEF DESCRIPTION OF THE FIGURES

***Figure 1*** is a schematic summarizing the RAS/RAF/MEK/ERK MAPK and PI3K signaling pathways.

***Figure 2*** is a graph demonstrating that the phosphorylation status of AKT affects the sensitivity of various cancer cell lines to compound **106.**

***Figures 3A, 3B, 3C*** and 3D are graphs and tables summarizing the cancer cell lines which were used in the experiments described herein. These tables also summarize the $IC_{50}$ values for compound **106** and compound **091** in several cancer cell lines.

***Figure 4*** is a schematic demonstrating the use of prognostic biomarkers for the enrichment of patients sensitive to compound **106** and the use of surrogate response markers to determine the response of patients after treatment with compound **106.**

***Figure 5*** is a graph demonstrating that *BRAF* mutated cancer cells produce the pro-inflammatory cytokine Interleukin-8 (IL-8).

***Figure 6*** is a graph demonstrating that IL-6 and IL-8 production is inhibited by Compound **106** in LOX melanoma cells *in vitro.*

***Figure 7*** is a graph demonstrating that tumor bearing mice sensitive to compound 106 show a significant decrease in plasma IL-8 levels after treatment with compound 106, whereas tumor bearing mice resistant to compound 106 do not show any significant change in plasma IL-8 levels after treatment with compound 106.

*Figure 8* is a set of Western blots demonstrating the protein levels of several response markers, e.g., phospho-ERK, Cyclin D1, p27 and phospho-pRB, after treatment with compound 106.

***Figures 9A and 9B*** are immunohistochemistry (IHC) stains demonstrating phospho-pRB and phospho-ERK staining in LOX melanoma xenografts.

***Figure 10*** is a graph demonstrating the response of s.c. DBTRG-05MG glioblastoma tumors to treatment with Compound 106.

***Figure 11*** is a graph demonstrating the response of s.c. LOX melanoma tumors to treatment with Compound 106.

## DETAILED DESCRIPTION OF THE INVENTION

The RAS/RAF/MEK/ERK MAPK signal transduction pathway regulates cell proliferation in diverse types of cells. Mutations in this pathway are often observed in transformed cell lines and frequently linked with human cancer (see, *e.g.*, Wallace et al. (2005) Current Topics in Medicinal Chemistry 5:215-219). Aspects of the present invention are based, at least in part, on the discovery that cancer cell lines with activated MAPK signaling and wild-type PI3K signaling, are sensitive to treatment with a zearalenone analog compound of Formula I, *e.g.*, Compound 106. The present invention provides, among other things, methods for prognosing the ability of a zearalenone analog compound of Formula I to

treat a cancer in a subject.

Other aspects of the invention are based, at least in part, on the discovery that the levels of certain proteins can be used as surrogate markers for response to treatment with a zearalenone analog compound, and the invention provides methods useful in determining whether a cancer in a subject is sensitive to treatment with a zearalenone analog compound.

In order to more clearly and concisely describe the subject matter of the claims, the following definitions are intended to provide guidance as to the meaning of specific terms used herein.

*Definitions*

It is to be noted that the singular forms "a," "an," and "the" as used herein include "at least one" and "one or more" unless stated otherwise. Thus, for example, reference to "a pharmacologically acceptable carrier" may include mixtures of two or more carriers as well as a single carrier.

As used herein, the terms "prognosis", "prognose", or "prognosing" refer to a prediction of a probability, course or outcome. Specifically, "prognosing the ability of a zearalenone analog compound to treat a cancer in a subject" refers to the prediction that the zearalenone analog compound is likely to be useful for treating a cancer in a subject. For example, the prognostic methods of the instant invention provide for determining whether a sample exhibits specific characteristics (*e.g.*, activated MAPK signaling, wild-type PI3K signaling, a mutation in the *BRAF* gene, the status of AKT phosphorylation, and/or wild-type *PTEN* sequence) which can be used to predict whether a zearalenone analog compound has the ability to treat a cancer in a subject.

"Treat", "treatment", "treating" or "treated" as used herein, refers to a cancer being cured, healed, alleviated, relieved, remedied, ameliorated, or improved. For example, the prognostic methods of the instant invention are useful in determining whether a zearalenone analog compound can slow or stop the progression of a specific cancer or a specific class of cancer *(e.g.,* a *BRAF* associated cancer).

The term "subject," as used herein, refers to animals such as mammals, including, but not limited to, humans, primates, cows, sheep, goats, horses, pigs, dogs, cats, rabbits, guinea pigs, rats, mice or other bovine, ovine, equine, canine, feline, rodent or murine species. In some embodiments, the subject is a human.

The term "activated MAPK signaling", as used herein, refers to signaling that is associated with or affected by an increase in the activity or function of the MAPK signaling pathway (see Figure 1). The RAS/RAF/MEK/ERK MAPK signaling pathway is viewed as an important pathway for anticancer therapies, based upon its central role in regulating the growth and survival of cells from a broad spectrum of human tumors. In one embodiment of the invention, determining whether a sample exhibits "activated MAPK signaling" comprises identifying a mutation in the *BRAF* gene in the sample, wherein a presence of a mutation in the *BRAF* gene, *e.g.*, V600E, is an indication of activated MAPK signaling. In another embodiment, determining whether a sample exhibits "activated MAPK signaling" comprises measuring BRAF protein kinase activity in a sample, wherein an increase in BRAF protein kinase activity in the sample as compared to a control sample is an indication of activated MAPK signaling. In another embodiment, determining whether a sample exhibits "activated MAPK signaling" comprises measuring the activity of a protein involved in the MAPK signaling pathway (such as MEK1, MEK2, ERK1, and/or ERK2, or any of the proteins well known in the art as being involved in this pathway, *e.g.*, those identified in Figure 1) in a sample, wherein an increase in the activity of the protein in the sample as compared to a control sample is an indication of activated MAPK signaling.

The term "wild-type PI3K signaling", as used herein, refers to signaling that is associated with the normal activity or function of the PI3K signaling pathway (see Figure 1). The PI3K signaling pathway is an important pathway for anticancer therapies, based on its central role in regulating apoptosis in cells. In one embodiment of the invention, determining whether a sample exhibits wild-type PI3K signaling comprises determining whether said sample exhibits a wild-type *PTEN* sequence, wherein the a lack of a mutation in the *PTEN* gene in the sample is an indication of wild-type PI3K signaling. In another embodiment, determining whether a sample exhibits wild-type PI3K signaling comprises measuring the activity of PTEN in a sample (*e.g.*, phosphatase activity), wherein a low to moderate level of PTEN activity in the sample as compared to a control sample is an indication of wild-type PI3K signaling. In another embodiment, determining whether a sample exhibits wild-type PI3K signaling comprises determining the level of phosphorylated AKT protein in a sample, wherein a low to moderate level of phosphorylated AKT protein in the sample is an indication of wild-type PI3K signaling. In another embodiment, determining whether a sample exhibits wild-type PI3K signaling comprises measuring the activity of AKT protein kinase in a sample, wherein a low to moderate level of protein kinase activity of AKT protein in the sample as compared to a control sample is an indication of wild-type PI3K signaling. The description describes that wild-type PI3K signaling may be determined by measuring the activity or mutation status of any protein involved in the PI3K signaling pathway, includingAKT, BAD, BCL-XL, Caspase 9, PDK, AFX or any of the proteins identified in Figure 1.

"Determining whether a sample exhibits a mutation" may be accomplished using any suitable method, such as techniques available in the art, *e.g.*, deoxyribonucleic acid sequencing of a sample, polymerase chain reaction (PCR)

amplification, reverse-transcriptase PCR analysis, single-strand conformation polymorphism analysis (SSCP), mismatch cleavage detection, heteroduplex analysis, Southern blot analysis, or Western blot analysis. These techniques are well known to one of ordinary skill in the art and are generally described in Sambrook, J. et al. (1989) "Molecular Cloning: A Laboratory Manual", Cold Spring Harbor Laboratory Press. Determining whether a sample exhibits a mutation may entail examination or all or part of a gene for the presence of a mutation (e.g., in a kinase domain).

The term "mutation in the *BRAF* gene", as used herein, refers to a *BRAF* gene sequence which contains one or more mutations that lead to activation or a gain in BRAF function. *BRAF* mutations are well known in the art. For a review *of BRAF* mutations, see Davies et al. (2002) Nature 417:949-954 and Rodriguez-Viciana et al. (2006) Science 311:1287-1290. The most common mutation in the *BRAF* gene is referred to as the V600E mutation (originally described as V599E) and accounts for more than 90% *of BRAF* mutations. Additional mutation sites are known in the art, such as those described in the Davies *et al.* and Rodriguez-Viciana *et al.* articles cited above. For example, *BRAF* mutations include G464E, G464V, G466A, G466E, G466V, G469A, G469E, E586K, F595L, G596R, L597V, L597R, L597S and V600D.

As used herein, "RAF" includes RAF protein isoforms RAF-1 (C-RAF), BRAF and/or A-RAF.

As used herein, "ERK" or "ERK1/2" refer to extracellular signal-regulated kinases ERK1 and/or ERK2, regardless of phosphorylation state. "Phospho-ERK" or "p-ERK" refers to phospho-ERK1 and/or phospho-ERK2.

As used herein, "AKT" or "AKT protein" refers to AKT1, AKT2 and/or AKT3 (regardless of phosphorylation state), which are members of the AKT family of protein kinases. "Phospho-AKT" or "p-AKT" refers to phospho-AKT1, phospho-AKT2 and/or phospho-AKT3.

In some embodiments, the level of phosphorylated AKT protein in a sample as compared to the total level of AKT protein in a sample is determined. In other embodiments, the level of phosphorylated AKT protein in a sample as compared to a control sample is determined.

Cells with wild-type PI3K signaling can exhibit a "low to moderate level of phosphorylated AKT protein", whereas activated PI3K signaling enhances phosphorylation of AKT. In some embodiments, the level of phosphorylated AKT is determined relative to a control sample, such as the level of phosphorylated AKT in normal tissue having wild-type PI3K signaling (*e.g.*, a range determined from the levels of phospho-AKT observed in normal tissue samples). In these embodiments, a "low to moderate level of phosphorylated AKT protein" will be similar to that observed in normal tissue (*e.g.*, falls within the normal range observed in normal tissue samples). In some embodiments, the level of phosphorylated AKT is determined relative to a control sample, such as the level of phosphorylated AKT in tumor samples from other subjects. For example, the level of phosphorylated AKT in tumor samples from a variety of subjects can be determined to define low to moderate levels which are sensitive to treatment with a zearalenone analog compound, and the sample of a subject of interest is compared to these.

When determined as compared to the total level of AKT protein in a sample, a "low to moderate level of phosphorylated AKT protein" indicates that for example, about 75% or less, about 60% or less, about 55% or less, about 50% or less, about 45% or less, about 40% or less, about 35% or less, about 30% or less, about 25% or less, about 20% or less, about 15% or less, about 10% or less, about 9% or less, about 8% or less, about 7% or less, about 6% or less, about 5% or less, about 4% or less, about 3% or less, about 2% or less, or about 1% or less of the AKT protein is phosphorylated in the sample.

In one embodiment, a low to moderate level of phosphorylated AKT protein in said sample is from about 1% to about 75% of the total level of AKT protein in the sample. In another embodiment, the low to moderate level of phosphorylated AKT protein in said sample is from about 1% to about 40% of the total level of AKT protein in the sample. In one embodiment, the low to moderate level of phosphorylated AKT protein in said sample is from about 30% to about 40% of the total level of AKT protein in the sample. In another embodiment, the low to moderate level of phosphorylated AKT protein in said sample is from about 1% to about 10%; from about 1% to about 20%; from about 10% to about 50%; or from about 20% to about 50% of the total level of AKT protein in the sample.

The level of phosphorylated AKT protein can be determined using any suitable method, such as Western blotting, immunohistochemistry (IHC), or FISH. In a preferred embodiment, Western blotting is used to determine the level of phosphorylated AKT protein. After performing the Western blot, a grading system of 1 to 10 can be utilized to characterize the level of phosphorylated AKT protein in the sample. In every instance, the total level of AKT protein in the sample is assigned a level of 10. This total level of AKT protein is then compared to the level of phosphorylated AKT protein in the sample. In one embodiment, the level of phosphorylated AKT protein in the sample is 75% of the total AKT protein in the sample, and is assigned a level of 7.5. In another embodiment, the level of phosphorylated AKT in the sample is 50% of the total AKT protein in the sample, and is assigned as a level of 5. In another embodiment, the level of phosphorylated AKT in the sample is 40% of the total AKT protein in the sample, and assigned as a level of 4. In another embodiment, the level of phosphorylated AKT in the sample is 30% of the total AKT protein in the sample, and assigned as a level of 3. In another embodiment, the level of phosphorylated AKT in the sample is 20% of the total AKT protein in the sample, and assigned as a level of 2. In another embodiment, the level of phosphorylated AKT in the sample is 10% of the total AKT protein in the sample, and assigned as a level of 1. In another embodiment, the level of

phosphorylated AKT in the sample is 5% of the total AKT protein in the sample, and assigned as a level of 0.5.

When this grading system is used, a "low to moderate level of phosphorylated AKT protein" corresponds to a level of 7.5 or less, for example, 7, 6.5, 6, 5.5, 5, 4.5, 4, 3.5, 3, 2.5, 2, 1.5, 1, 0.5 or 0.1. In some embodiments, a low to moderate level of phosphorylated AKT protein in the sample is from about level 1 to about level 7.5, from about level 1 to about level 7, from about level 1 to about level 6, from about level 1 to about level 5, from about level 1 to about level 4, from about level 1 to about level 3, from about level 1 to about level 2, from about level 0.5 to about level 1, from about level 2 to about level 5, from about level 3 to about level 6, from about level 4 to about level 7 or from about level 2 to about level 6.

Cells with wild-type PI3K signaling can exhibit a "low to moderate level of expression of AKT protein", whereas activated PI3K signaling enhances expression of AKT. In some embodiments, the level of expression of AKT protein is determined relative to a control sample, such as the level of expression of AKT in normal tissue having wild-type PI3K signaling (*e.g.*, a range determined from the levels of expression of AKT observed in normal tissue samples). In these embodiments, a "low to moderate level of expression of AKT protein" will be similar to that observed in normal tissue (*e.g.*, falls within the normal range observed in normal tissue samples). In some embodiments, the level of expression of AKT is determined relative to a control sample, such as the level of expression of AKT in tumor samples from other subjects. For example, the level of expression of AKT in tumor samples from a variety of subjects can be determined to define low to moderate levels which are sensitive to treatment with a zearalenone analog compound, and the sample of a subject of interest is compared to these.

The term "wild-type *PTEN* sequence", as used herein, refers to a *PTEN* sequence which does not contain any mutations that lead to a loss in PTEN function or to a *PTEN* sequence which has been examined for one or more mutational hot spots and examination does not reveal a mutation. Over one hundred *PTEN* mutations have been identified and are well known in the art. For a review of *PTEN* mutations, see Guanti et al. (2000) Human Molecular Genetics 9 (2):283-287. For example, exons 7 and 8 of the *PTEN* gene sequence contain an (A)6 repeat and mononucleotide repeat sequences. These sites are frequent targets for mutation in cancer. Most frequently, a one base pair deletion in the (A) 6 repeat of exon 7 or exon 8 creates premature stop, which consequently leads to the loss of gene function. For example, Exon 7 and/or exon 8 are examples of mutational hot spots. For example, exon 7 and/or exon 8 can be sequenced and if no mutation is detected, the sequence can be considered to be "wild-type *PTEN* sequence" for the purposes of the assay.

The term "*BRAF* mutated cancer", as used herein, refers to cancers that are associated with one or more mutations in the *BRAF* gene that lead to activation or a gain in BRAF function. Human cancers often contain somatic missense mutations in the *BRAF* gene (see, *e.g.*, Davies et al. (2002) Nature 417:949). The *BRAF* mutations are often in the kinase domain of BRAF, with the predominant mutation, V600E, accounting for 90% of *BRAF* mutations. Other *BRAF* mutations include G464E, G464V, G466A, G466E, G466V, G469A, G469E, E586K, F595L, G596R, L597V, L597R, L597S and V600D. As a result of the mutation in the *BRAF* gene, *BRAF* mutated cancers demonstrate elevated kinase activity, leading to the activation of MEK (mitogen activated-protein kinase/extracellular signal-regulated kinase), which then triggers ERK phosphorylation and activates the downstream pathway. Exemplary *BRAF* mutated cancers are discussed in more detail herein, and may include, *e.g.*, melanoma *(e.g.,* metastatic melanoma), thyroid cancer (*e.g.*, papillary thyroid carcinoma), colorectal cancer (*e.g.*, colorectal carcinoma), brain tumors (*e.g.*, primary brain tumors, *e.g.*, glioblastoma), ovarian cancer, leukemia (*e.g.*, chronic myeloid leukemia and/or acute lymphoblastic leukemia (ALL)), breast cancer, neural cancer (*e.g.*, glioma, neuroblastoma or retinoblastoma), multiple myeloma, and B-cell lymphoma. Cancers designated as *"BRAF* associated" are cancers which have been chosen because of their apparent association with specific protein mutations in BRAF, as described above.

As used herein, the term "resistance" refers to the occasion where a subject becomes less responsive to a zearalenone analog compound, *e.g.*, Compound 106, over time. Accordingly, in some embodiments, resistance to a zearalenone analog compound refers to a subject's complete non-responsiveness to the compound (*e.g.*, where rate of growth of a tumor is not inhibited). In some embodiments, resistance to a zearalenone analog compound refers to a subject's partial non-responsiveness to the compound (*e.g.*, where the rate of growth of a tumor is inhibited only to a very low degree, such as an inhibition of the growth of a tumor by about 1%, 2%, 3%, 4%, 5%, 6%, 7%, 8%, 9%, 10%, 15%, 20% or 25%). The quality of being resistant to a zearalenone analog compound is a highly variable one, with different tumors exhibiting different levels of "resistance" to a given zearalenone analog compound, under different conditions. In other embodiments, resistance to a zearalenone analog compound refers to a subject's complete or partial non-responsiveness to the compound as compared to a previous administration of the compound.

The term "sensitive to treatment", as used herein, refers to the occasion where a cancer in a subject is responsive to treatment with a zearalenone analog compound, *e.g.*, Compound 106. In some embodiments, complete responsiveness of the cancer to a zearalenone analog compound (*e.g.*, where the growth of a tumor is inhibited) is observed. In some embodiments, partial responsiveness of the cancer to a zearalenone analog compound (*e.g.*, where the rate of growth of a tumor is inhibited to some degree, such as an inhibition of the growth of the tumor by about 95%, 90%, 85, 80%, 75%, 70%, 65%, 60%, 55%, 50%, 45% or 40%) is observed. The quality of being sensitive to treatment with a zearalenone analog compound may vary, with different tumors exhibiting different levels of "sensitivity" to a given zearalenone analog compound, under different conditions. In one embodiment, the term sensitive to treatment refers to the effective treatment of a cancer with a zearalenone analog compound.

Numerous values and ranges are recited in connection with various embodiments of the present invention, *e.g.*, amount of a compound of the invention present in a composition. It is to be understood that where a range is given (*e.g.*, from X to Y), the range includes X, Y and all values which fall between X and Y, unless explicitly stated otherwise. The term "about" as used herein in association with parameters, ranges and amounts, means that the parameter or amount is within ± 1% of the stated parameter or amount.

*Methods of Prognosis*

The present invention provides methods for prognosing the ability of a zearalenone analog compound to treat cancer, such as *BRAF* mutated cancer, *e.g.*, melanoma (*e.g.*, metastatic melanoma), thyroid cancer (*e.g.*, papillary thyroid carcinoma), colorectal cancer (*e.g.*, colorectal carcinoma), brain tumors (*e.g.*, primary brain tumors, glioblastoma), ovarian cancer, leukemia (*e.g.*, chronic myeloid leukemia and/or acute lymphoblastic leukemia (ALL)), breast cancer, neural cancer (*e.g.*, glioma, neuroblastoma or retinoblastoma), multiple myeloma, and B-cell lymphoma.

In one aspect, methods of the invention generally include determining whether a sample derived from a subject suffering from a cancer exhibits activated MAPK signaling as compared to a control sample and/or determining whether the sample exhibits wild-type PI3K signaling as compared to a control sample, wherein activated MAPK signaling and/or wild-type PI3K signaling in the sample as compared to the control sample indicates that the zearalenone analog compound has the ability to treat the cancer in the subject, thereby prognosing the ability of the zearalenone analog compound to treat the cancer in the subject.

It is believed that the RAS/RAF/MEK/ERK MAPK signal transduction pathway (see Figure 1) regulates cell proliferation in diverse types of cells. Mutations in this pathway are often observed in transformed cell lines and frequently linked with human cancer. Davies et al. (Nature 417, 949-954, 2002) previously discovered that *BRAF* (encoding an isoform of RAF) somatic missense mutations occur in 67% of all malignant melanomas and 12% of all colorectal cancers. The *BRAF* mutants typically encode a mutation in the kinase domain of BRAF, with the predominant mutation, V600E, accounting for 90% *of BRAF* mutations. Other *BRAF* mutations include G464E, G464V, G466A, G466E, G466V, G469A, G469E, E586K, F595L, G596R, L597V, L597R, L597S and V600D. As a result of a mutation in the *BRAF* gene, *BRAF* mutated cancers demonstrate elevated kinase activity, leading to the activation of MEK (mitogen activated-protein kinase/ extracellular signal-regulated kinase kinase), which then triggers ERK phosphorylation and activates the downstream pathway. Therefore, the invention provides a new strategy for prognosing the ability of a zearalenone analog compound to treat cancer by determining the level of MAPK signaling in a sample derived from a subject with cancer. The invention also provides a new strategy for prognosing the ability of a zearalenone analog compound to treat cancer by determining whether a sample derived from a subject with cancer exhibits a *BRAF* mutation.

In certain embodiments, methods of the invention are useful for prognosing the ability of a zearalenone analog compound to treat tumors with activated MAPK signaling, including, but not limited to, those with *BRAF* mutations. Three proteins have received the most attention as targets for activated MAPK signaling (see Table 1 below, modified from Table 1 in Nature Reviews of Cancer: 4, 2004). Mutations in these three proteins could lead to activation of the MEK-ERK pathway. Specifically, ovarian cancer, thyroid cancer, colorectal cancer and melanoma show high frequencies of *BRAF* mutations.

**Table 1**

| Tumor type | Pathway mutations in patient tumors |
|---|---|
| Colon | *KRAS* (45%), *BRAF* (*12%*) |
| Pancreatic | *KRAS* (90%) |
| Ovarian | *BRAF (30%)* |
| Melanoma | *NRAS(15%), BRAF* (67%) |
| Non-small cell lung | *KRAS* (35%) |
| Papillary thyroid | *HRAS*, *KRAS* and *NRAS* (60%); *BRAF* (30-70%) |
| ALL, AML | *NRAS* (30%) |

Accordingly, in some embodiments, determining whether a sample exhibits activated MAPK signaling comprises identifying a mutation in the *BRAF* gene in the sample as compared to a control sample (*e.g.*, normal tissue from the same subject). For example, the mutation in the *BRAF* gene may be V600E (originally described as V599E), G464E

(originally described as G463E), G464V (originally described as G465V), G466A (originally described as G465A), G466E (originally described as G465E), G466V (originally described as G465V), G469A (originally described as G468A), G469E (originally described as G468E), E586K (originally described as E585K), F595L (originally described as F594L), G596R (originally described as G595R), L597V (originally described as L596V), L597R (originally described as L596R), L597S (originally described as L596S) or V600D (originally described as V599D). As many such mutations are known, when detecting such mutations, the known wild-type sequence from normal tissues of other subjects can be considered as a control.

The mutation can be determined by any suitable method, including art known techniques, such as polymerase chain reaction (PCR) amplification, reverse-transcriptase PCR analysis, single-strand conformation polymorphism analysis (SSCP), mismatch cleavage detection, heteroduplex analysis, Southern blot analysis, Western blot analysis, and deoxyribonucleic acid sequencing of the gene. These techniques are well known in the art and described in, for example, Sambrook, J. et al. (1989) "Molecular Cloning: A Laboratory Manual", Cold Spring Harbor Laboratory Press.

Determining whether a sample exhibits activated MAPK signaling may also be achieved by measuring RAF protein kinase activity in the sample (*e.g.*, RAF-1, A-RAF, BRAF), wherein an increase in RAF protein kinase activity in the sample as compared to a control sample is an indication of activated MAPK signaling. In other embodiments, determining whether said sample exhibits activated MAPK signaling comprises measuring the activity of a protein involved in the MAPK signaling pathway (such as MEK1, MEK2, ERK1, ERK2, or any of the proteins well known in the art as being involved in this pathway, *e.g.*, those identified in Figure 1) in a sample, wherein an increase in the activity of the protein in the sample as compared to the control sample is an indication of activated MAPK signaling.

Protein activity can be measured by any suitable method, including any of a variety of methods known in the art. For example, kinase activity can be measured by enzyme-linked immunosorbent assay (ELISA), by determining the phosphorylation state of downstream proteins using radioactive means, for example $^{32}P$ or $^{33}P$-gammaphosphate incorporation, by assays employing labeled antibodies, including immunoprecipitation, blotting, and gel electrophoresis, by immunohistochemistry, or by fluorescent in situ hybridization (FISH). Other methods for measuring kinase activity are described in WO95/04136, EP0730740B1, U.S. Patent No. 5,599,681, and U.S. Patent No. 6,942,987. For example, RAF-1 or BRAF kinase activity can be determined using RAF-1 immunoprecipitation kinase cascade kits or BRAF kinase cascade kits (Upstate Biotechnology (Millipore)), and

MEK/ERK activity can be measured using a coupled MEK/ERK activation assay (see, *e.g.*, Stokoe et al. (1994) Science 264:1463-1467). ERK activity can also be determined using immunoprecipitated protein or with a p42/p44MAP kinase enzyme assay kit (see, *e.g.*, Yoon et al. (2004) Am. J. Physiol. Renal Physiol. 286:F417-F424).

In some embodiments, determining whether a sample exhibits wild-type PI3K signaling comprises determining the mutation status of the *PTEN* gene in said sample, wherein the lack of a loss of function mutation in the *PTEN gene* in the sample is an indication of wild-type PI3K signaling. For example, if a one base pair deletion in the (A)6 repeat of exon 7 or exon 8 that leads to a premature stop, is detected, the presence of the mutation is an indication that PI3K signaling is altered relative to wild-type. The presence or absence of a *PTEN* mutation can be determined by any suitable technique, such as art known techniques, including polymerase chain reaction (PCR) amplification, reverse-transcriptase PCR analysis, single-strand conformation polymorphism analysis (SSCP), mismatch cleavage detection, heteroduplex analysis, Southern blot analysis, western blot analysis, and deoxyribonucleic acid sequencing of the gene.

In other embodiments, determining whether a sample exhibits wild-type PI3K signaling comprises measuring the level of phosphorylated AKT protein in the sample, wherein a low to moderate level of phosphorylated AKT in the sample is an indication of wild-type PI3K signaling. In some embodiments, measuring the level of AKT phosphorylation is determined by Western blotting, immunohistochemistry or fluorescent in situ hybridization (FISH).

As indicated above, in some embodiments, the level of phosphorylated AKT protein in a sample is determined by comparing the level of phosphorylated AKT protein in said sample to the total level of AKT protein in a sample. In other embodiments, the level of phosphorylated AKT protein in a sample is determined by comparing the level of phosphorylated AKT protein in said sample as compared to a control sample.

The level of phosphorylated AKT protein may be determined using any suitable method, such as Western blotting, immunohistochemistry (IHC), or FISH. In a preferred embodiment, Western blotting is used to determine the level of phosphorylated AKT protein. As explained herein, after performing the Western blot, a grading system of 1 to 10 can be utilized to characterize the level of phosphorylated AKT protein in the sample.

In other embodiments, determining whether a sample exhibits wild-type PI3K signaling comprises measuring the activity of AKT protein in the sample, wherein a lack of an increase or decrease in the activity of AKT protein in the sample as compared to a control sample is an indication of wild-type PI3K signaling. Protein activity can be measured by a variety of methods. For example, kinase activity can be measured by enzyme-linked immunosorbent assay (ELISA), by determining the phosphorylation state of downstream proteins using radioactive means, for example $^{32}P$ or $^{33}P$-gammaphosphate incorporation, by assays employing labeled antibodies, including immunoprecipitation, blotting, and gel electrophoresis, by immunohistochemistry, or by fluorescent in situ hybridization (FISH). Other methods for measuring kinase activity are described in WO95/04136, EP0730740B1, U.S. Patent No. 5,599,681, and U.S. Patent No. 6,942,987.

Samples useful in the methods of the invention include any tissue, cell, biopsy, bodily fluid sample, extract, fraction or component thereof, for example samples including proteins or nucleic acids. For example, a sample may be a tissue, a cell, whole blood, serum, plasma, buccal scrape, saliva, cerebrospinal fluid, urine, stool, or bronchoalveolar lavage. In one embodiment, the tissue sample is a gastric tissue sample, a small intestine tissue sample, a large intestine tissue sample, or a skin sample. In a preferred embodiment, a sample is a tumor biopsy sample, which comprises tumor tissue from the biopsy of a tumor.

Samples may be obtained from a subject by any suitable method, including, for example, by the use of a biopsy or by scraping or swabbing an area or by using a needle to aspirate bodily fluids. Methods for collecting various samples are well known in the art. Samples derived from a subject can be obtained by such methods, and may optionally have undergone further processing steps (*e.g.*, freezing, fractionation, fixation, etc.).

Tissue samples suitable for detecting and quantitating MAPK signaling or PI3K signaling may be fresh, frozen, or fixed according to methods known to one of skill in the art. Suitable tissue samples are preferably sectioned and placed on a microscope slide for further analyses. Alternatively, solid samples, *i.e.*, tissue samples, may be solubilized and/or homogenized and subsequently analyzed as soluble extracts.

In one embodiment, a freshly obtained biopsy sample is frozen using, for example, liquid nitrogen or difluorodichloromethane. The frozen sample is mounted for sectioning using, for example, OCT, and serially sectioned in a cryostat. The serial sections are collected on a glass microscope slide. For immunohistochemical staining the slides may be coated with, for example, chrome-alum, gelatine or poly-L-lysine to ensure that the sections stick to the slides. In another embodiment, samples are fixed and embedded prior to sectioning. For example, a tissue sample may be fixed in, for example, formalin, serially dehydrated and embedded in, for example, paraffin. In embodiments where phospho-proteins are to be detected, an inhibitor of dephosphorylation can be used in the process. For example, a reagent such as Phospho-Guard™ can be used to preserve specimens or samples for immunohistochemistry (*e.g.*, Phospho-Guard™ IHC Fixation Kit (Targeted Molecular Diagnostics)).

The skilled man can select an appropriate control sample for the assay in question. For example, a normal tissue sample may serve as a control for tumor tissue (*e.g.*, from the same subject). In some embodiments, a sample from a subject is compared to samples obtained from normal subjects. In some cases, wild-type sequence information or the level of expression of a response marker from samples obtained from normal subjects or normal tissues can serve as controls, avoiding the need to obtain a separate control sample from the subject. For example, when determining whether a subject exhibits a mutation in the *BRAF* gene, a normal tissue sample from the same subject can be used as a control if desired. Optionally, for detection of mutations known to activate BRAF, such as V600E, G464E, G464V, G466A, G466E, G466V, G469A, G469E, E586K, F595L, G596R, L597V, L597R, L597S and V600D, the known wild-type sequence from normal tissues of other subjects can be considered as a control. In the methods of the invention, the control samples in each steps are suitable control samples. Thus, they can be from the same or different tissues and/or subjects, and can be processed in a manner suitable for assessing the mutational status, activity or response marker in question.

Once the sample is obtained any method suitable for detecting and quantitating MAPK or PI3K signaling may be used (either at the nucleic acid or at the protein level). Such methods are well known in the art and include but are not limited to Western blots, Northern blots, Southern blots, immunohistochemistry, ELISA, *e.g.*, amplified ELISA, immunoprecipitation, immunofluorescence, flow cytometry, immunocytochemistry, mass spectrometrometric analyses, *e.g.*, MALDI-TOF and SELDI-TOF, nucleic acid hybridization techniques, nucleic acid reverse transcription methods, and nucleic acid amplification methods. In particular embodiments, the expression or activity level of the AKT, phospho-AKT, BRAF, PTEN, RAF, MEK1, MEK2, ERK1, ERK2, ERK and phospho-ERK proteins is detected on a protein level using, for example, antibodies that specifically bind these proteins, such as the ones described in, for example, U.S. Patent No. 6,982,318, U.S. Publication No. 2002/0150954, and U.S. Publication No. 2007/0020232.

The present invention also provides various methods of treating a cancer in a subject (*e.g.*, a *BRAF* mutated cancer). In some embodiments, the methods of treating a cancer in a subject comprise a) carrying out the steps of a method of prognosing the ability of a zearalenone analog compound to treat a cancer in a subject as described herein, and b) administering a therapeutically effective amount of a composition comprising a zearalenone analog compound to the subject, if the results of step a) are indicative that a zearalenone analog compound has the ability to treat the cancer in the subject.

In other embodiments, the methods of treating a cancer in a subject comprise a) evaluating the results of an assessment of a sample derived from the subject as described herein, and b) administering a therapeutically effective amount of a composition comprising a zearalenone analog compound to the subject, if the results of step a) are indicative that a zearalenone analog compound has the ability to treat the cancer in the subject. For example, in one embodiment, the method of treating a cancer in a subject comprises a) evaluating the results of an assessment of a sample derived from the subject for activated MAPK signaling as compared to a control sample and for wild-type PI3K signaling as compared to a control sample; and b) administering a therapeutically effective amount of a composition comprising a zearalenone analog compound to the subject, if the results of the assessment indicate that the sample exhibits activated

MAPK signaling and wild-type PI3K signaling. In another embodiment, the method of treating a cancer in a subject comprises a) evaluating the results of an assessment of a sample derived from the subject for the presence of a mutation in the *BRAF* gene and for the level of phosphorylated AKT protein in the sample as compared to the total level of AKT protein in the sample or as compared to a control sample; and b) administering a therapeutically effective amount of a composition comprising a zearalenone analog compound to the subject, if the results of the assessment indicate that the sample exhibits a mutation in the *BRAF* gene *(e.g.,* a V600E mutation in the *BRAF* gene) and a low to moderate level of phosphorylated AKT protein. In yet another embodiment, the method of treating a cancer in a subject comprises a) evaluating the results of an assessment of a sample derived from the subject for the presence of mutation in the *BRAF* gene and for the mutational status of the *PTEN* gene; and b) administering a therapeutically effective amount of a composition comprising a zearalenone analog compound to the subject, if the results of the assessment indicate that the sample exhibits a mutation in the *BRAF* gene *(e.g.,* a V600E mutation in the *BRAF* gene) and wild-type *PTEN* sequence. In another embodiment, the method of treating a cancer in a subject comprises a) evaluating the results of an assessment of a sample derived from the subject for the presence of a mutation in the *BRAF* gene; and b) administering a therapeutically effective amount of a composition comprising a zearalenone analog compound to the subject, if the results of the assessment indicate that the sample exhibits a mutation in the *BRAF* gene *(e.g.,* a V600E mutation in the *BRAF* gene). In another embodiment, the method of treating a cancer in a subject comprises a) evaluating the results of an assessment of a sample derived from the subject for the level of phosphorylated AKT protein in the sample as compared to the total level of AKT protein in the sample or as compared to a control sample; and b) administering a therapeutically effective amount of a composition comprising a zearalenone analog compound to the subject, if the results of the assessment indicate that the sample exhibits a low to moderate level of phosphorylated AKT protein. In another embodiment, the method of treating a cancer in a subject comprises a) evaluating the results of an assessment of a sample derived from the subject for the level of expression of AKT protein in the sample as compared to a control sample; and b) administering a therapeutically effective amount of a composition comprising a zearalenone analog compound to the subject, if the results of the assessment indicate that the sample exhibits a low to moderate level of expression of AKT protein. In yet another embodiment, the method of treating a cancer in a subject comprises a) evaluating the results of an assessment of a sample derived from the subject for the activity of AKT protein in the sample as compared to the activity of AKT protein in the sample or as compared to a control sample; and b) administering a therapeutically effective amount of a composition comprising a zearalenone analog compound to the subject, if the results of the assessment indicate that the sample exhibits a low to moderate level of activity of AKT protein. In yet another embodiment, the method of treating a cancer in a subject comprises a) evaluating the results of an assessment of a sample derived from the subject for the mutational status of the *PTEN* gene; and b) administering a therapeutically effective amount of a composition comprising a zearalenone analog compound to the subject, if the results of the assessment indicate that the sample exhibits wild-type *PTEN* sequence. Evaluating the results of an assessment entails a review of results of an assessment of a sample in connection with determining whether or not a subject can benefit from treatment.

*Methods of Determining Whether a Cancer is Sensitive to Treatment with a Zearalenone Analog Compound*

The description describes a method of determining whether a cancer in a subject is sensitive to treatment with a zearalenone analog compound. The method described comprises: a) measuring the level of a response marker in a sample obtained from the subject prior to treatment, wherein the response marker is a cytokine, phospho-ERK, Cyclin D1, phospho-pRB, or p27; b) measuring the level of the response marker in a sample obtained from the subject after treatment; and c) comparing the level of the response marker in the sample obtained prior to treatment with the zearalenone analog compound with the level of the response marker in the sample obtained after treatment with the zearalenone analog compound, wherein a decrease in the level of the cytokine (*e.g.*, IL-6, IL-8), phospho-ERK, Cyclin D1, or phospho-pRB response marker, or an increase in the level of response marker p27, in the sample obtained after treatment as compared to the level of the response marker in the sample obtained prior to treatment is an indication that the cancer in the subject is sensitive to treatment with a zearalenone analog compound. The method described encompasses use of any one or more of such markers to determine sensitivity to treatment. The level of a response marker can be determined by measuring expression levels. The level of the response marker can be measured by measuring the level of the protein or by measuring the level of the corresponding mRNA. The level of expression of a proliferation marker, such as Ki-67 or proliferating cell nuclear antigen (PCNA), is also monitored, whereby decreased expression of a response marker can be correlated decreased cellular proliferation as a result of treatment (*e.g.*, Ki-67 levels decrease as proliferation decreases).

The response marker can be cytokine. Accordingly, the method of determining whether a cancer in a subject is sensitive to treatment with a zearalenone analog compound described comprises, a) measuring the level of expression of a cytokine in a sample obtained from said subject prior to treatment with the zearalenone analog compound; b) measuring the level of expression of said cytokine in a sample obtained from said subject after treatment with the

zearalenone analog compound; c) comparing the level of expression of cytokine in the sample obtained prior to treatment with the zearalenone analog compound with the level of expression of cytokine in the sample obtained after treatment with the zearalenone analog compound, wherein a decrease in the level of expression in the sample obtained after treatment with the zearalenone analog compound as compared to the level of expression in the sample obtained prior to treatment with the zearalenone analog compound is an indication that the cancer in the subject is sensitive to treatment with a zearalenone analog compound. In one embodiment, the level of expression is determined by measuring the level of mRNA. The level of expression can be determined by measuring the level of the cytokine (at the protein level).

Accordingly, the method describes comprises a) measuring the level of a cytokine, *e.g.*, IL-8, IL-1, IL-2, IL-6 or TNFα, in a sample obtained from the subject prior to treatment; b) measuring the level of the cytokine in a sample obtained from the subject after treatment; and c) comparing the level of the cytokine in the sample obtained prior to treatment with the zearalenone analog compound with the level of the cytokine in the sample obtained after treatment with the zearalenone analog compound, wherein a decrease in the level of the cytokine in the sample obtained after treatment as compared to the level of the cytokine in the sample obtained before treatment is an indication that the cancer in the subject is sensitive to treatment with a zearalenone analog compound.

The description describes that the cytokine can be IL-8. The cytokine can be IL-6. The cytokine can be TNFα. The cytokine can be IL-1. The cytokine can be IL-2. The cytokine can be GM-CSF, IL-1 alpha, IL-1 beta, IL-2, IL-3, IL-4, IL-5, IL-6, IL-7, IL-8, IL-10, IL-12, IFN-alpha, IFN-beta, IFN-gamma, MIP-1 alpha, MIP-1 beta, TGF-beta, TNF alpha, or TNF beta.

The level of expression of the cytokine, e.g., IL-8, IL-1, IL-2, IL-6 or TNFα, in the sample can be measured using PCR, standard ELISA or Western blotting. The sample comprises plasma or blood isolated from the patient. The sample comprises a tumor tissue or biopsy sample.

A decrease in the level of expression of a cytokine in the sample obtained after treatment as compared to the level of expression of the cytokine obtained before treatment indicates that the cancer in the subject is sensitive to treatment with a zearalenone analog compound. The level of expression of the cytokine in the sample obtained from the subject after treatment with the zearalenone analog compound is decreased by 20%, 30%, 40%, 50%, 55%, 60%, 65%, 70%, 75%, 80%, 85%, 90%, 95%, 96%, 97%, 98%, or 99% as compared to the level of expression of the cytokine in the sample obtained from the subject before treatment with the zearalenone analog compound.

The description describes a method of determining whether a cancer in a subject is sensitive to treatment with a zearalenone analog compound. The method described includes: a) measuring the level a response marker in a sample obtained from the subject prior to treatment, wherein the response marker is phospho-ERK, Cyclin D1, phospho-pRB, or p27; b) measuring the level of the response marker in a sample obtained from the subj ect after treatment; and c) comparing the level of the response marker in the sample obtained prior to treatment with the zearalenone analog compound with the level of the response marker in the sample obtained after treatment with the zearalenone analog compound, wherein a decrease in the level of the phospho-ERK, Cyclin D1, or phospho-pRB response marker, or an increase in the level of response marker p27 in the sample obtained after treatment as compared to the level of the response marker in the same obtained before treatment is an indication that the cancer in the subject is sensitive to treatment with a zearalenone analog compound.

The level of the response marker, *e.g.*, phospho-ERK, Cyclin D1, or phospho-pRB, in the sample obtained from the subject after treatment with the zearalenone analog compound is decreased by 20%, 30%, 40%, 50%, 55%, 60%, 65%, 70%, 75%, 80%, 85%, 90%, 95%, 96%, 97%, 98%, or 99% as compared to the sample obtained from the subject before treatment with the zearalenone analog compound.

The levels of phospho-pRB or p-ERK are determined relative to total phospho-pRB or p-ERK, respectively.

The level of the response marker, *e.g.*, p27, in the sample obtained from the subject after treatment with the zearalenone analog compound is increased by 20%, 30%, 40%, 50%, 55%, 60%, 65%, 70%, 75%, 80%, 85%, 90%, 95%, 96%, 97%, 98%, 99%, 100%, 125%, 150, 175%, 200%, 250%, 300%, 350%, 400%, 450%, or 500% as compared to the sample obtained from the subject before treatment with the zearalenone analog compound.

The phrase "after treatment," includes any time after any administration of the zearalenone analog compound. For example, samples can be obtained prior to administration and at one or more time points during and/or after administration. For example, if the drug is administered by infusion, blood samples for determination of the level of cytokine in plasma can be obtained prior to infusion, as well as near the end of the infusion, 8 hours after the end of infusion, 24 hours after the end of infusion, 48 hours after the end of infusion, and/or 72 hours after the end of infusion. In another example, tumor biopsy tissue can be obtained prior to treatment and after treatment, and cytokine levels can be analyzed (*e.g.*, by PCR, such as quantitative PCR). In yet another example, tumor biopsy tissue can be obtained prior to treatment and after treatment, and levels response markers such as phospho-ERK, Cyclin D1, phospho-pRB, and/or p27 can be determined (*e.g.*, by immunohistochemistry, such as semi-quantitative IHC). For example, if drug is administered by infusion, biopsies can be obtained prior to infusion and post-infusion (*e.g.*, 24-72 hours post-infusion). If cycles of treatment are used, sampling can be performed in one or more cycles.

The level of the cytokine or response marker in the sample can be measured by assaying cytokine or response

marker levels by ELISA. The level of a cytokine or response marker in a sample can be measured by Western blotting. The level of cytokine or response marker in a sample can be measured by immunohistochemistry.

Suitable samples and methods for obtaining them are described above. Skin biopsies can be used as a surrogate tissue, such that changes in the level of a response marker can be detected in a normal skin sample of a subject treated with a zearalenone analog compound. For example, skin biopsies obtained from an area of normal skin to the level of the subcutaneous tissue can be obtained from a subject prior to treatment and after treatment. Preferably, pre- and post-treatment skin biopsies are obtained form the same anatomic area, but on opposite sides of the body of the subject (*e.g.*, opposite sides of the upper thorax, superclavicular area, upper extremity). For example, if drug is administered by infusion, skin biopsies can be obtained prior to infusion and post-infusion (*e.g.*, 24-72 hours post-infusion). If cycles of treatment are used, sampling can be performed in one or more cycles (*e.g.*, post-infusion in first and/or subsequent cycles). The sample is normal skin and the response marker is selected from the group consisting of phospho-ERK and phospho-pRB.

A general principle of the prognostic methods of the invention involves preparing a sample or reaction mixture that may contain a cytokine or response marker, and a probe, under appropriate conditions and for a time sufficient to allow the cytokine or response marker and probe to interact and bind, thus forming a complex that can be removed and/or detected in the reaction mixture. These assays can be conducted in a variety of ways.

For example, one method to conduct such an assay would involve anchoring the probe onto a solid phase support, also referred to as a substrate, and detecting target marker/probe complexes anchored on the solid phase at the end of the reaction, whereby a sample from a subject, which is to be assayed for presence and/or concentration of a cytokine or response marker, can be anchored onto a carrier or solid phase support. The reverse situation is possible, in which the probe can be anchored to a solid phase and a sample from a subject can be allowed to react as an unanchored component of the assay.

There are many established methods for anchoring assay components to a solid phase. These include, without limitation, marker or probe molecules which are immobilized through conjugation of biotin and streptavidin. Such biotinylated assay components can be prepared from biotin-NHS (N-hydroxy-succinimide) using techniques known in the art (*e.g.*, biotinylation kit, Pierce Chemicals, Rockford, IL), and immobilized in the wells of streptavidin-coated 96 well plates (Pierce Chemical). In certain embodiments, the surfaces with immobilized assay components can be prepared in advance and stored. Other suitable carriers or solid phase supports for such assays include any material capable of binding the class of molecule to which the marker or probe belongs. Well-known supports or carriers include, but are not limited to, glass, polystyrene, nylon, polypropylene, nylon, polyethylene, dextran, amylases, natural and modified celluloses, polyacrylamides, gabbros, and magnetite.

In order to conduct assays with the above mentioned approaches, the non-immobilized component is added to the solid phase upon which the second component is anchored. After the reaction is complete, uncomplexed components may be removed (e.g., by washing) under conditions such that any complexes formed will remain immobilized upon the solid phase. The detection of cytokine or response marker/probe complexes anchored to the solid phase can be accomplished in a number of methods outlined herein.

The probe, when it is the unanchored assay component, can be labeled for the purpose of detection and readout of the assay, either directly or indirectly, with detectable labels discussed herein and which are well-known to one skilled in the art.

It is also possible to directly detect cytokine or response marker/probe complex formation without further manipulation or labeling of either component (marker or probe), for example by utilizing the technique of fluorescence energy transfer (see, for example, Lakowicz et al., U.S. Patent No. 5,631,169; Stavrianopoulos, et al., U.S. Patent No. 4,868,103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

Determination of the ability of a probe to recognize a cytokine or response marker can be accomplished without labeling either assay component (probe or marker) by utilizing a technology such as real-time Biomolecular

Interaction Analysis (BIA) (see, *e.g.*, Sjolander, S. and Urbaniczky, C., 1991, Anal. Chem. 63:2338-2345 and Szabo et al., 1995, Curr. Opin. Struct. Biol. 5:699-705). As used herein, "BIA" or "surface plasmon resonance" is a technology for studying biospecific interactions in real time, without labeling any of the interactants (*e.g.*, BIAcore). Changes in the mass at the binding surface (indicative of a binding event) result in alterations of the refractive index of light near the surface (the optical phenomenon of surface plasmon resonance (SPR)), resulting in a detectable signal

which can be used as an indication of real-time reactions between biological molecules.

Alternatively, analogous prognostic assays can be conducted with a cytokine or response marker and a probe as solutes in a liquid phase. In such an assay, the complexed cytokine or response marker and probe are separated from uncomplexed components by any of a number of standard techniques, including but not limited to: differential centrifugation, chromatography, electrophoresis and immunoprecipitation. In differential centrifugation, cytokine or response marker/probe complexes may be separated from uncomplexed assay components through a series of centrifugal steps, due to the different sedimentation equilibria of complexes based on their different sizes and densities (see, for example, Rivas, G., and Minton, A.P., 1993, Trends Biochem Sci. 18(8):284-7). Standard chromatographic techniques may also be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the cytokine or response marker/probe complex as compared to the uncomplexed components may be exploited to differentiate the complex from uncomplexed components, for example through the utilization of ion-exchange chromatography resins. Such resins and chromatographic techniques are well known to one skilled in the art (see, *e.g.*, Heegaard, N.H., 1998, J. Mol. Recognit. Winter 11(1-6):141-8; Hage, D.S., and Tweed, S.A. J Chromatogr B Biomed Sci Appl 1997 Oct 10;699(1-2):499-525). Gel electrophoresis may also be employed to separate complexed assay components from unbound components (see, *e.g.*, Ausubel et al., ed., Current Protocols in Molecular Biology, John Wiley & Sons, New York, 1987-1999). In this technique, protein or nucleic acid complexes are separated based on size or charge, for example. In order to maintain the binding interaction during the electrophoretic process, non-denaturing gel matrix materials and conditions in the absence of reducing agent are typically preferred. Appropriate conditions to the particular assay and components thereof will be well known to one skilled in the art.

The level of cytokine or response marker mRNA can be determined both by *in situ* and by *in vitro* formats in a sample derived from a subject using methods known in the art. Many expression detection methods use isolated RNA. For *in vitro* methods, any RNA isolation technique that does not select against the isolation of mRNA can be utilized for the purification of RNA from a tissue derived from a subject (see, *e.g.,* Ausubel et al., ed., Current Protocols in Molecular Biology, John Wiley & Sons, New York 1987-1999). Additionally, large numbers of tissue samples can readily be processed using techniques well known to those of skill in the art, such as, for example, the single-step RNA isolation process of Chomczynski (1989, U.S. Patent No. 4,843,155).

The isolated mRNA can be used in hybridization or amplification assays that include, but are not limited to, Southern or Northern analyses, polymerase chain reaction analyses (*e.g.*, quantiative PCR) and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to a mRNA or genomic DNA encoding a marker of the present invention. Other suitable probes for use in the diagnostic assays of the invention are described herein. Hybridization of an mRNA with the probe indicates that the cytokine or response marker in question is being expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the markers.

An alternative method for determining the level of mRNA of a cytokine or response marker in a sample involves the process of nucleic acid amplification, *e.g.*, by rtPCR (the experimental embodiment set forth in Mullis, 1987, U.S. Patent No. 4,683,202), ligase chain reaction (Barany, 1991, Proc. Natl. Acad. Sci. USA, 88:189-193), self sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi et al., 1988, Bio/Technology 6:1197), rolling circle replication (Lizardi et al., U.S. Patent No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic-acid molecule comprising the nucleotide sequence flanked by the primers.

For *in situ* methods, mRNA does not need to be isolated from the sample (*e.g.,* tumor cells) prior to detection. In such methods, a cell or tissue sample is prepared/processed using known histological methods. The sample is then immobilized on a support, typically a glass slide, and then contacted with a probe that can hybridize to mRNA that

encodes the marker.

As an alternative to making determinations based on the absolute expression level of the cytokine or response marker, determinations may be based on the normalized expression level of the cytokine or response marker. Expression levels can be normalized by correcting the absolute expression level of a marker by comparing its expression to the expression of a gene that is not a cytokine or response marker, *e.g.*, a housekeeping gene that is constitutively expressed. Suitable genes for normalization include housekeeping genes such as the actin gene, or epithelial cell-specific genes. This normalization allows the comparison of the expression level in one sample, *e.g.*, a sample derived from a subject with cancer, to another sample, *e.g.*, a sample derived from a subject without cancer, or between samples from different sources.

Alternatively, the expression level can be provided as a relative expression level. For example, to determine a relative expression level of a marker, the level of expression of the cytokine or response marker can be determined for one or more samples, for example 10 or more samples of normal versus cancer cell isolates, preferably 50 or more samples, prior to the determination of the expression level for the sample in question. The mean expression level of each of the genes assayed in the larger number of samples is determined and this is used as a baseline expression level for the cytokine or response marker in normal versus cancer cells. The expression level of the cytokine or response marker determined for the test sample (absolute level of expression) is then divided by the mean expression value obtained for that cytokine or response marker. This provides a relative expression level.

A cytokine or response marker protein can be detected. A preferred agent for detecting a cytokine or response marker can be an antibody capable of binding to such a protein or a fragment thereof, preferably an antibody with a detectable label. Antibodies can be polyclonal, or more preferably, monoclonal. An intact antibody, or a fragment or derivative thereof (*e.g.*, Fab or $F(ab')_2$) can be used. The term "labeled", with regard to the probe or antibody, is intended to encompass direct labeling of the probe or antibody by coupling (*i.e.*, physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

Proteins from cancer cells can be isolated using techniques that are well known to those of skill in the art. The protein isolation methods employed can, for example, be such as those described in Harlow and Lane (Harlow and Lane, 1988, Antibodies: A Laboratory Manual, Cold Spring Harbor Laboratory Press, Cold Spring Harbor, New York).

A variety of formats can be employed to determine whether a sample contains a protein that binds to a given antibody. Examples of such formats include, but are not limited to, enzyme immunoassay (EIA), radioimmunoassay (RIA), Western blot analysis and enzyme linked immunoabsorbant assay (ELISA). A skilled artisan can readily adapt known protein/antibody detection methods for use in determining whether cancer cells express a marker.

In one format, antibodies, or antibody fragments or derivatives, can be used in methods such as Western blots, immunohistochemical or immunofluorescence techniques to detect the expressed proteins. In such uses, it is generally preferable to immobilize either the antibody or proteins on a solid support. Suitable solid phase supports or carriers include any support capable of binding an antigen or an antibody. Well-known supports or carriers include glass, polystyrene, polypropylene, polyethylene, dextran, nylon, amylases, natural and modified celluloses, polyacrylamides, gabbros, and magnetite.

One skilled in the art will know many other suitable carriers for binding antibody or antigen, and will be able to adapt such support for use with the present invention. For example, protein isolated from tumor biopsy tissues can be run on a polyacrylamide gel electrophoresis and immobilized onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

<u>*Zearalenone Analog Compounds*</u>

The present invention is directed to methods for prognosing the ability of a zearalenone analog compound of Formula I to treat cancer in a subject. Zearalenone analog compounds are well known in the art and include those disclosed in U.S. Serial No. 60/951,901, filed on July 25, 2007, 60/951,906, filed on July 25, 2007, U.S. Serial No. 12/180,408, filed on July 25, 2008, U.S. Serial No. 60/951,892, filed on July 25, 2007, U.S. Serial No. 12/180,423, filed on July 25, 2008, U.S. Patent Application No. 10/507,067, U.S. Application Publication No. 2004/0224936, GB 323845, EP606044, WO00/38674, JP840893, WO96/13259, U.S. Patent No. 5,728,726, 5,674,892, and 5,795,910. Recently, it was discovered that zearalenone analog compounds have unique multikinase inhibition profiles and can cross through the blood-brain barrier, which may be useful against specific cancers. For example, it has been shown that zearalenone analog compounds can inhibit MAPKKs, including MEK1 and MEKK1, Growth Factor Receptor Tyrosine Kinases (*e.g.*, FLT-3, TRKB, EPHA2), ABL Tyrosine Kinase, and members of the PAN-SRC tyrosine kinase family (e.g., C-src, Fyn, Lyn, Lck, Yes).

The zearalenone analog compound is a compound of formula (I):

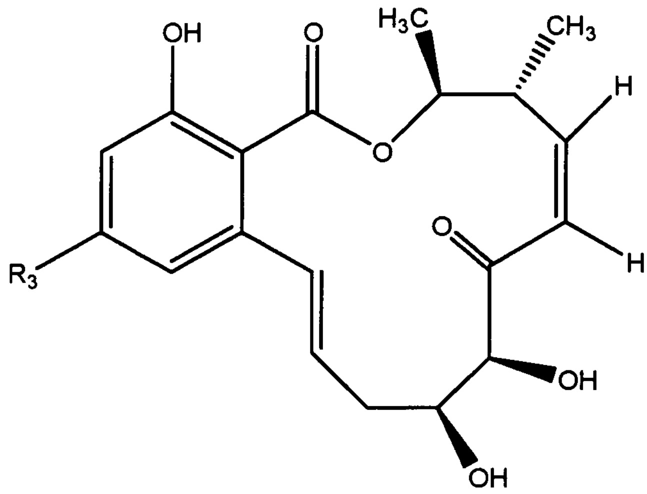

(I)

wherein

$R_3$ is -$NHR_1$, and $R_1$ is $C_1$-$C_3$ alkyl substituted with 0, 1, or 2 hydroxyl moieties, or a pharmaceutically acceptable salt or ester thereof.

In some embodiments, $R_3$ is an unsubstituted $C_{1-3}$ alkyl-amino. In some embodiments, $R_3$ is methylamino. In other embodiments, $R_3$ is ethylamino. In some embodiments, $R_3$ is a $C_{1-3}$ alkyl-amino substituted with one hydroxyl moiety. In some embodiments, $R_3$ is a $C_{1-3}$ alkyl-amino substituted with two hydroxyl moieties. The hydroxyl moieties can be on any of the carbons in the $C_{1-3}$ alkyl chain. Additionally, more than one hydroxyl moiety can be on a single carbon of the $C_{1-3}$ alkyl chain. In some embodiments, there is a hydroxyl moiety on the 2-carbon of the alkyl chain. In some embodiments, $R_3$ is hydroxyethylamino, *e.g.*, 2-hydroxyethylamino. In other embodiments, $R_3$ is dihydroxypropylamino, *e.g.*, 2,3-dihydroxypropylamino. In some embodiments, the $C_{1-3}$ alkyl is an acyclic $C_{1-3}$ alkyl chain.

As used herein, "alkyl" groups include saturated hydrocarbons having one or more carbon atoms, including straight-chain alkyl groups, cyclic alkyl groups (or "cycloalkyl" or "alicyclic" or "carbocyclic" groups) (*e.g.*, cyclopropyl), and branched-chain alkyl groups (*e.g.*, isopropyl).

In some embodiments, the zearalenone analog compound is at least one compound selected from the group consisting of:

| | |
|---|---|
| 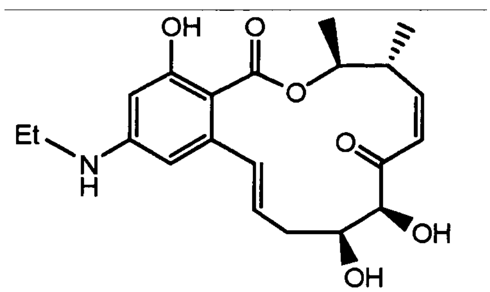 (Compound 106) | 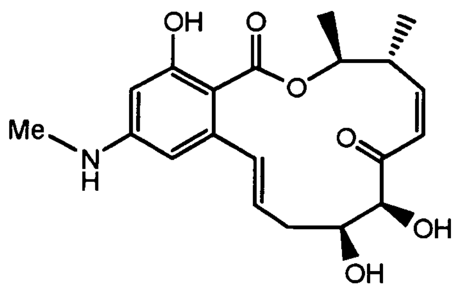 (Compound 091) |
| 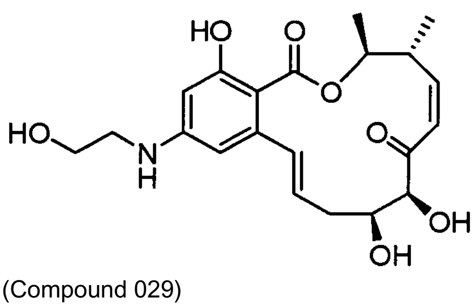 (Compound 029) | 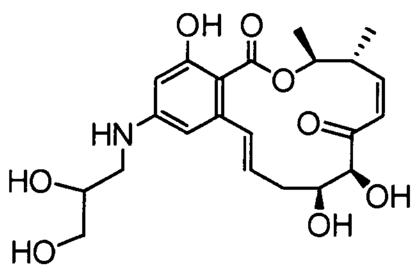 (Compound 114) |

and pharmaceutically acceptable salts or prodrugs thereof.

A zearalenone analog compound may include one or more asymmetric centers, and, thus, can exist in various isomeric forms, *e.g.*, stereoisomers and/or diastereomers. Thus, zearalenone analog compounds and pharmaceutical compositions containing zearalenone analog compounds may be in the form of an individual enantiomer, diastereomer or geometric isomer, or may be in the form of a mixture of stereoisomers. In certain embodiments, the zearalenone analog compounds are enantiopure compounds. In certain other embodiments, mixtures of stereoisomers or diastereomers may be used in the methods of the invention.

Zearalenone analog compounds for use in the methods of the present invention may also have one or more double bonds that can exist as either the Z or E isomer, unless otherwise indicated. The invention additionally encompasses the use of compounds which exist as individual isomers substantially free of other isomers and alternatively, as mixtures of various isomers, *e.g.*, racemic mixtures of stereoisomers.

The compounds for use in the methods of the present invention may further exist as one or a combination of crystalline forms, *e.g.*, polymorphs, solvates or hydrates of compound of formula (I). Various crystalline forms may be identified and/or prepared using different solvents, or different mixtures of solvents for recrystallization; by performing crystallizations at different temperatures; or by using various modes of cooling, ranging from very fast to very slow cooling during crystallizations. Different crystalline forms may also be obtained by heating or melting the compound followed by gradual or fast cooling. The presence of polymorphs may be determined by solid probe NMR spectroscopy, IR spectroscopy, differential scanning calorimetry, powder X-ray diffractogram and/or other techniques.

*Synthetic Methodology*

Zearalenone analog compounds of Formula I useful for practicing the methods of the present invention may be prepared using the synthetic methods described in, for example, U.S. Serial No. 60/951,901, filed on July 25, 2007, U.S. Serial No. 60/951,906, filed on July 25, 2007, U.S. Serial No. 12/180,408, filed on July 25, 2008, U.S. Serial No. 60/951,892, filed on July 25, 2007, U.S. Serial No. 12/180,423, filed on July 25, 2008, WO 05/023792 (*e.g.*, at pages 32-38), and WO 03/076424 *(e.g.,* at pages 28-36). These references in combination with the information contained herein and the additional body of knowledge with regard to macrolide chemistry provides a person of skill in the art with guidance on synthetic strategies, protecting groups, and other materials and methods useful for the synthesis of the zearalenone analog compounds that may be used in the methods of the present invention. For example, the foregoing patent documents provide background information on preparing compounds similar to the zearalenone analog compounds described herein or relevant intermediates, as well as information on formulation, uses, and administration of such compounds.

*Pharmaceutical Compositions*

The zearalenone analog compounds may be administered to a subject using a pharmaceutical composition. Suitable pharmaceutical compositions comprise any one of the compounds described herein (or a pharmaceutically acceptable salt or ester thereof), and optionally comprise a pharmaceutically acceptable carrier. In certain embodiments, these compositions optionally further comprise one or more additional therapeutic agents.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts of amines, carboxylic acids, and other types of compounds, are well known in the art. For example, S.M. Berge, et al. describe pharmaceutically acceptable salts in detail in J. Pharmaceutical Sciences, 66: 1-19 (1977). The salts can be prepared *in situ* during the final isolation and purification of the compounds of the invention, or separately by reacting a free base or free acid function with a suitable reagent, as described generally below. For example, a free base function can be reacted with a suitable acid. Furthermore, where the compounds of the invention carry an acidic moiety, suitable pharmaceutically acceptable salts thereof may, include metal salts such as alkali metal salts, *e.g.* sodium or potassium salts; and alkaline earth metal salts, *e.g.* calcium or magnesium salts. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hernisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

The term "pharmaceutically acceptable ester", as used herein, refers to esters that hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include formates, acetates, propionates, butyrates, acrylates and ethylsuccinates.

As described above, the pharmaceutical compositions may additionally comprise a pharmaceutically acceptable carrier. Such a carrier includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutical compositions and known techniques for the preparation thereof. Except insofar as any conventional carrier medium is incompatible with a zearalenone analog compound, such as by producing any undesirable biological effect or otherwise interacting in a deleterious manner with any other component(s) of the pharmaceutical composition, its use is contemplated to be within the scope of this invention. Some examples of materials which can serve as pharmaceutically acceptable carriers include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil, sesame oil; olive oil; corn oil and soybean oil; glycols; such as propylene glycol; esters such as ethyl oleate and ethyl laurate; agar; buffering agents such as magnesium hydroxide and aluminum hydroxide; alginic acid; pyrogenfree water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Compositions for use in the present invention may be formulated to have any concentration of the zearalenone analog compound desired. In some embodiments, the composition is formulated such that it comprises at least a therapeutically effective amount of the zearalenone analog compound. A therapeutically effective amount is an amount sufficient to achieve the desired therapeutic effect, under the conditions of administration, such as an amount sufficient to treat a cancer. In some embodiments, the composition is formulated such that it comprises an amount that would not cause one or more unwanted side effects. In certain embodiments, compositions are formulated so that the zearalenone analog compound is present at a concentration of between about 1 mg/mL and about 20 mg/mL; between about 1 mg/mL and about 15 mg/mL; between about 1 mg/mL and about 10 mg/mL; between about 2 mg/mL and about 9 mg/mL; between about 3 mg/mL and about 8 mg/mL; between about 4 mg/mL and about 7 mg/mL; between about 4 mg/mL and about 6 mg/mL. In certain embodiments, compositions are formulated such that the compound is present at a concentration of about 5 mg/mL.

*Kits*

The description describes compositions and kits for prognosing the ability of a zearalenone analog compound to treat cancer in a subject or for determining whether a cancer in a subject is sensitive to treatment with a zearalenone analog compound. These kits include one or more of the following: reagents for obtaining and/or preparing samples, *e.g.*, tumor biopsy or blood samples; reagents for determining whether a sample exhibits activated MAPK signaling; reagents for determining whether a sample exhibits wild-type PI3K signaling; probes and reagents for determining whether a sample exhibits a mutation in a gene, *e.g.*, the *RAF* gene, *e.g.*, *BRAF*; probes and reagents for determining whether a sample exhibits a wild-type sequence of a gene, *e.g.,* the *PTEN gene;* reagent for determining DNA hypermethylation in a sample; reagents for determining the level of phosphorylated AKT protein in a sample; reagents for determining the level of expression of AKT protein in a sample; reagents for determining protein activity, *e.g.*, RAF activity (*e.g.*, BRAF activity), MEK1 activity, ERK1 activity, MEK2 activity, ERK2 activity, or AKT activity; reagents for measuring the level of a cytokine, *e.g.*, IL-8, IL-1, IL-2, IL-6, or TNF$\alpha$, in a sample; reagents for measuring the level of another response marker, *e.g.*, phospho-ERK, Cyclin D1, phospho-pRB, or p27, in a sample; and instructions for use.

The kits described may optionally comprise additional components useful for performing the methods of the invention. By way of example, the kits may comprise fluids (*e.g.*, SSC buffer) suitable for annealing complementary nucleic acids or for binding an antibody with a protein with which it specifically binds, one or more sample compartments,

an instructional material which describes performance of a method of the invention, a sample of normal cells, a sample of cancer cells, and the like.

This invention is further illustrated by the following examples which should not be construed as limiting.

## EXAMPLES

### Example 1: *BRAF* Mutation Cells are Sensitive to Treatment with Compound 106

Cell growth inhibition following treatment with a zearalenone analog compound, Compound 106, was assessed in a panel of cancerous cell lines from different tissue types, which carry mutations in *BRAF* and/or *RAS*. A panel of 21 cell lines was tested with varying concentrations of Compound 106. All assays were performed in a 96 well format. Cell viability was assessed after four days following treatment. Cell viability was assessed with a MTS Assay (CellTiter96®AqueousOne Solution Cell Proliferation Assay, Promega) in a panel of cell lines carrying no mutations or various *BRAF* (V600E) and/or *RAS* mutations to determine the effect of *BRAF* and *RAS* genotype on drug sensitivity.

The results are shown in Figure 3A, which is a graph depicting the $IC_{50}$ values for the cell growth inhibition of several cell lines and demonstrates that *BRAF* mutated colorectal cancer, breast cancer and melanoma cancer cell lines were all sensitive to treatment with Compound 106. *BRAF* mutated cell lines, such as the colorectal cancer cell lines HT-29 and Colo-205, breast cancer cell lines MDA-MB-435 and DU4475, and melanoma cell lines SK-MEL-3, SK-MEL-24 and SK-MEL-28 were very sensitive to treatment with the zearalenone analog Compound 106 and had an $IC_{50}$ value of less than 100 nM. These results demonstrate that cell lines with a *BRAF* mutation are more sensitive to treatment with a zearalenone analog compound, *e.g.*, Compound 106.

In a separate experiment, Compounds 091 and 106 were tested in solid cancer cell lines from different tissue types (shown in Figure 3B for 21 cell lines for compound 091 and 19 cell lines for compound 106). All cell lines were introduced into 96-well plates and grown in the absence or continuous presence of 0.3-10000 nM of either compound 091 or compound 106 for 96 hours. Cell growth was assessed using a CellTiter-Glo® Luminescent Cell Viability Assay (Promega) or a methylene blue assay. $IC_{50}$ values were determined as the concentration of a substance which inhibits cell growth by 50% compared to untreated cell populations. Cell lines which carried a *BRAF* V600E mutation were very sensitive to Compounds 091 and 106 in the low-nM or sub-μM concentration range, as shown in Figure 3B.

Cell viability was also assessed in a panel of 31 melanoma cell lines carrying different mutations in *BRAF* following treatment with a zearalenone analog compound, Compound 106. All assays were performed in a 96 well format. Cell viability was assessed after four days following treatment. Cell viability was assessed with a MTS Assay (CellTiter96®AqueousOne Solution Cell Proliferation Assay, Promega) in a panel of cell lines carrying various BRAF mutations to determine the effect *of BRAF* genotype on drug sensitivity. The mutational status of these cell lines and IC50 in nmol/L is depicted in Figure 3C.

An analysis of the results is summarized in Table 2, which illustrates that cell lines carrying mutations in *BRAF* were statistically associated with sensitivity to Compound 106. Sensitivity was defined as an $IC_{50}$ < 100nmol/L. Specifically, of the 20 cell lines carrying mutations in *BRAF* (20 cell lines), 75% of the cell lines were sensitive to Compound 106. Conversely, in the 11 cell lines with wild-type *BRAF*, only 27% of the cell lines were sensitive to Compound 106 (Fisher's Exact test, two tailed = P<0.01).

**Table 2**: Effect of *BRAF* Mutation on Compound 106 Sensitivity in a Panel of 31 Melanoma Cell Lines

| **Cut-Off $IC_{50}$ < 100 nmol/L** | **Cell Lines with Wild-Type *BRAF* Gene (11 cell lines)** | **Cell Lines with Mutant *BRAF* Gene (20 cell lines)** | **Total Cell Lines (31 cell lines)** |
|---|---|---|---|
| % of Sensitive | **3/11 (27%)** | **15/20 (75%)** | 18/31 (58%) |
| % of Less Sensitive | 8/11 (73%) | 5/20 (25%) | 13/31 (42%) |
| Total | 11/11 (100%) | 20/20 (100%) | 31/31 (100%) |

### Example 2: Effect of *BRAF* and *PTEN* Mutations and *BRAF* and phospho-AKT

### Levels on Compound 106 Sensitivity in a Panel of Melanoma Cell Lines

Cell viability was assessed in a panel of melanoma cell lines carrying different mutations in *BRAF*, and *PTEN* following treatment with the MEK inhibitor, Compound 106. The panel of 31 melanoma cell lines was tested with eight concentrations of Compound 106 ranging from 10μmol/L to 0.0003 μmol/L of Compound 106. (The panel of cell lines

also carries mutations in one or more additional genes associated with cancer).

All assays were performed in a 96 well format. Cell viability was assessed after four days following treatment. Cell viability was assessed with a MTS Assay (CellTiter96®AqueousOne Solution Cell Proliferation Assay, Promega) to determine the effect of genotype on drug sensitivity. Individual $IC_{50}$ values are shown in Figure 3C.

Further analysis of the results was conducted in which sensitivity was defined as an $IC_{50}$ < 500nmol/L. The results of this analysis are summarized in Table 3 and reveal that sensitivity to Compound 106 was statistically associated with wild-type *PTEN* status. Specifically, of the 23 cell lines with wild-type *PTEN,* 12 cell lines were sensitive to Compound 106. Conversely, in the 8 cell lines with mutant *PTEN* or loss of *PTEN,* only 3 cell lines were sensitive (Fisher's Exact test, two tailed = P<0.01).

Phospho-AKT expression levels were also assessed. Table 3 illustrates that phospho-AKT expression affects sensitivity to Compound 106.

**Table 3**: *PTEN* Mutation/Deletion or p-AKT Levels Modulate Sensitivity to Compound 106 in 20 Melanoma Cell Lines Carrying *BRAF* Mutations

| | % of Cell lines with $IC_{50}$ <100 nmol/L | *BRAF* Wild Type (11 cell lines) | *BRAF* Mutant (20 cell lines) | Total |
|---|---|---|---|---|
| $P_{TEN}$ gene | Wild Type (23 cell lines) | 3/9 (33%) | **12/14 (86%)** | 15/23 (65%) |
| | Mutant (8 cell lines) | 0/2 (0%) | **3/6 (50%)** | 4/8 (50%) |
| | Total | 3/11 (27%) | 15/20 (75%) | 19/31 (61%) |
| p-AKT expression | Low (GO-01) | **3/10 (30%)** | **11/14 (79%)** | 14/24 (58%) |
| | High (G2-G3) | **0/1 (0%)** | **4/6 (67%)** | 4/7 (57%) |
| | Total | 3/11 (27%) | 15/20 (75%) | 18/31 (58%) |

In a separate experiment, Compound 106 was tested in a panel of *BRAF* mutant (V600E) melanoma cell lines. Some of the cell lines contained mutations in the *PTEN* gene, as shown in Figure 3D. (This set of cell lines represents 17 additional cell lines relative to Figure 3C.) The melanoma cell lines were introduced into 96-well plates and grown in the absence or continuous presence of compound 106. $IC_{50}$ values were determined as the concentration of a substance which inhibits cell growth by 50% compared to untreated cell populations. Cell lines which carried a *BRAF* mutation and wild-type *PTEN* status were very sensitive to Compound 106, as shown in Figure 3D.

These results demonstrate that cell lines with activated MAPK signaling and wild-type PI3K signaling are more sensitive to a zearalenone analog compound, *e.g.*, Compound 106.

**<u>Example 3: AKT Phosphorylation Status Affects Compound 106 Sensitivity in a Panel of Melanoma Cell Lines</u>**

In order to determine whether Compound 106 resistance is associated with constitutive AKT phosphorylation as a result of activation of the PI3K signaling pathway, a panel of 10 *BRAF* mutated melanoma cell lines and a *BRAF* wild-type glioblastoma cancer cell line, SF-295, were tested using different concentrations of Compound 106 ranging from 10μmol/L to 0.0003 μmol/L.

All assays were performed in a 96 well format. For these cell lines, protein lysates were collected in a separate experiment, which allowed for the evaluation of the constitutive level of AKT phosphorylation and correlation between the level of phosphorylated AKT (pAKT) and Compound 106 sensitivity. Cell growth was assessed using CellTiter-Glo® Luminescent Cell Viability Assay (Promega). Phosphorylated AKT protein was analyzed by Western blotting, with total AKT being used as a loading control.

Elevated expression of pAKT, which can reflect activation of the PI3K signaling pathway, was observed in cell lines which are resistant to Compound 106, including RPMI-7951 and SF-295 (see, *e.g.*, Figure 2). Elevated expression of pAKT was not observed in cell lines which are sensitive to Compound 106. These data demonstrate that a zearalenone analog compound, *e.g.*, Compound 106, has the ability to treat cancer in cell lines with activated MAPK signaling and wild-type PI3K signaling (see, *e.g.*, Figure 4). These data further demonstrate that, a zearalenone analog compound, *e.g.*, Compound 106, has the ability to treat cancer in cancer cell lines containing a mutated *BRAF* and a wild-type *PTEN.* Taken together, these techniques provide a method of prognosing the ability of a zearalenone analog compound to treat cancer in a subject.

## Example 4 (not an example of the invention): *BRAF* Mutated Cancer Cells Produce Pro-Inflammatory Cytokine Interleukin-8 (IL-8)

In order to determine whether IL-8 is produced by melanoma cells, fourteen melanoma cell lines carrying the V600E *BRAF* mutation were evaluated *in vitro* for IL-8 cytokine expression levels by ELISA. As demonstrated in Figure 5, LOX, SK-MEL-24, UACC-62, and COLO-829 cell lines, which carry a *BRAF* mutation, all showed high IL-8 expression at the protein level.

## Example 5 (not an example of the invention): Change in Plasma IL-8 in Tumor Bearing Mice Treated with Compound 106

In order to determine whether plasma IL-8 levels could be measurable in tumor xenograft bearing mice and whether plasma IL-8 levels change after treatment with Compound 106, COLO-829 and UACC-62 melanoma xenografts were established as Compound 106 sensitive xenografts in female nude mice. SF-295 human glioblastoma (*BRAF* wild-type) xenografts were established as an Compound 106 resistant tumor model in female nude mice. Female athymic NU/NU mice were inoculated subcutaneously with COLO-829, UACC-62 human melanoma cancer cells, or SF-295 human glioblastoma cells. Those animals that developed tumors of approximately 250 $mm^3$ were selected and randomized to two groups. The experiment consisted of a vehicle-treated and 40 mg/kg Compound 106-treated groups of 8 mice per group on the first day of treatment. Compound 106 was administered intravenously (i.v.) on a QD x 4 (every day for a total of four injections) dosing schedule. The subcutaneous tumor volumes were measured on the first day of treatment and 24 hours after the fourth treatment. Heparinized blood was collected from these mice 24 hours after the fourth treatment with Compound 106 using a cardiac puncture, and plasma was isolated. The levels of human IL-8 in the plasma were determined by ELISA.

As demonstrated in Figure 7, at 40 mg/kg, Compound 106 almost completely blocked plasma IL-8 levels 24 hours after the fourth dosing in the xenografts which were sensitive to Compound 106, namely UACC-62 and COLO-829. In contrast, treatments with Compound 106 did not change the plasma IL-8 levels in SF-295 tumor bearing mice, which were resistant to treatment with Compound 106. Based on the foregoing results, it is evident that a reduction in plasma IL-8 may serve as a surrogate marker to measure the response of a tumor to a zearalenone analog compound, *e.g.*, Compound 106.

## Example 6 (not an example of the invention): Plasma IL-8 Levels can be used to Detect a Response to Compound 106

In order to determine whether plasma IL-8 can be detected in plasma, or blood, from human cancer patients, six plasma samples from melanoma patients were obtained from a tumor tissue bank (Asterand). Six melanoma tissues from metastatic sites were also obtained from the same patients. As a control, six plasma samples were also obtained from healthy volunteers. IL-8 plasma levels were determined by ELISA. *BRAF* mutations were detected by PCR analysis.

As shown in Table 4, IL-8 was detected in all six clinical samples tested. All six cancer patients had a *BRAF* mutation, as indicated by the PCR analysis.

**Table 4**: Plasma IL-8 Levels in Advanced Melanoma Patients

| Sample ID | Sex | Tissue | ***BRAF* Mutation** (indicated by the bolded, underlined nucleotide) | Plasma IL-8 (pg/mL) |
|---|---|---|---|---|
| 30517 | Female | Lymph Node | **A**GG | 26.5 |
| 30529 | Male | Soft Tissue | G**A**G | 12.0 |
| 31068 | Female | Lymph Node | A**A**G | 13.4 |
| 40968 | Male | Small Intestine | G**A**G | 48.2 |
| 48323 | Female | Lymph Node | G**A**G | 41.7 |
| 48617 | Female | Lymph Node | G**A**G | 155.0 |
| Normal | | | GTG | <3 |
| <3; below detection limit | | | | |

These results demonstrate that IL-8 can be detected in plasma from patients with advanced stage melanoma.

## Example 7: Zearalenone Analog Compounds Decrease Protein Levels of IL-8 and IL-6 in Cancer Cell Lines and Affect Secretion of IL-8 and IL-6 by *BRAF* Mutated Cells

The purpose of this study was to investigate the effects of a zearalenone analog compound, such as Compound 106, *in vitro* on the secretion of IL-8 and IL-6 by *BRAF*-mutated LOX melanoma cells (Davies H. et al., "Mutations of the BRAF gene in human cancer", Nature, 417: 949-954 (2002)).

Compound 106 (5.0 mg) was weighed and dissolved in 100% anhydrous DMSO (dimethyl sulfoxide, Sigma-Aldrich®, St. Louis, MO) to produce a 10 mmol/L stock solution of 3.89 mg/mL. Aliquots of the 10 mmol/L stock solution were stored at - 80°C. Then, on each day of an experiment, an aliquot of the stock solution was thawed and diluted 1: 10 by adding RPMI-1640 culture medium to obtain a 1 mmol/L solution. Four serial 1:10 dilutions were made from 1 mmol/L working solution by adding 10% DMSO in RPMI-1640 culture medium to obtain 4 additional working solutions. Each of these working stock solutions was further diluted with culture media to obtain diluted working solutions ranging from 1 nmol/L to 10,000 nmol/L. Five mL of each of these diluted working solutions was added to each dish.

LOX human melanoma cells were originally obtained from the DCTD Tumor Repository (Frederick, MD). The cells were grown in monolayer cultures in RPMI-1640 growth media containing 10% fetal bovine serum (FBS) at 37°C in a 5% $CO_2$ humidified incubator.

LOX human melanoma cells were plated at 1 x $10^6$ cells into 100 mm dishes. After 48 hours, cells were washed three times with phosphate buffered saline (PBS) and were cultured with RPMI-1640 medium containing 0.1% FBS for another 24 hours. After removing the cell culture medium, fresh RPMI-1640 medium containing Compound 106 (1, 10, 100, 1000, or 10,000 nmol/L) was added to each culture dish and incubated for 24 hours (see Table 5). The control received 0.1% DMSO in RPMI-1640 culture medium alone to measure spontaneous secretion of IL-6 and IL-8. Three separate experiments were performed in duplicate to calculate the mean $\pm$ SEM.

**Table 5**: Sample Combinations for Determining IL-6 and IL-8 Secretion

| Sample | Cell number | Treatment |
|---|---|---|
| 1 | No cells (blank) | 0.1% DMSO in culture medium (vehicle) |
| 2 | No cells (blank) | 0.1% DMSO in culture medium (vehicle) |
| 3 | 1 x $10^6$ | 0.1% DMSO in culture medium (vehicle) |
| 4 | 1 x $10^6$ | 0.1% DMSO in culture medium (vehicle) |
| 5 | 1 x $10^6$ | 1 nmoL Compound 106 |
| 6 | 1 x $10^6$ | 1 nmoL Compound 106 |
| 7 | 1 x $10^6$ | 10 nmoL Compound 106 |
| 8 | 1 x $10^6$ | 10 nmoL Compound 106 |
| 9 | 1 x $10^6$ | 100 nmoL Compound 106 |
| 10 | 1 x $10^6$ | 100 nmoL Compound 106 |
| 11 | 1 x $10^6$ | 1,000 nmoL Compound 106 |
| 12 | 1 x $10^6$ | 1,000 nmoL Compound 106 |
| 13 | 1 x $10^6$ | 10,000 nmoL Compound 106 |
| 14 | 1 x $10^6$ | 10,000 nmoL Compound 106 |

After a 24 hour incubation with Compound 106 or 0.1% DMSO, 5 mL of cell culture supernatant was collected. Any particulate material was removed by centrifugation. All samples were stored in the -80°C freezer until analysis. Human IL-6 or IL-8 was determined using an ELISA kit (Human IL-8 ELISA Set, Catalog No. 555244, BD Biosciences, San Diego, CA and Human IL-6 ELISA Set, Catalog No. 555220, BD Biosciences, San Diego, CA) with the assay procedure provided by the kits. Absorbance was read on a VERSAMAX™ (Molecular Devices, now part of MDS Analytical Technologies, Sunnyvale CA).

After removing the cell culture supernatant, 1.0 mL of trypsin was added to the cells for 3 minutes, and then 4 mL of media containing 10% FBS was added for cell counting. Cells were counted using a hematocytometer (Bright Line Counting Chamber, Hausser Scientific, Horsham, PA) with a depth of 0.1 mm. The total number of cells was calculated as follows:

$$\text{Total number of cells in 5 mL} = C \times 10^4 \text{ /mL} \times 5\text{mL (C: actual cell count in 1 mm}^2\text{)}$$

The level of IL-6 or IL-8 was determined as ng/1 x $10^6$ cells. Inhibition of IL-6 or IL-8 secretion by Compound 106 was expressed as percent of control using the formula below:

$$\text{Percent of control} = \text{(value of treatment – mean value of blank)/(value of control – mean value of blank)} \times 100$$

The software, Graphpad Prism® (ver. 4, San Diego, CA) was used for calculation of mean $IC_{50}$ values.

LOX human melanoma cells, which carry a *BRAF* mutation, spontaneously secreted IL-6 (15.5 ± 5.1 ng/million cells) and IL-8 (104.7 ± 38.9 ng/million cells) up to 24 hours, respectively (Table 6 and Table 7). The protein levels of IL-6 and IL-8 were decreased by Compound 106 in the LOX melanoma cell line with mean calculated $IC_{50}$ values of 21.8 and 10.5 nmol/L, respectively, in a concentration dependent manner (Figure 6 and Tables 8 and 9). These results demonstrate that changes in levels of these proteins can serve as pharmacodynamic markers to measure biological responses to a zearalenone analog compound, such as Compound 106.

**Table 6**: Inhibitory Effect of Compound 106 on Spontaneous IL-6 Secretion in Three Separate Experiments

| Compound 106 (nmol/L) | IL-6 (pg/million cells) | | | IL-6 (ng/million cells) | |
|---|---|---|---|---|---|
| | Experiment 1 | Experiment 2 | Experiment 3 | Mean | SEM |
| 0 | 11998 | 25610 | 8905 | 15.5 | 5.1 |
| 1 | 5890 | 13741 | 10140 | 9.9 | 2.3 |
| 10 | 3253 | 12105 | 10745 | 8.7 | 2.8 |
| 100 | 347 | 643 | 485 | 0.5 | 0.1 |
| 1,000 | 547 | 986 | 257 | 0.6 | 0.2 |
| 10,000 | 165 | 337 | 132 | 0.2 | 0.1 |

**Table 7**: Inhibitory Effect of Compound 106 on Spontaneous IL-8 Secretion in Three Separate Experiments

| Compound 106 (nmol/L) | IL-8 (pg/million cells) | | | IL-8 (ng/million cells) | |
|---|---|---|---|---|---|
| | Experiment 1 | Experiment 2 | Experiment 3 | Mean | SEM |
| 0 | 105021 | 172037 | 37172 | 104.7 | 38.9 |
| 1 | 112318 | 213228 | 60753 | 128.8 | 44.8 |
| 10 | 47049 | 90632 | 42942 | 60.2 | 15.3 |
| 100 | 3079 | 3249 | 3101 | 3.1 | 0.1 |
| 1,000 | 3390 | 2273 | 1494 | 2.4 | 0.6 |
| 10,000 | 5179 | 1384 | 2179 | 2.9 | 1.2 |

**Table 8**: Inhibitory Effect of Compound 106 on Protein Level of IL-6 in Three Separate Experiments

| Compound 106 (nmol/L) | Percent (%) of control | | | Mean % of control | SEM | n |
|---|---|---|---|---|---|---|
| | Experiment 1 | Experiment 2 | Experiment 3 | | | |
| 0.0 | 100 | 100 | 100 | 100 | 0 | 3 |
| 1 | 46.1 | 53.6 | 115.0 | 72.2 | 20.9 | 3 |
| 10 | 21.5 | 47.1 | 121.3 | 65.0 | 28.4 | 3 |

(continued)

| Compound 106 (nmol/L) | Percent (%) of control | | | Mean % of control | SEM | n |
|---|---|---|---|---|---|---|
| | Experiment 1 | Experiment 2 | Experiment 3 | | | |
| 100 | 2.9 | 2.5 | 5.5 | 3.6 | 0.9 | 3 |
| 1,000 | 4.6 | 3.8 | 2.9 | 3.8 | 0.5 | 3 |
| 10,000 | 1.3 | 1.3 | 1.6 | 1.4 | 0.0 | 3 |

**Table 9**: Inhibitory Effect of Compound 106 on Protein Level of IL-8 in Three Separate Experiments

| Compound 106 (nmol/L) | Percent (%) of control | | | Mean % of control | SEM | n |
|---|---|---|---|---|---|---|
| | Experiment 1 | Experiment 2 | Experiment 3 | | | |
| 0.0 | 100 | 100 | 100 | 100 | 0 | 3 |
| 1 | 106.9 | 123.9 | 163.4 | 131.4 | 16.7 | 3 |
| 10 | 44.8 | 52.7 | 115.5 | 71.0 | 22.4 | 3 |
| 100 | 2.9 | 1.9 | 8.3 | 4.4 | 2.0 | 3 |
| 1,000 | 3.2 | 1.3 | 4.0 | 2.9 | 0.8 | 3 |
| 10,000 | 4.9 | 0.8 | 5.9 | 3.9 | 1.6 | 3 |

Moreover, as demonstrated in Figure 6, treatment with Compound 106 almost completely abrogated IL-6 ($IC_{50}$ of 21.8 nmol/L) and IL-8 ($IC_{50}$ of 10.5 nmol/L) protein expression in the LOX melanoma cell line, which carries the V600E *BRAF* mutation and is wild-type for *PTEN.* Based on the foregoing results, it is evident that a reduction in plasma IL-8 and/or IL-6 levels serves as a surrogate marker to measure the response of a tumor to a zearalenone analog compound, *e.g.*, Compound 106.

**Example 8 (not an example of the invention): Levels of IL-8 and IL-6 Indicate Whether a Cancer is Sensitive to Treatment with a Zearalenone Analog Compound**

In order to determine whether a cancer is sensitive to treatment with a zearalenone analog compound, the level of IL-6 or IL-8 is evaluated at various times in plasma using an ELISA assay. Blood is drawn prior to treatment, during treatment and after treatment. For example, blood can be drawn prior to infusion, just before the end of infusion, 8, 24, 48 and 72 hours after the end of infusion. This can be done for one or more infusions of a treatment cycle.

In addition, quantitative PCR is performed on tumor biopsy tissue obtained prior to treatment and post-infusion to determine mRNA levels of IL-6 and/or IL-8.

In each case above, the level of IL-6 or the level of IL-8 is used as a pharmacodynamic marker to assess the effect of zearalenone analog compound (as a surrogate marker of drug efficacy). A decrease in IL-6 or IL-8 levels during or post-treatment indicates that the cancer is sensitive to treatment with a zearalenone analog compound.

**Example 9: Protein Levels of Response Markers After Treatment with Compound 106**

In order to identify markers that could serve as response markers for determining whether a cancer in a subject is sensitive to treatment with a zearalenone analog compound, phospho-ERK, total ERK protein, phospho-pRB, total pRB, and Cyclin D1 protein levels in a cell line sensitive to Compound 106 (*e.g.*, SK-MEL-28) and cell lines resistant to Compound 106 (*e.g.*, AU-565) were examined by Western blotting. 2 x $10^6$ cancer cells (SK-MEL-28 and AU-565) were plated into a 100 mm dish and incubated for two days. Test compound, *e.g.*, Compound 106, was then added to each dish for 24 hours at a concentration ranging from 10 nmol/L to 3 μmol/L. Culture medium was added to the cells as a control. SK-MEL-28 and AU-565 cell lysate proteins were subjected to SDS-PAGE under reducing condition and immunoblotted with an antibody against phospho-ERK1/2 (catalog #9101, Cell Signaling Technology®, Danvers, MA), phospho-pRB (catalog #9307, Cell Signaling Technology®, Danvers, MA), or Cyclin D1 (catalog# sc-8396, Santa Cruz, CA). ERK1 (catalog# sc-93, Santa Cruz, CA) or pRb (catalog # sc-102, Santa Cruz, CA) antibody was used for the detection of the total amount of protein.

Expression levels of ERK and proteins which are important for the $G_1$/S transition (Cyclin D1, p27, phospho-pRB) were examined by Western blotting in the sensitive cell line, SK-MEL-28, and in the resistant cell line, AU-565. As indicated in Figure 8, protein levels of phospho-ERK were inhibited by compound 106 in both the sensitive and resistant cell lines in a concentration dependent manner. Importantly, protein levels of Cyclin D1, which phosphorylates retinoblastoma protein (pRB) were decreased in parallel with a decrease in phospho-pRB protein in sensitive cell lines. Protein levels of p27, an inhibitor of CDK, were increased in the presence of compound 106 in the sensitive line. These results demonstrate that zearalenone analog compounds transcriptionally inhibit Cyclin D1 expression followed by inhibition of phosphorylation of pRB protein, leading to cell cycle arrest. Thus, these markers can serve as pharmacodynamic markers for response to treatment with a zearalenone analog compound, *e.g.*, Compound 106.

**Example 10: Immunohistochemistry of Human Melanoma Tissues**

Formalin-fixed, paraffin-embedded human melanoma tumor samples were obtained and processed for immunohistochemistry (IHC) with the antibodies described in Table 10. IHC procedure (1) described below was used if the incubation with primary antibody was for 1 hour as indicated in Table 10. IHC procedure (2) described below was used if the incubation with primary antibody was overnight as indicated in Table 10.

**Table 10**: Antibodies, Retrievals and Incubation Conditions for Immunohistochemistry

| Ab name | Source, Cat# | Species Type | Retrieval (96 °C, 20 min) | Primary Ab, final conc | Primary Ab, incubation | Localization |
|---|---|---|---|---|---|---|
| ERK 1/2 | Cell Signaling ® Cat. No. 4695 | Rabbit Polyclonal IgG[1] | Citrate pH 6 | 0.44 μg/mL | Overnight at 4°C | cytoplasm |
| p-ERK 1/2 Thr202/Tyr 204 | Sigma®, Cat. No. M9692 | Mouse Monoclonal IgG1[2] | EDTA pH 8.0 | 3 μg/mL | 1 hr, Rm temp | nuclear |
| AKT (pan-specific) | Cell Signaling ® Cat. No. 4691 | Rabbit Polyclonal IgG[3] | Citrate pH 6 | 0.5 μg/mL | Overnight at 4°C | cytoplasm |
| Cyclin D1 | Lab Vision, Cat. No. RB-9041 | Rabbit, Epitope Specific IgG[4] | Citrate pH 6 | 2 μg/mL | 1 hr, Rm temp | nuclear |
| p-AKT (Ser473) | Cell Signaling ®Cat. No. 3787 | Rabbit Polyclonal IgG[5] | Citrate pH 6 | 0.5 μg/mL | Overnight at 4 °C | cytoplasm |
| p-RB (Ser780) | Sigma®, Cat. No. R6275 | Rabbit Polyclonal IgG[6] | EDTA pH 8.0 | 0.6 μg/mL | 1 hr, Rm temp | nuclear |
| RB | Cell Signaling ®Cat. No. 9309 | Mouse Monoclonal IgG2a[7] | Citrate pH 6 | 1.4 μg/mL | 1 hr, Rm temp | nuclear |

(continued)

| Ab name | Source, Cat# | Species Type | Retrieval (96 °C, 20 min) | Primary Ab, final conc | Primary Ab, incubation | Localization |
|---|---|---|---|---|---|---|
| Ki67 | Lab Vision, Cat. No. RB-9043 | Rabbit, Epitope Specific IgG | Citrate pH 6 | 0.67 μg/mL | 1 hr, Rm temp | nuclear |

[1] Rabbit mAb 137F5 detects endogenous levels of total p44/42 MAP kinase (ERK1/ERK2) protein.
[2] Monoclonal anti-MAP kinase antibody reacts specifically with the diphosphorylated form of MAP kinase (ERK-1 and ERK-2).
[3] AKT (pan) (C67E7) rabbit mAb detects endogenous levels of total AKT protein.
[4] This rabbit mAb detects a C-terminal epitope of Cyclin D1.
[5] Rabbit mAb 736E11 detects AKT1 if phosphorylated at serine 473, and detects AKT2 and AKT3 if phosphorylated at the corresponding site.
[6] Anti-phospho-Retinoblastoma (pSer780) antibody recognizes RB phosphorylated at Ser780 and does not react with non-phosphorylated RB.
[7] Mouse anti-RB mAb 4H1 detects endogenous levels of total RB protein.

**a. IHC Procedure (1)**

IHC Procedure (1) was used for all Primary Antibodies requiring a 1-hour incubation at Room Temperature. IHC methods were conducted using Lab Vision Autostainer 360 and Lab Vision LP-AP detection kits (UltraVision LP Large Volume Detection System AP Polymer (Ready-To-Use), Catalog No. TL-125-AL). Human melanoma sections (5 μ thickness) were deparafinized and rehydrated. Epitope retrieval was conducted in Lab Vision Pretreatment Module, 96°C, no boiling cycle, 20 mintues, followed by a 20 minute cooling period (to 75°C). Retrieval buffers were either EDTA or Citrate, as indicated in Table 10: (a) EDTA buffer, pH 8.0 (Lab Vision, Cat. No. TA-250-PM2X); or (b) Citrate buffer, pH 6.0 (Lab Vision, Cat. No. TA-250-PM1X).

Slides were transferred to the Autostainer and the following program was used: Pre-rinse (TBS-Tween buffer, Lab Vision, Cat. No. TA-999-TT); Non-specific protein block, 10 min (UV Block, part of the LP-AP kit); Primary Antibody: 60 min; 3 rinses in TBS-Tween Buffer; Antibody Enhancer (part of the LP-AP kit),10 min; 3 rinses in TBS-Tween Buffer; Labeled Polymer - AP conjugated (part of the LP-AP kit), 15 min; 3 rinses in TBS-Tween Buffer; Substrate: Fast Red (prepared from the Fast Red substrate system (Lab Vision, Cat. No. TA-125-AF)); 1 rinse in water; and 2 rinses in distilled water.

Slides were counterstained with hematoxylin Gill III (VWR®, Cat. No. 15204-268). (This chromogen is not solvent resistant, and cannot be used with xylene / alcohols). The hematoxylin counterstain procedure was as follows: Hematoxylin, 30 sec; $dH_2O$; Tap water rinse, 5 min; Bluing reagent, 30 sec; Tap water rinse, 5 min; $dH_2O$.

Slides were cover-slipped manually using Vision Mount Mounting Media (Lab Vision, Cat. No. TA-125-UG). This mounting medium is compatible with Lab Vision's Fast Red. Number 1.5 coverslip glass was used for optimal microscope analysis (with objectives optimized for No. 1.5 coverslips). Analyses were done within 1 week of IHC staining, because Lab Vision Fast Red is not permanent.

**b. IHC Procedure (2)**

IHC Procedure (2) was used for all Primary Antibodies requiring an overnight (18 hr) incubation at 4 °C.

IHC methods were conducted using Lab Vision Autostainer 360 and Lab Vision LP-AP detection kits (UltraVision LP Large Volume Detection System AP Polymer (Ready-To-Use), Cat. No. TL-125-AL). Human melanoma sections (5 μ thickness) were deparafinized and rehydrated. Epitope retrieval was conducted in Lab Vision Pretreatment Module, 96 °C, no boiling cycle, 20 min, followed by a 20 min cooling period (to 75 °C). Retrieval buffers were either EDTA or Citrate, as indicated in Table 10: (a) EDTA buffer, pH 8.0 (Lab Vision, Cat. No. TA-250-PM2X); or (b) Citrate buffer, pH 6.0 (Lab Vision, Cat. No. TA-250-PM1X).

The steps up to and including the primary Ab were done manually: Pre-rinse (TBS-Tween buffer, Lab Vision, Cat. No. TA-999-TT); Non-specific protein block, 10 min (UV Block, part of the LP-AP kit); Primary Antibody: overnight (18 hours) at 4 °C in the humidity chamber (to prevent the slides from drying out). On overnight incubations, a hydrophobic barrier pen was used to draw an oval around the tissue to prevent the Ab from running off the slide during the long incubation period.

Slides were then transferred to the Autostainer and the following program was used: 3 rinses in TBS-Tween Buffer; Antibody Enhancer (part of the LP-AP kit), 10 min; 3 rinses in TBS-Tween Buffer; Labeled Polymer - AP conjugated

(part of the LP-AP kit), 15 min; 3 rinses in TBS-Tween Buffer; Substrate: Fast Red (prepared from the Fast Red substrate system, Lab Vision, Cat. No. TA-125-AF); 1 rinse in water; and 2 rinses in distilled water.

Slides were counterstained with hematoxylin Gill III (VWR Cat. No. 15204-268). (This chromogen is not solvent resistant, and cannot be used with xylene / alcohols). The hematoxylin counterstain procedure was as follows: Hematoxylin, 30 sec; $dH_2O$; Tap water rinse, 5 min; Bluing reagent, 30 sec; Tap water rinse, 5 min; $dH_2O$.

Slides were coverslipped manually using Vision Mount Mounting Media (Lab Vision, Cat. No. TA-125-UG). Number 1.5 coverslip glass was used for optimal microscope analysis (with objectives optimized for No. 1.5 coverslips). Analyses were done within 1 week of IHC staining, because Lab Vision Fast Red is not permanent.

The foregoing procedures and conditions were effective in detecting ERK 1/2, phospho-ERK, AKT, Cyclin D1, p-AKT, p-RB, RB and Ki67 in human melanoma tumor samples (commercially available clinical samples).

## Example 11: Time-dependent Inhibition of ERK and Phospho-pRb Phosphorylation in LOX Xenografts

LOX human melanoma cells, which carry the V600E *BRAF* mutation, were originally obtained from the DCTD Tumor Repository (Frederick, MD). The cells were grown in monolayer cultures in DCTD-recommended growth media at 37 °C in a 5% $CO_2$ humidified incubator. On the day of subcutaneous (s.c.) injections of the cells, growth medium was removed, flasks were washed with PBS, and cells were collected with trypsinization. The cells were washed and collected by centrifugation, and then resuspended in ice-cold PBS at a concentration of $1x10^7$ cells/mL. The cells ($1x10^6$ cells) were inoculated s.c. in female athymic NU/NU mice near the right axillary area using a 27-gauge needle with a volume of 0.1 mL, and allowed to grow. Compound **106** was administered intravenously (i.v.) on a single dosing at the dosage level of 40 mg/kg with a volume of injection 0.1 mL per 10g body weight. Then, the mice were euthanized at different time points including 0, 1, 2, 4, 8, 12, 24, 48, 72 h after dosing. The tumor tissue was dissected and fixed with 10% formalin for immunohistochemistry.

For immunohistochemical detection of phospho-ERK 1/2, rabbit mAb 20G11 was used at a final concentration of 0.36 μg/ml (Cell Signaling, Cat. No. 4376). This mAb detects endogenous levels of p44 and p42 MAP Kinase (ERK1 and ERK2) when dually phosphorylated at Thr202 and Tyr204 of ERK1 (Thr185 and Tyr187 of ERK2), and singly phosphorylated at Thr202. Conditions were essentially as described in IHC Procedure (2), with incubation overnight (18 hr) at 4 °C and EDTA retrieval buffer. Secondary reagent was goat anti-rabbit antibody, conjugated to alkaline phosphatase.

Results are shown in Figure 9A. Phospho-ERK staining was observed at the 0 hour time-point. Phospho-ERK staining steadily declined until essentially no observable staining was evident by the 8 hour time-point. By 12 hours, phospho-ERK staining was again observed, and essentially recovered to baseline levels within about 24 hours. Ki67 staining, which detects a marker of cell proliferation, decreased substantially over the first 12 hours and was suppressed to below detection between 24 and 48 hours, through 72 hours.

These results demonstrate that phospho-ERK can be used as a pharmacodynamic marker to monitor the efficacy of treatment with a zearalenone analog compound, and decreased levels are indicative that a cancer is sensitive to treatment.

For immunohistochemical detection of phospho-RB, anti-phospho-Retinoblastoma (pSer780) antibody (Sigma, Cat. No. R6275) was used (Table 10). Conditions were as described in IHC Procedure (1), with incubation for one hour at room temperature and EDTA retrieval buffer. Secondary reagent was anti-rabbit antibody, conjugated to horseradish peroxidase.

Results are shown in Figure 9B. Prominent phospho-pRB staining was observed initially; however, phospho-pRB level was suppressed to below detection between 48 and 72 hours. These results demonstrate that phospho-pRB can be used as a pharmacodynamic marker to monitor the efficacy of treatment with a zearalenone analog compound, and that decreased levels of phospho-pRB indicate that a cancer is sensitive to treatment with a zearalenone analog compound.

## Example 12: Effects of Compound 106 Treatment on Growth of Subcutaneous DBTRG-05MG Human Glioblastoma Xenografts *In Vivo*

The purpose of this study was to investigate anticancer activity of Compound 106 in a human DBTRG-05MG glioblastoma xenograft model. DBTRG-05MG human glioblastoma cancer cells, which carry the *BRAF* (V600E) mutation, were grown in monolayer cultures in ATCC-recommended growth media at 37 °C in a 5% $CO_2$ humidified incubator. Cells were expanded in T225 flasks for the *in vivo* study. On the day of injection, growth medium was removed, flasks were washed with PBS, and cells were collected by trypsinization. The cells were washed and collected by centrifugation, and resuspended in ice-cold PBS.

Female athymic NU/NU mice (6 week old, Charles River Laboratories (Wilmington, MA)) were inoculated subcutaneously (s.c.) with $5x10^6$ DBTRG-05MG human glioblastoma cancer cells. Those animals that developed tumors

of approximately 150 $mm^3$ were selected and randomized into five groups.

The study consisted of a vehicle-treated group and four drug-treated groups of 8 mice per group for a total of 40 mice on the first day of treatment. All treatments were initiated on day 21. Compound 106 was administered intravenously (i.v.) on a Q4D x 3 (days 21, 25, and 29) dosing schedule at dosages of 5, 10, 20 and 40 mg/kg with a volume of injection of 0.1 mL per 10 g body weight (Q4D x 3 = every four days for a total of three injections). The control group was treated with vehicle alone (20% Captisol in water; Captisol® (Sulfobutyl Ether Beta Cycolodextrin, Sodium Salt; Cydex, Inc., KS)).

General health of the mice was monitored and mortality was recorded daily. Tumor dimensions and animal body weights were recorded twice a week starting on the first day of treatment. The s.c. tumor volumes were measured and animals were weighed twice weekly starting with the first day of treatment. Tumor volume was determined by caliper measurement (mm) and using the following formula:

$$(l \text{ x } w^2)/2 = \text{mm}^3$$

where *l* and *w* refer to the larger and smaller dimensions obtained from each measurement.

Relative body weight (RBW) was calculated as follows:

RBW = body weight on the day of measurement/body weight on the first day of treatment.

The mean and standard error of the mean (SEM) of tumor volume and relative body weight for each experimental group were calculated.

The study was terminated 60 days after cancer cell transplantation. In each Compound 106 treatment group, measurements were also terminated when average tumor volume reached two doublings (4-fold tumor volume) from the first day of treatment.

Statistical analysis of the control group versus Compound 106 treatment groups was performed by a one way analysis of variance (ANOVA) followed by Dunnett's multiple comparison test for each dose of test compound in the experiment for tumor volume. A value of $P < 0.05$ was considered statistically significant under a two-sided hypothesis. All statistical analyses were performed using GraphPad Prism® software (version 4, San Diego, CA). The results are shown in Figure 10. Data show the mean + SEM. Animals were treated intravenously on days 21, 25, and 29 (Q4D x 3; indicated by arrows in the figure). Asterisks (*) indicate $P < 0.01$ vs. control group.

Administration of Compound 106 at all four doses tested, 5, 10, 20, and 40 mg/kg, caused statistically significant anticancer activity. One out of eight animals in each of the groups treated with Compound 106 at dosages of 5 mg/kg and 20 mg/kg, respectively, were tumor-free on day 60. These results indicate that growth of s.c.-implanted DBTRG-05MG human glioblastoma xenografts (which carry the *BRAF* (V600E) mutation) was highly sensitive to intermittent administration of Compound 106 at all dosages tested, 5, 10, 20, and 40 mg/kg.

## Example 13. Effects of Compound 106 Treatment on Growth of Subcutaneous LOX Human Melanoma Xenografts *In Vivo*

The purpose of this study was to investigate anticancer activity of Compound 106 in a LOX human melanoma xenograft model. LOX human melanoma cells, which carry a *BRAF* (V600E) mutation, were obtained from the DCTD Tumor Repository (Frederick, MD). Cells were grown in monolayer cultures in T225 flask with RPMI-1640 growth media at 37 °C in a 5% $CO_2$ humidified incubator. Cells were further cultivated in T225 flasks for the *in vivo* study. On the day of subcutaneous (s.c.) injections of the cells, growth medium was removed, flasks were washed with PBS, and cells were collected with trypsinization. Cells were washed and centrifuged for 3 minutes, and then resuspended in ice-cold PBS.

Female athymic NU/NU mice were inoculated s.c. with 1 x $10^6$ LOX human melanoma cells. Those animals that developed tumors of approximately 150 $mm^3$ were selected and randomized to five groups. The study consisted of a vehicle-treated group and four drug-treated groups of 8 mice per group for a total of 40 mice on the first day of treatment. All treatments were initiated on day 6. Compound 106 was administered intravenously (i.v.) on a Q4Dx3 (days 6, 10, and 14) dosing schedule at dosages of 5, 10, 20 and 40 mg/kg with a volume of injection of 0.1 mL per 10 g body weight. The control group was treated with vehicle (20% Captisol in water) alone.

The s.c. tumor volumes were measured and animals were weighed twice weekly starting with the first day of treatment. Tumor volume and relative body weight (RBW) were determined as described in Example 12.

The study was terminated fifty-nine days after cancer cell transplantation. For each Compound 106 treatment group, measurements were also terminated when mean tumor volume reached two doublings (4-fold tumor volume) from the first day of treatment.

Statistical analysis was by ANOVA as described in Example 12, and the results are shown in Figure 11. Data show the mean + SEM. Animals were treated intravenously on days 6, 10, and 14 (Q4D x 3; indicated by arrows in Figure 11). Tumor measurements on tumor bearing mice in all surviving groups were stopped on day 37. Then, tumor measurements were continued for tumor-free mice in the 10 mg/kg (1/8), 20 mg/kg (5/8), and 40 mg/kg (7/8) Compound 106 treatment groups. The study was terminated on day 59. Asterisks (*) indicate P<0.05 vs. control group.

Administration of Compound 106 at three of the doses tested, 10, 20, and 40 mg/kg, caused statistically significant anticancer activity. Additionally, one, five, and seven out of eight animals in the groups treated with Compound 106 at doses of 10, 20, or 40 mg/kg, respectively, were tumor-free at the end of the study on day 59. These results indicate that growth of s.c.-implanted LOX human melanoma xenografts (which carry the *BRAF* (V600E) mutation and are wild-type for *PTEN*) was highly sensitive to intermittent administration of Compound 106 at doses of 10, 20, and 40 mg/kg.

Compound 106 was also tested in other xenograft models, in which *BRAF*-mutated cells, including breast, colon, and melanoma cell lines, or wildtype cells, including pancreatic cell lines, were transplanted subcutaneously into female nude mice. Compound 106 or a vehicle control was administered intravenously. In the *BRAF*-mutated xenograft models, at 40 mg/kg of Compound 106 and a dosing regimen of QD x 5 for 2 weeks, Compound 106 showed inhibitory effects ranging from tumor stasis (but not regression) during treatment (> 80% tumor growth inhibition) to about 73% tumor regression. In the wildtype xenograft models, at 40 mg/kg of Compound 106 and a dosing regimen of QD x 5 for 2 weeks, Compound 106 showed inhibitory effects ranging from 30-40% tumor regression.

## Claims

1. A method of prognosing the ability of a zearalenone analog compound to treat a cancer in a subject, the method comprising:

   a) determining whether a sample derived from said subject exhibits activated MAPK signaling as compared to a control sample; and
   b) determining whether said sample exhibits wild-type PI3K signaling as compared to a control sample,
   wherein the zearalenone analog compound is a compound of formula (I)

   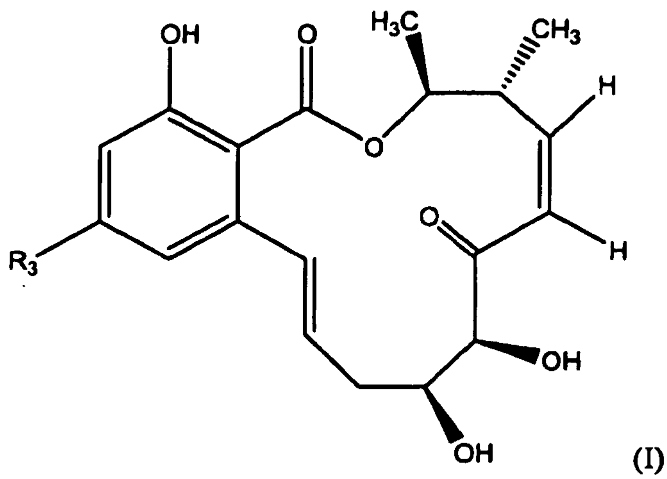

   (I)

   wherein $R_3$ is $-NHR_1$, and $R_1$ is $C_1$-$C_3$ alkyl substituted with 0, 1, or 2 hydroxyl moieties, or a pharmaceutically acceptable salt or ester thereof, and
   wherein activated MAPK signaling and wild-type PI3K signaling in said sample as determined in steps a) and b) indicates that a zearalenone analog compound has the ability to treat the cancer in the subject, thereby prognosing the ability of a zearalenone analog compound to treat the cancer in the subject.

2. The method of claim 1, wherein determining whether said sample exhibits activated MAPK signaling comprises identifying a mutation in the *BRAF* gene in said sample which increases BRAF activity, wherein the presence of

[[a]] the mutation in the *BRAF* gene in said sample is an indication of activated MAPK signaling.

**3.** The method of claim 2, wherein the mutation in the *BRAF* gene is selected from the group consisting of V600E, G464E, G464V, G466A, G466E, G466V, G469A, G469E, E586K, F595L, G596R, L597V, L597R, L597S and V600D.

**4.** The method of claim 1, wherein determining whether said sample exhibits activated MAPK signaling comprises measuring BRAF activity in said sample, wherein an increase in BRAF activity in said sample as compared to a control sample is an indication of activated MAPK signaling.

**5.** The method of claim 1, wherein determining whether said sample exhibits activated MAPK signaling comprises measuring the activity of one more proteins selected from the group consisting of MEK1, MEK2, ERK1 and ERK2 in said sample, wherein an increase in the activity of one or more of said proteins in said sample as compared to a control sample is an indication of activated MAPK signaling.

**6.** The method of claim 1, wherein determining whether said sample exhibits wild-type PI3K signaling comprises determining the mutational status of the *PTEN* gene in said sample, wherein the lack of a mutation in the *PTEN* gene in said sample is an indication of wild-type PI3K signaling.

**7.** The method of claim 1, wherein determining whether said sample exhibits wild-type PI3K signaling comprises determining the level of phosphorylated AKT protein in said sample as compared to the total level of AKT protein in said sample or as compared to a control sample, wherein a low to moderate level of phosphorylated AKT protein in said sample is an indication of wild-type PI3K signaling.

**8.** The method of claim 7, wherein the level of AKT phosphorylation is determined by Western blotting, immunohistochemistry (IHC) or fluorescent in situ hybridization (FISH).

**9.** The method of claim 1, wherein determining whether said sample exhibits wild-type PI3K signaling comprises measuring the activity of the AKT protein in said sample, wherein a low to moderate level of activity of the AKT protein in said sample as compared to a control sample is an indication of wild-type PI3K signaling.

**10.** The method of claim 1 wherein step a) comprises determining whether a sample derived from said subject exhibits a mutation in the *BRAF* gene which increases BRAF activity; and step b) comprises determining the level of phosphorylated AKT protein in said sample as compared to the total level of AKT protein in said sample or as compared to a control sample, wherein the presence of a mutation in the *BRAF* gene is an indication of activated MAPK signaling, and a low to moderate level of phosphorylated AKT protein in said sample as determined in step b) is an indication of wild-type PI3K signaling.

**11.** The method of claim 1 wherein step a) comprises determining whether a sample derived from said subject exhibits a mutation in the *BRAF* gene which increases BRAF activity; and step b) comprises determining whether said sample exhibits a wild-type *PTEN* sequence, wherein the presence of a mutation in the *BRAF* gene is an indication of activated MAPK signaling,_and a wild-type *PTEN* sequence in said sample is an indication of wild-type PI3K signaling.

**12.** The method of claim 11, further comprising measuring the activity of AKT protein in a sample from the subject, wherein a low to moderate level of activity of AKT protein in said sample as compared to a control sample indicates that a zearalenone analog compound has the ability to treat the cancer in the subject, thereby prognosing the ability of a zearalenone analog compound to treat the cancer in the subject.

**13.** The method of claim 11, further comprising determining the level of phosphorylated AKT protein in a sample from said subject as compared to the total level of AKT protein in the sample or as compared to a control sample, wherein a low to moderate level of phosphorylated AKT protein in the sample as compared to the total level of AKT protein in the sample or as compared to the control sample indicates that a zearalenone analog compound has the ability to treat the cancer in the subject, thereby prognosing the ability of a zearalenone analog compound to treat the cancer in the subject.

**14.** The method of claim 10 wherein step a) comprises determining whether a sample derived from said subject exhibits a V600E mutation in the *BRAF* gene, wherein the presence of a V600E mutation in the *BRAF* gene is an indication of activated MAPK signaling.

**15.** The method of any one of claims 1, 10, 11 or 14, wherein said sample derived from said subject is a tumor biopsy.

**16.** The method of any one of claims 10 or 11 wherein the mutation in the *BRAF* gene is V600E or
wherein the mutation in the *BRAF* gene is a mutation in the kinase domain of *BRAF* or
wherein the mutation in the *BRAF* gene is selected from the group consisting of V600E, G464E, G464V, G466A, G466E, G466V, G469A, G469E, E586K, F595L, G596R, L597V, L597R, L597S and V600D.

**17.** The method of any one of claims 10 or 11, wherein determining whether said sample exhibits a mutation in the *BRAF* gene is accomplished using a technique selected from the group consisting of polymerase chain reaction (PCR) amplification reaction, reverse-transcriptase PCR analysis, single-strand conformation polymorphism analysis (SSCP), mismatch cleavage detection, heteroduplex analysis, Southern blot analysis, Western blot analysis, and deoxyribonucleic acid sequencing of said sample.

**18.** The method of any one of claims 10 or 14, wherein the level of phosphorylated AKT protein in said sample is determined by Western blot, immunohistochemistry (IHC) or fluorescent in situ hybridization (FISH).

**19.** The method of any one of claims 10 or 14, wherein the level of phosphorylated AKT protein in said sample as compared to the total level of AKT protein in said sample is determined, and wherein said low to moderate level of phosphorylated AKT protein in said sample is from about level 1 to about level 4 as compared to the total level of AKT protein in said sample.

**20.** The method of claim 1 wherein step a) comprises determining whether a sample derived from the subject exhibits a mutation in the *BRAF* gene which activates BRAF; and step b) comprises determining the expression level of AKT protein in the sample as compared to a control sample, wherein the presence of a mutation in the *BRAF* gene is an indication of activated MAPK signaling,_and a low to moderate level of expression of AKT protein as determined in step b) is an indication of wild-type PI3K signaling.

**21.** The method of any one of claims 1-20 wherein the zearalenone analog compound is a compound of formula (I) or a pharmaceutically acceptable salt or ester thereof, and wherein $R_3$ is an unsubstituted $C_{1-3}$ alkyl-amino or wherein $R_3$ is a group selected from the group consisting of methylamino and ethylamino.

**22.** The method of claim 21, wherein the zearalenone analog compound is the compound:

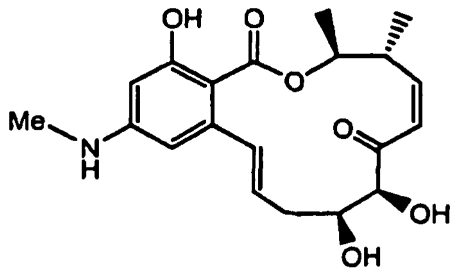

**(Compound 091)**

or a pharmaceutically acceptable salt or ester thereof or wherein the zearalenone analog compound is

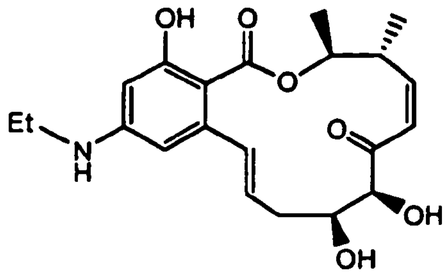

**(Compound 106)**

or a pharmaceutically acceptable salt or ester thereof.

**23.** The method of any one of the preceding claims, wherein the cancer is selected from the group consisting of breast cancer, melanoma, thyroid cancer, colorectal cancer, pancreatic cancer, brain tumors, ovarian cancer, leukemia, neural cancer, glioma, neuroblastoma, retinoblastoma, multiple myeloma and B-cell lymphoma.

**24.** The method of any one of the preceding claims, wherein the cancer is a *BRAF* mutated cancer optionally wherein the *BRAF* mutated cancer is selected from the group consisting of metastatic melanoma, papillary thyroid carcinoma, colorectal carcinoma, and a primary brain tumor.

**25.** A composition comprising a therapeutically effective amount of a zearalenone analog compound of Formula (I),

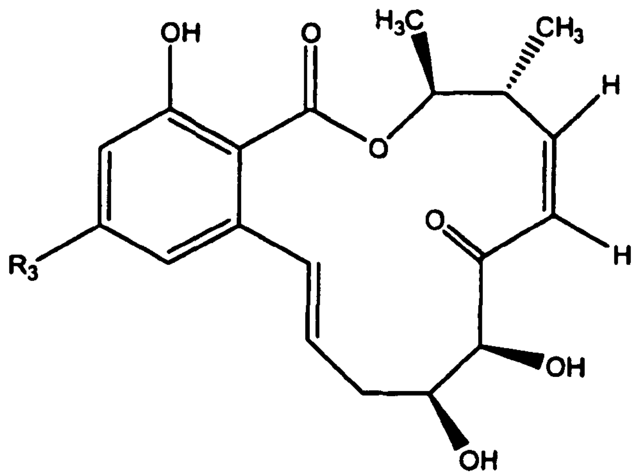

(I)

wherein $R_3$ is $-NHR_1$, and $R_1$ is $C_1$-$C_3$ alkyl substituted with 0, 1, or 2 hydroxyl moieties, or a pharmaceutically acceptable salt or ester thereof, for use in treating cancer in a subject based on an evaluation of the results of an assessment of a sample derived from said subject for activated MAPK signaling as compared to a control sample and for wild-type PI3K signaling as compared to a control sample, wherein the evaluation indicates that the sample has activated MAPK signaling and wild-type PI3K signaling.

**26.** The composition of claim 25, wherein the zearalenone analog compound is a compound of formula (I) or a pharmaceutically acceptable salt or ester thereof, and wherein $R_3$ is an unsubstituted $C_{1-3}$ alkyl-amino or wherein $R_3$ is a group selected from the group consisting of methylamino and ethylamino.

**27.** The composition of claim 26, wherein the zearalenone analog compound is the compound:

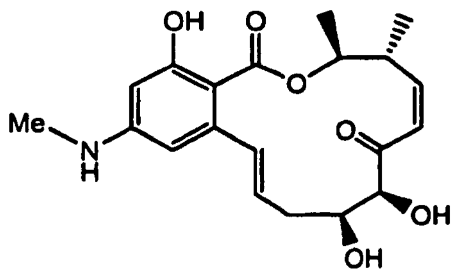

(Compound 091)

or a pharmaceutically acceptable salt or ester thereof or wherein the zearalenone analog compound is

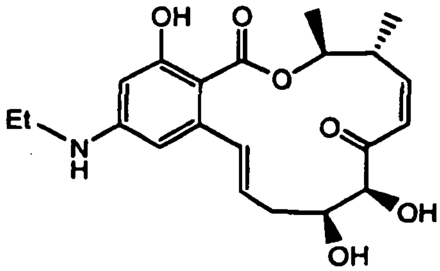

(Compound 106)

or a pharmaceutically acceptable salt or ester thereof.

**28.** The composition of any one of claims 25 to 27, wherein the cancer is selected from the group consisting of breast cancer, melanoma, thyroid cancer, colorectal cancer, pancreatic cancer, brain tumors, ovarian cancer, leukemia, neural cancer, glioma, neuroblastoma, retinoblastoma, multiple myeloma and B-cell lymphoma.

**29.** The composition of any one of claims 25 to 28, wherein the cancer is a *BRAF* mutated cancer optionally wherein the *BRAF* mutated cancer is selected from the group consisting of metastatic melanoma, papillary thyroid carcinoma, colorectal carcinoma, and a primary brain tumor.

**Patentansprüche**

**1.** Verfahren zum Prognostizieren der Fähigkeit einer Zearalenonanalogverbindung, Krebs bei einem Patienten zu behandeln, wobei das Verfahren umfasst:

a) Bestimmen, ob eine Probe, die von dem Patienten abgeleitet ist, einen aktivierten MAPK-Signalweg ("MAPK signaling") im Vergleich zu einer Kontrollprobe aufweist; und
b) Bestimmen, ob die Probe einen Wildtyp-P13K-Signalweg ("P13K signaling") im Vergleich zu einer Kontrollprobe aufweist, wobei die Zearalenonanalogverbindung eine Verbindung der
Formel (I)

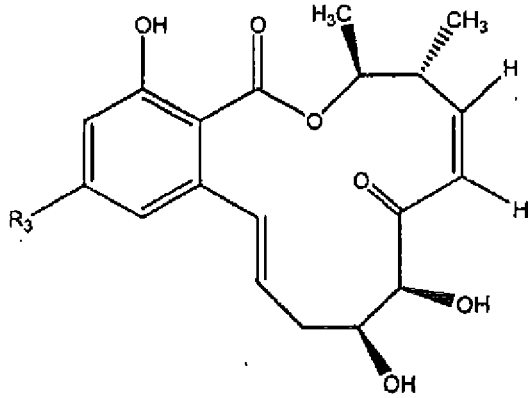

(I)

ist, wobei $R_3$ -$NHR_1$ ist und $R_1$ $C_1$-$C_3$-Alkyl, substituiert mit 0, 1 oder 2 Hydroxylresten, oder ein pharmazeutisch annehmbares Salz oder ein Ester davon ist, und
wobei der aktivierte MAPK-Signalweg und Wiltyp-P13K-Signalweg in der Probe, wie in Schritt a) und b) bestimmt, anzeigt, dass eine Zearalenonanalogverbindung die Fähigkeit hat, Krebs bei dem Patienten zu behandeln, wodurch die Fähigkeit einer Zearalenonanalogverbindung prognostiziert wird, Krebs bei dem Patienten zu behandeln.

**2.** Verfahren nach Anspruch 1, wobei das Bestimmen, ob die Probe einen aktivierten MAPK-Signalweg aufweist, das Identifizieren einer Mutation im BRAF-Gen in der Probe umfasst, die die BRAF-Aktivität erhöht, wobei die Gegenwart

von [[a]] der Mutation im BRAF-Gen in der Probe ein Hinweis auf einen aktivierten MAPK-Signalweg ist.

3. Verfahren nach Anspruch 2, wobei die Mutation im BRAF-Gen ausgewählt ist aus der Gruppe bestehend aus V600E, G464E, G464V, G466A, G466E, G466V, G469A, G469E, E586K, F595L, G596R, L597V, L597R, L597S und V600D.

4. Verfahren nach Anspruch 1, wobei das Bestimmen, ob die Probe einen aktivierten MAPK-Signalweg aufweist, das Messen einer BRAF-Aktivität in der Probe umfasst, wobei ein Anstieg in der BRAF-Aktivität in der Probe im Vergleich zu einer Kontrollprobe ein Hinweis auf einen aktivierten MAPK-Signalweg ist.

5. Verfahren nach Anspruch 1, wobei das Bestimmen, ob die Probe einen aktivierten MAPK-Signalweg aufweist, das Messen der Aktivität eines oder mehrerer Proteine, die ausgewählt sind aus der Gruppe bestehend aus MEK1, MEK2, ERK1 und ERK2, in der Probe umfasst, wobei ein Anstieg in der Aktivität eines oder mehrerer dieser Proteine in der Probe im Vergleich zu einer Kontrollprobe ein Hinweis auf einen aktivierten MAPK-Signalweg ist.

6. Verfahren nach Anspruch 1, wobei das Bestimmen, ob die Probe einen Wildtyp-P13K-Signalweg aufweist, das Bestimmen des Mutationsstatus des PTEN-Gens in der Probe umfasst, wobei das Fehlen einer Mutation im PTEN-Gen in der Probe ein Hinweis auf einen Wildtyp-P13K-Signalweg ist.

7. Verfahren nach Anspruch 1, wobei das Bestimmen, ob die Probe einen Wildtyp-P13K-Signalweg aufweist, das Bestimmen des Wertes eines phosphorylierten AKT-Proteins in der Probe im Vergleich zum Gesamtwert an AKT-Protein in der Probe oder im Vergleich zu einer Kontrollprobe umfasst, wobei ein niederer bis mäßiger Wert eines phosphorylierten AKT-Proteins in der Probe ein Hinweis auf einen Wildtyp-P13K-Signalweg ist.

8. Verfahren nach Anspruch 7, wobei der Wert einer AKT-Phosphorylierung durch Western-Blotting, Immunhistochemie (IHC) oder Fluoreszenz-in-situ-Hybridisierung (FISH) bestimmt wird.

9. Verfahren nach Anspruch 1, wobei das Bestimmen, ob die Probe einen Wildtyp-P13K-Signalweg aufweist, das Messen der Aktivität des AKT-Proteins in der Probe umfasst, wobei ein niederer bis mäßiger Wert der Aktivität des AKT-Proteins in der Probe im Vergleich zu einer Kontrollprobe ein Hinweis auf einen Wildtyp-P13K-Signalweg ist.

10. Verfahren nach Anspruch 1, wobei Schritt a) das Bestimmen umfasst, ob eine Probe, die von dem Patienten abgeleitet ist, eine Mutation im BRAF-Gen aufweist, die die BRAF-Aktivität erhöht; und Schritt b) das Bestimmen des Wertes eines phosphorylierten AKT-Proteins in der Probe im Vergleich zum Gesamtwert eines AKT-Proteins in der Probe oder im Vergleich zu einer Kontrollprobe umfasst, wobei das Vorhandensein einer Mutation im BRAF-Gen ein Hinweis auf einen aktivierten MAPK-Signalweg ist und ein niederer bis mäßiger Wert eines phosphorylierten AK-T-Proteins in der Probe, wie in Schritt b) bestimmt, ein Hinweis auf einen Wildtyp-P13K-Signalweg ist.

11. Verfahren nach Anspruch 1, wobei Schritt a) das Bestimmen umfasst, ob eine Probe, die von dem Patienten abgeleitet ist, eine Mutation im BRAF-Gen aufweist, die die BRAF-Aktivität erhöht; und Schritt b) das Bestimmen umfasst, ob die Probe eine Wildtyp-PTEN-Sequenz aufweist, wobei das Vorhandensein einer Mutation im BRAF-Gen ein Hinweis auf einen aktivierten MAPK-Signalweg ist und eine Wildtyp-PTEN-Sequenz in der Probe ein Hinweis auf einen Wildtyp-P13K-Signalweg ist.

12. Verfahren nach Anspruch 11, des Weiteren umfassend das Messen der Aktivität des AKT-Proteins in einer Probe von dem Patienten, wobei ein niederer bis mäßiger Wert der Aktivität des AKT-Proteins in der Probe im Vergleich zu einer Kontrollprobe anzeigt, dass eine Zearalenonanalogverbindung die Fähigkeit hat, den Krebs bei dem Patienten zu behandeln, wodurch die Fähigkeit einer Zearalenonanalogverbindung prognostiziert wird, den Krebs bei dem Patienten zu behandeln.

13. Verfahren nach Anspruch 11, des Weiteren umfassend das Bestimmen des Wertes eines phosphorylierten AKT-Proteins in einer Probe von dem Patienten im Vergleich zum Gesamtwert an AK-T-Protein in der Probe oder im Vergleich zu einer Kontrollprobe, wobei ein niederer bis mäßiger Wert eines phosphorylierten AK-T-Proteins in der Probe im Vergleich zum Gesamtwert an AKT-Protein in der Probe oder im Vergleich zur Kontrollprobe anzeigt, dass eine Zearalenonanalogverbindung die Fähigkeit hat, den Krebs bei dem Patienten zu behandeln, wodurch die Fähigkeit einer Zearalenonanalogverbindung prognostiziert wird, den Krebs bei dem Patienten zu behandeln.

14. Verfahren nach Anspruch 10, wobei Schritt a) das Bestimmen umfasst, ob eine Probe, die von dem Patienten abgeleitet ist, eine V600E-Mutation im BRAF-Gen aufweist, wobei das Vorhandensein einer V600E-Mutation im

BRAF-Gen ein Hinweis auf einen aktivierten MAPK-Signalweg ist.

**15.** Verfahren nach einem der Ansprüche 1, 10, 11 oder 14, wobei die Probe, die von dem Patienten abgeleitet ist, eine Tumorbiopsie ist.

**16.** Verfahren nach einem der Ansprüche 10 oder 11, wobei die Mutation im BRAF-Gen V600E ist oder wobei die Mutation im BRAF-Gen eine Mutation in der Kinasedomäne von BRAF ist oder wobei die Mutation im BRAF-Gen ausgewählt ist aus der Gruppe bestehend aus V600E, G464E, G464V, G466A, G466E, G466V, G469A, G469E, E586K, F595L, G596R, L597V, L597R, L597S und V600D.

**17.** Verfahren nach einem der Ansprüche 10 oder 11, wobei das Bestimmen, ob die Probe eine Mutation im BRAF-Gen aufweist, unter Verwendung einer Technik ausgeführt wird, die ausgewählt ist aus der Gruppe bestehend aus Polymerasekettenreaktion (PCR), Amplifikationsreaktion, Umkehr-Transkriptase-PCR-Analyse, Einzelstrang-Konformations-Polymorphismus-Analyse (SSCP), Mismatch Cleavage Detection, Heteroduplexanalyse, Southern-Blot-Analyse, Western-Blot-Analyse und Desoxyribonukleinsäuresequenzierung der Probe.

**18.** Verfahren nach einem der Ansprüche 10 oder 14, wobei der Wert des phosphorylierten AKT-Proteins in der Probe durch Western Blot, Immunhistochemie (IHC) oder Fluoreszenz-in-situ-Hybridisierung (FISH) bestimmt wird.

**19.** Verfahren nach einem der Ansprüche 10 oder 14, wobei der Wert des phosphorylierten AKT-Proteins in der Probe im Vergleich zum Gesamtwert an AKT-Protein in der Probe bestimmt wird und wobei der niedere bis mäßige Wert des phosphorylierten AK-T-Proteins in der Probe von einem Wert 1 bis zu einem Wert von etwa 4 im Vergleich zum Gesamtwert an AKT-Protein in der Probe reicht.

**20.** Verfahren nach Anspruch 1, wobei Schritt a) das Bestimmen umfasst, ob eine Probe, die von dem Patienten abgeleitet ist, eine Mutation im BRAF-Gen aufweist, die BRAF aktiviert; und Schritt b) das Bestimmen des Expressionswertes des AKT-Proteins in der Probe im Vergleich zu einer Kontrollprobe umfasst, wobei das Vorhandensein einer Mutation im BRAF-Gen ein Hinweis auf einen aktivierten MAPK-Signalweg ist und ein niederer bis mäßiger Expressionswert des AKT-Proteins, wie in Schritt b) bestimmt, ein Hinweis auf einen Mildtyp-P13K-Signalweg ist.

**21.** Verfahren nach einem der Ansprüche 1 bis 20, wobei die Zearalenonanalogverbindung eine Verbindung der Formel (I) oder ein pharmazeutisch annehmbares Salz oder ein Ester davon ist und wobei $R_3$ ein unsubstituiertes $C_{1-3}$-Alkylamino ist oder wobei $R_3$ eine Gruppe ist, die ausgewählt ist aus der Gruppe bestehend aus Methylamino und Ethylamino.

**22.** Verfahren nach Anspruch 21, wobei die Zearalenonanalogverbindung die Verbindung:

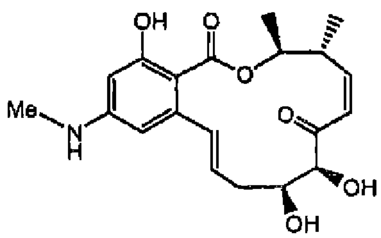

(Verbindung 091)

oder ein pharmazeutisch annehmbares Salz oder ein Ester davon ist oder wobei die Zearalenonanalogverbindung

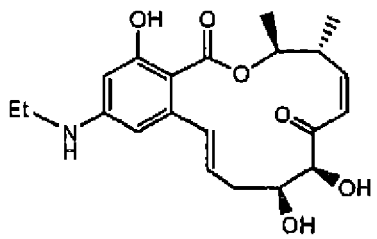

(Verbindung 106)

oder ein pharmazeutisch annehmbares Salz oder ein Ester davon ist.

**23.** Verfahren nach einem der vorangehenden Ansprüche, wobei der Krebs ausgewählt ist aus der Gruppe bestehend aus Brustkrebs, Melanomen, Schilddrüsenkrebs, Kolorektalkrebs, Bauchspeicheldrüsenkrebs, Hirntumoren, Eierstockkrebs, Leukämie, Nervenkrebs, Gliomen, Neuroblastomen, Retinoblastomen, multiplen Myelomen und B-Zelllymphomen.

**24.** Verfahren nach einem der vorangehenden Ansprüchen, wobei der Krebs ein BRAF-mutierter Krebs ist, wobei optional der BRAF-mutierte Krebs ausgewählt ist aus der Gruppe bestehend aus metastatischen Melanomen, papillären Schilddrüsenkarzinomen, Kolorektalkarzinomen und einem primären Hirntumor.

**25.** Zusammensetzung, enthaltend eine therapeutisch wirksame Menge einer Zearalenonanalogverbindung der Formel (I)

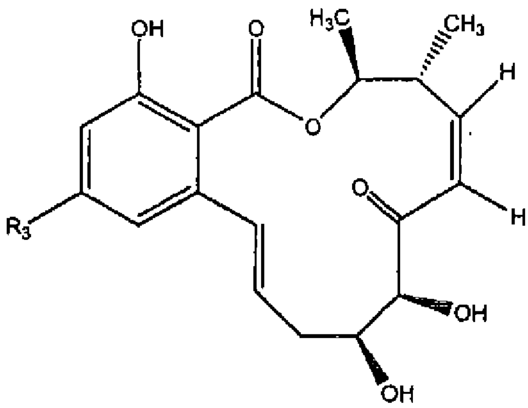

(I)

wobei $R_3$ -NHR$_1$ ist und $R_1$ $C_1$-$C_3$-Alkyl, substituiert mit 0, 1 oder 2 Hydroxylresten, oder ein pharmazeutisch annehmbares Salz oder ein Ester davon ist, zur Verwendung in der Behandlung von Krebs bei einem Patienten auf der Basis einer Auswertung der Ergebnisse einer Bewertung einer Probe, die von dem Patienten abgeleitet ist, auf aktivierten MAPK-Signalweg im Vergleich zu einer Kontrollprobe und auf einen Wildtyp-P13K-Signalweg im Vergleich zu einer Kontrollprobe, wobei die Auswertung anzeigt, dass die Probe einen aktivierten MAPK-Signalweg und Wildtyp-P13K-Signalweg aufweist.

**26.** Zusammensetzung nach Anspruch 25, wobei die Zearalenonanalogverbindung eine Verbindung der Formel (I) oder ein pharmazeutisch annehmbares Salz oder ein Ester davon ist und wobei $R_3$ ein unsubstituiertes $C_{1-3}$-Alkylamino ist oder wobei $R_3$ eine Gruppe ausgewählt aus der Gruppe ist, die aus Methylamino und Ethylamino besteht.

**27.** Zusammensetzung nach Anspruch 26, wobei die Zearalenonanalogverbindung die Verbindung

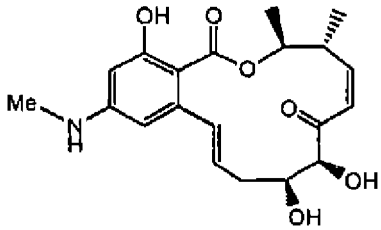

(Verbindung 091)

oder ein pharmazeutisch annehmbares Salz oder ein Ester davon ist oder wobei die Zearalenonanalogverbindung

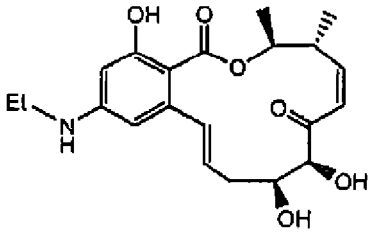

(Verbindung 106)

oder ein pharmazeutisch annehmbares Salz oder ein Ester davon ist.

**28.** Zusammensetzung nach einem der Ansprüche 25 bis 27, wobei der Krebs ausgewählt ist aus der Gruppe bestehend aus Brustkrebs, Melanomen, Schilddrüsenkrebs, Kolorektalkrebs, Bauchspeicheldrüsenkrebs, Hirntumoren, Eierstockkrebs, Leukämie, Nervenkrebs, Gliomen, Neuroblastomen, Retinoblastomen, multiplen Myelomen und B-Zelllymphomen.

**29.** Zusammensetzung nach einem der Ansprüche 25 bis 28, wobei der Krebs ein BRAF-mutierter Krebs ist, wobei optional der BRAF-mutierte Krebs ausgewählt ist aus der Gruppe bestehend aus metastatischen Melanomen, papillären Schilddrüsenkarzinomen, Kolorektalkarzinomen und einem primären Hirntumor.

## Revendications

**1.** Procédé de pronostic de la capacité d'un composé analogue de la zéaralénone à traiter un cancer chez un sujet, le procédé comprenant :

a) déterminer si un échantillon provenant dudit sujet présente une signalisation MAPK active par rapport à un échantillon témoin ; et
b) déterminer si ledit échantillon présente une signalisation PI3K de type sauvage par rapport à un échantillon témoin,
le composé analogue de la zéaralénone étant un
composé de formule (I)

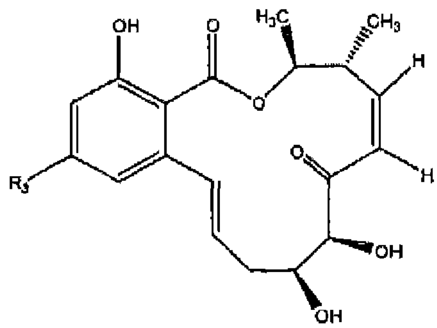

(I)

dans laquelle $R_3$ est -$NHR_1$, et $R_1$ est un alkyle en $C_1$ à $C_3$ substitué avec 0, 1 ou 2 fragments hydroxyles, ou l'un de ses sels ou esters pharmaceutiquement acceptables, et

dans lequel la signalisation MAPK active et la signalisation PI3K de type sauvage dans ledit échantillon, telles que déterminées dans les étapes a) et b), indiquent qu'un composé analogue de la zéaralénone a la capacité à traiter le cancer du sujet, ce qui permet de pronostiquer la capacité d'un composé analogue de la zéaralénone à traiter le cancer du sujet.

**2.** Procédé selon la revendication 1, dans lequel l'étape consistant à déterminer si ledit échantillon présente une signalisation MAPK active comprend l'identification d'une mutation dans le gène BRAF dans ledit échantillon qui augmente l'activité de BRAF, dans lequel la présence de [[a]] la mutation dans le gène BRAF dans ledit échantillon est une indication de la signalisation MAPK active.

**3.** Procédé selon la revendication 2, dans lequel la mutation du gène BRAF est choisie dans le groupe constitué par V600E, G464E, G464V, G466A, G466E, G466V, G469A, G469E, E586K, F595L, G596R, L597V, L597R, L597S et V600D.

**4.** Procédé selon la revendication 1, dans lequel l'étape consistant à déterminer si ledit échantillon présente une signalisation MAPK active comprend la mesure de l'activité de BRAF dans ledit échantillon, dans lequel une augmentation de l'activité de BRAF dans ledit échantillon par rapport à un échantillon témoin est une indication de la signalisation MAPK active.

**5.** Procédé selon la revendication 1, dans lequel l'étape consistant à déterminer si ledit échantillon présente une signalisation MAPK active comprend la mesure de l'activité d'une protéine ou de plusieurs protéines choisies dans le groupe constitué par MEK1, MEK2, ERK1 et ERK2 dans ledit échantillon, dans lequel une augmentation de l'activité d'une ou de plusieurs desdites protéines dans ledit échantillon par rapport à un échantillon témoin est une indication de la signalisation MAPK active.

**6.** Procédé selon la revendication 1, dans lequel l'étape consistant à déterminer si ledit échantillon présente une signalisation PI3K de type sauvage comprend la détermination du statut mutationnel du gène PTEN dans ledit échantillon, dans lequel l'absence de mutation dans le gène PTEN dans ledit échantillon est une indication de la signalisation PI3K de type sauvage.

**7.** Procédé selon la revendication 1, dans lequel l'étape consistant à déterminer si ledit échantillon présente une signalisation PI3K de type sauvage comprend la détermination du taux de protéines AKT phosphorylées dans ledit échantillon par rapport au taux total de protéines AKT dans ledit échantillon ou par rapport à un échantillon témoin, dans lequel un taux faible à modéré de protéines AKT phosphorylées dans ledit échantillon est une indication de la signalisation PI3K de type sauvage.

**8.** Procédé selon la revendication 7, dans lequel le taux de phosphorylation AKT est déterminé par transfert de Western, immunohistochimie (IHC) ou hybridation in situ en fluorescence (FISH).

**9.** Procédé selon la revendication 1, dans lequel l'étape consistant à déterminer si ledit échantillon présente une signalisation PI3K de type sauvage comprend la mesure de l'activité de la protéine AKT dans ledit échantillon, dans lequel un niveau faible à modéré d'activité de la protéine AKT dans ledit échantillon par rapport à un échantillon témoin est une indication de la signalisation PI3K de type sauvage.

**10.** Procédé selon la revendication 1, dans lequel l'étape a) comprend le fait de déterminer si un échantillon provenant dudit sujet présente une mutation dans le gène BRAF qui augmente l'activité de BRAF ; et l'étape b) comprend le fait de déterminer le taux de protéines AKT phosphorylées dans ledit échantillon par rapport au taux total de protéines AKT dans ledit échantillon ou par rapport à un échantillon témoin, la présence d'une mutation dans le gène BRAF étant une indication de la signalisation MAPK active et un taux faible à modéré de protéines AKT phosphorylées dans ledit échantillon, tel que déterminé dans l'étape b), étant une indication de la signalisation PI3K de type sauvage.

**11.** Procédé selon la revendication 1, dans lequel l'étape a) comprend le fait de déterminer si un échantillon provenant dudit sujet présente une mutation dans le gène BRAF qui augmente l'activité de BRAF ; et l'étape b) comprend le fait de déterminer si ledit échantillon présente une séquence PTEN de type sauvage, la présence d'une mutation dans le gène BRAF étant une indication de la signalisation MAPK active et une séquence PTEN de type sauvage dans ledit échantillon étant une indication de la signalisation PI3K de type sauvage.

**12.** Procédé selon la revendication 11, comprenant en outre la mesure de l'activité de la protéine AKT dans un échantillon provenant du sujet, dans lequel un niveau d'activité faible à modéré de la protéine AKT dans ledit échantillon par rapport à un échantillon témoin indique qu'un composé analogue de la zéaralénone a la capacité à traiter le cancer du sujet, ce qui permet de pronostiquer la capacité d'un composé analogue de la zéaralénone à traiter le cancer du sujet.

**13.** Procédé selon la revendication 11, comprenant en outre la détermination du taux de protéines AKT phosphorylées dans un échantillon provenant dudit patient par rapport au taux total de protéines AKT dans l'échantillon ou par rapport à un échantillon témoin, un taux faible à modéré de protéines AKT phosphorylées dans l'échantillon par rapport au taux total de protéines AKT dans l'échantillon ou par rapport à un échantillon témoin indiquant qu'un composé analogue de la zéaralénone a la capacité à traiter le cancer du sujet, ce qui permet de pronostiquer la capacité d'un composé analogue de la zéaralénone à traiter le cancer du sujet.

**14.** Procédé selon la revendication 10, dans lequel l'étape a) comprend le fait de déterminer si un échantillon provenant dudit sujet présente une mutation V600E dans le gène BRAF, la présence d'une mutation V600E dans le gène BRAF étant une indication de la signalisation MAPK active.

**15.** Procédé selon l'une quelconque des revendications 1, 10, 11 ou 14, dans lequel ledit échantillon provenant dudit sujet est une biopsie de tumeur.

**16.** Procédé selon l'une quelconque des revendications 10 ou 11, dans lequel la mutation dans le gène BRAF est V600E ou
dans lequel la mutation dans le gène BRAF est une mutation dans le domaine kinase de BRAF ou
dans lequel la mutation dans le gène BRAF est choisie dans le groupe constitué par V600E, G464E, G464V, G466A, G466E, G466V, G469A, G469E, E586K, F595L, G596R, L597V, L597R, L597S et V600D.

**17.** Procédé selon l'une quelconque des revendications 10 ou 11, dans lequel l'étape qui consiste à déterminer si ledit échantillon présente une mutation dans le gène BRAF est réalisée au moyen d'une technique choisie dans le groupe constitué par une amplification en chaîne par polymérase (PCR), une analyse PCR par transcriptase inverse, une analyse du polymorphisme de conformation simple brin (SSCP), une détection de clivage de mésappariement, une analyse d'hétéroduplex, une analyse par transfert de Southern, une analyse par transfert de Western et un séquençage d'acide désoxyribonucléique dudit échantillon.

**18.** Procédé selon l'une quelconque des revendications 10 ou 14, dans lequel le taux de protéines AKT phosphorylées dans ledit échantillon est déterminé par transfert de Western, immunohistochimie (IHC) ou hybridation in situ en fluorescence (FISH).

**19.** Procédé selon l'une quelconque des revendications 10 ou 14, dans lequel le taux de protéines AKT phosphorylées dans ledit échantillon par rapport au taux total de protéines AKT dans ledit échantillon est déterminé, et dans lequel ledit taux faible à modéré de protéines AKT phosphorylées dans ledit échantillon est un taux d'environ 1 à environ 4 par rapport au taux total de protéines AKT dans ledit échantillon.

**20.** Procédé selon la revendication 1, dans lequel l'étape a) comprend le fait de déterminer si un échantillon provenant du sujet présente une mutation dans le gène BRAF, qui active BRAF ; et l'étape b) comprend la détermination du taux d'expression des protéines AKT dans l'échantillon par rapport à un échantillon témoin, la présence d'une

mutation dans le gène BRAF étant une indication de la signalisation MAPK active et un taux faible à modéré d'expression des protéines AKT, tel que déterminé dans l'étape b), étant une indication de la signalisation PI3K de type sauvage.

**21.** Procédé selon l'une quelconque des revendications 1 à 20, dans lequel le composé analogue de la zéaralénone est un composé de formule (I); ou l'un de ses sels ou esters pharmaceutiquement acceptables, et dans lequel $R_3$ est un alkyl-amino en $C_1$ à $C_3$ non substitué ou dans lequel $R_3$ est un groupe choisi dans le groupe constitué par le méthylamino et l'éthylamino.

**22.** Procédé selon la revendication 21, dans lequel le composé analogue de la zéaralénone est le composé

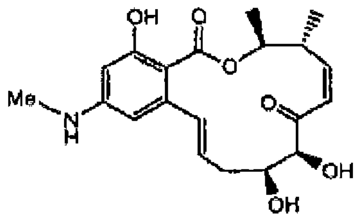

(Composé 091)

ou l'un de ses sels ou esters pharmaceutiquement acceptables, ou dans lequel le composé analogue de la zéaralénone est

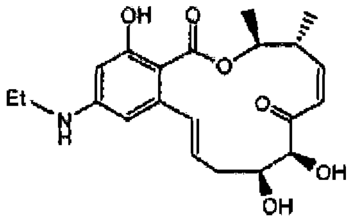

(Composé 106)

ou l'un de ses sels ou esters pharmaceutiquement acceptables.

**23.** Procédé selon l'une quelconque des revendications précédentes, dans lequel le cancer est choisi dans le groupe constitué par un cancer du sein, un mélanome, un cancer de la thyroïde, un cancer colorectal, un cancer du pancréas, les tumeurs du cerveau, un cancer des ovaires, une leucémie, un cancer neural, un gliome, un neuroblastome, un rétinoblastome, un myélome multiple et un lymphome B.

**24.** Procédé selon l'une quelconque des revendications précédentes, dans lequel le cancer est un cancer à mutation BRAF, facultativement dans lequel le cancer à mutation BRAF est choisi dans le groupe constitué par un mélanome métastatique, un carcinome thyroïdien papillaire, un carcinome colorectal et une tumeur primaire du cerveau.

**25.** Composition comprenant une quantité thérapeutiquement efficace d'un composé analogue de la zéaralénone de formule (I),

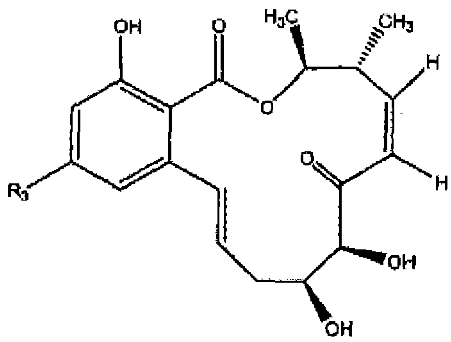

(I)

dans laquelle $R_3$ est -$NHR_1$, et $R_1$ est un alkyle en $C_1$ à $C_3$ substitué avec 0, 1 ou 2 fragments hydroxyles, ou l'un de ses sels ou esters pharmaceutiquement acceptables, pour un usage dans le traitement d'un cancer chez un sujet sur la base d'une évaluation des résultats d'une identification dans un échantillon provenant dudit sujet d'une signalisation MAPK active par rapport à un échantillon témoin et d'une signalisation PI3K de type sauvage par rapport à un échantillon témoin, l'évaluation indiquant que l'échantillon présente une signalisation MAPK active et une signalisation PI3K de type sauvage.

**26.** Composition selon la revendication 25, dans laquelle le composé analogue de la zéaralénone est un composé de formule (I) ou l'un de ses sels ou esters pharmaceutiquement acceptables, et dans laquelle $R_3$ est un alkyl-amino en $C_1$ à $C_3$ non substitué ou dans laquelle $R_3$ est un groupe choisi dans le groupe constitué par le méthylamino et l'éthylamino.

**27.** Composition selon la revendication 26, dans laquelle le composé analogue de la zéaralénone est le composé

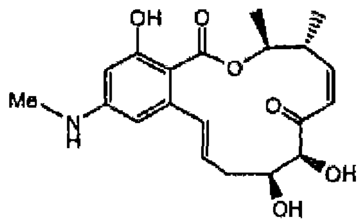

(Composé 091)

ou l'un de ses sels ou esters pharmaceutiquement acceptables, ou dans laquelle le composé analogue de la zéaralénone est

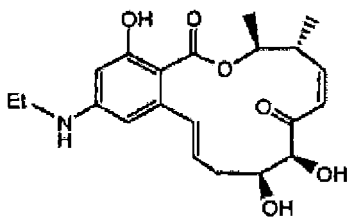

(Composé 106)

ou l'un de ses sels ou esters pharmaceutiquement acceptables.

**28.** Composition selon l'une quelconque des revendications 25 à 27, dans lequel le cancer est choisi dans le groupe constitué par un cancer du sein, un mélanome, un cancer de la thyroïde, un cancer colorectal, un cancer du pancréas, les tumeurs du cerveau, un cancer des ovaires, une leucémie, un cancer neural, un gliome, un neuroblastome, un rétinoblastome, un myélome multiple et un lymphome B.

**29.** Composition selon l'une quelconque des revendications 25 à 28, dans laquelle le cancer est un cancer à mutation BRAF, facultativement dans lequel le cancer à mutation BRAF est choisi dans le groupe constitué par un mélanome métastatique, un carcinome thyroïdien papillaire, un carcinome colorectal et une tumeur primaire du cerveau.

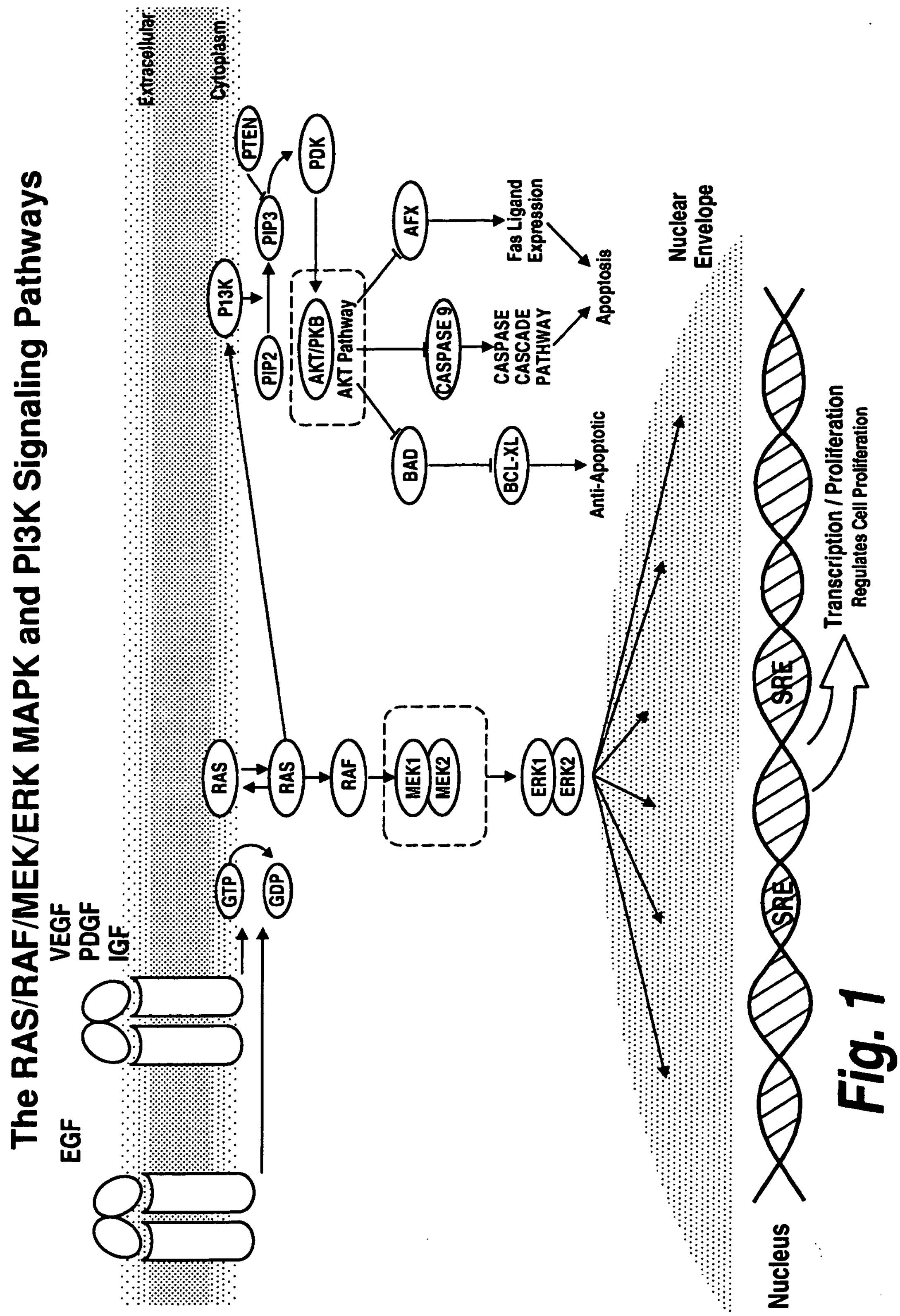

The RAS/RAF/MEK/ERK MAPK and PI3K Signaling Pathways
EGF
VEGF
PDGF
IGF
Extracellular
Cytoplasm
GTP
GDP
RAS
RAS
RAF
MEK1
MEK2
ERK1
ERK2
P13K
PIP2
PIP3
PTEN
PDK
AKT/PKB
AKT Pathway
BAD
BCL-XL
Anti-Apoptotic
CASPASE 9
CASPASE
CASCADE
PATHWAY
AFX
Fas Ligand
Expression
Apoptosis
Nuclear
Envelope
Nucleus
SRE
SRE
Transcription / Proliferation
Regulates Cell Proliferation


Fig. 1

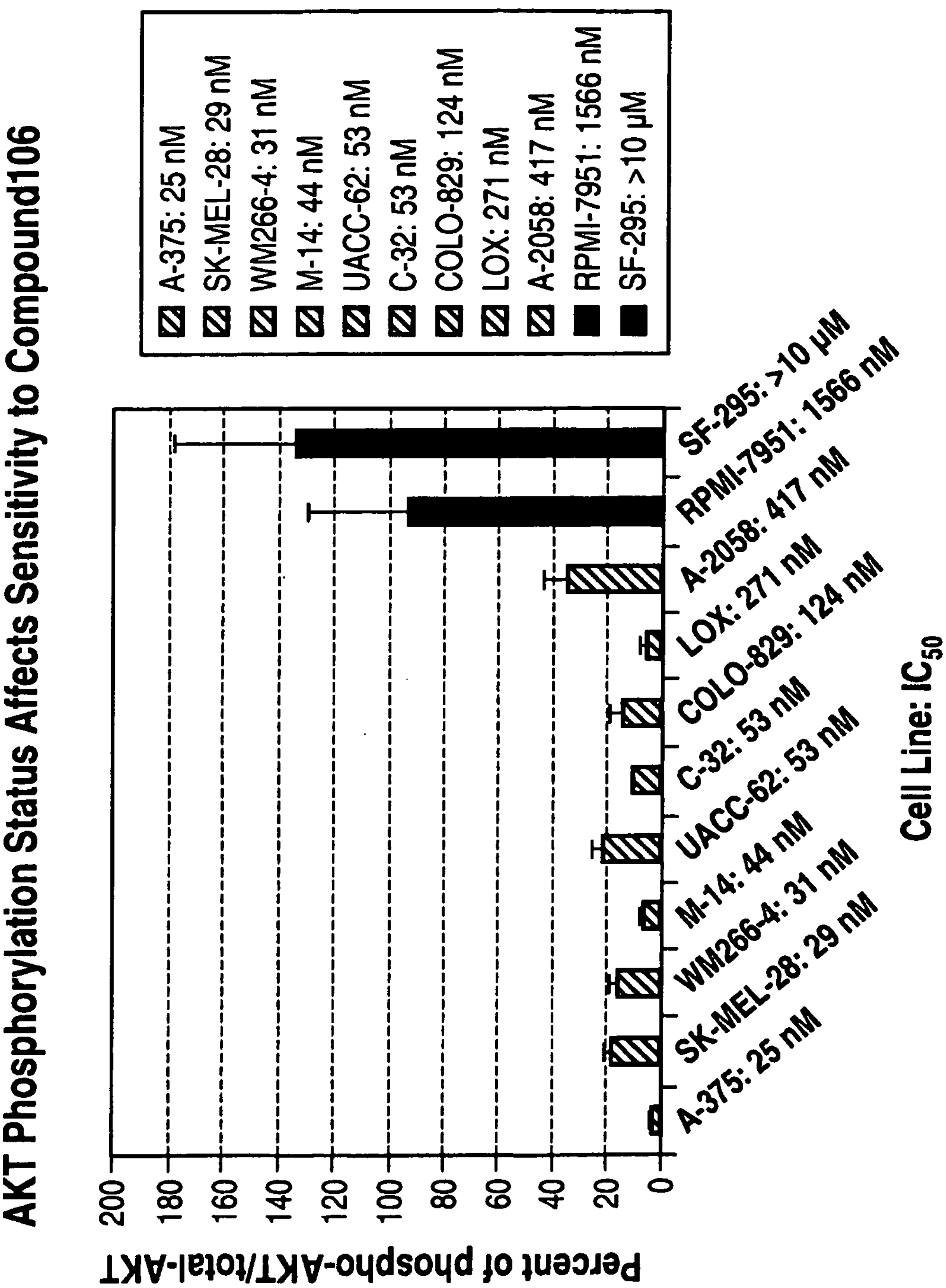
AKT Phosphorylation Status Affects Sensitivity to Compound106
Percent of phospho-AKT/total-AKT
0
20
40
60
80
100
120
140
160
180
200
A-375: 25 nM
SK-MEL-28: 29 nM
WM266-4: 31 nM
M-14: 44 nM
UACC-62: 53 nM
C-32: 53 nM
COLO-829: 124 nM
LOX: 271 nM
A-2058: 417 nM
RPMI-7951: 1566 nM
SF-295: >10 µM
Cell Line: $IC_{50}$

*Fig. 2*

Fig. 3A

*BRAF*-Mutated Cells are Highly Sensitive to Compound 106

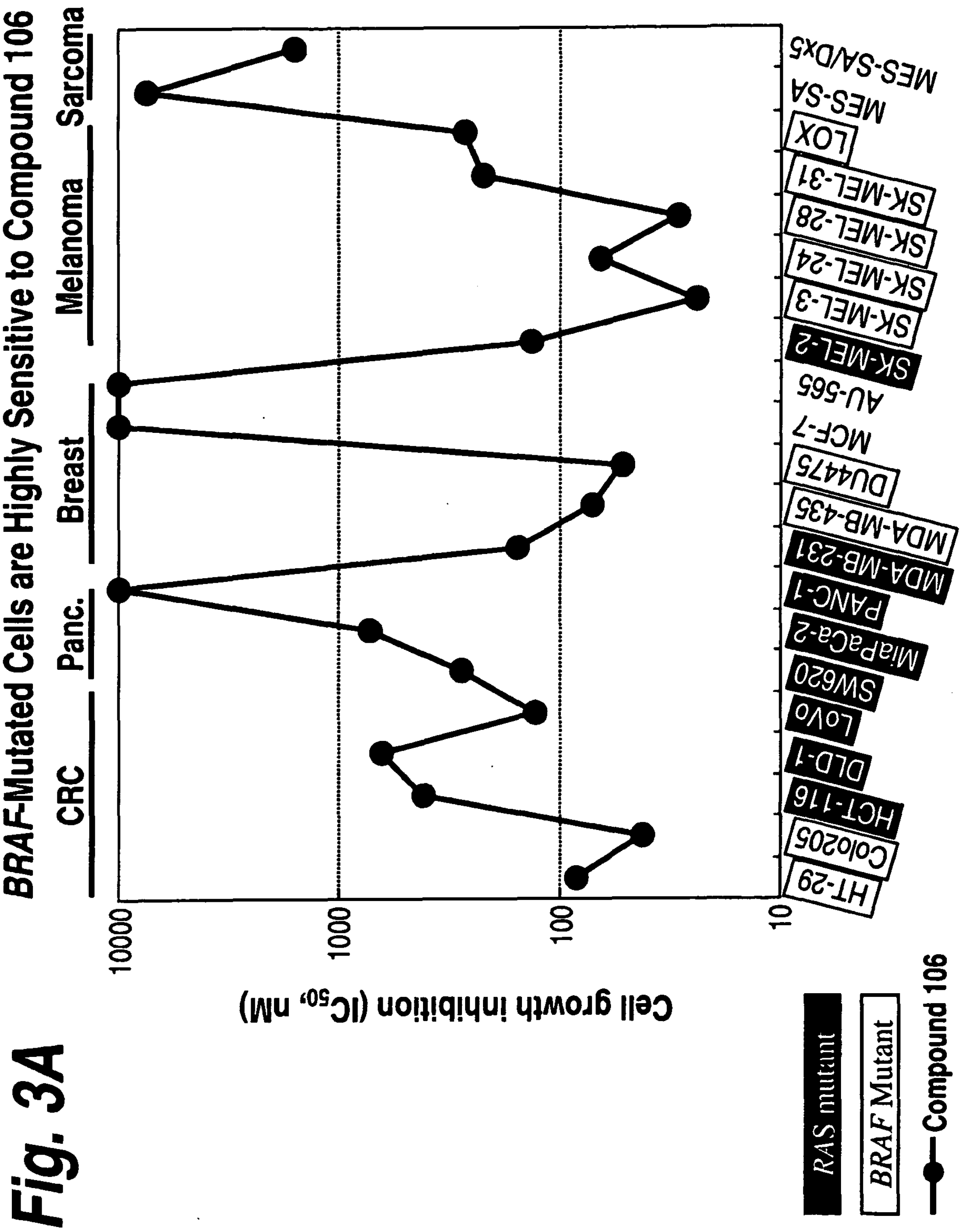

## Inhibition of *BRAF* Mutated Cancer Cell Growth

| Cell Line | Description | *K-RAS* mutation | *BRAF* mutation | Mean $IC_{50}$, nmol/L | |
|---|---|---|---|---|---|
| | | | | Cpd 106 | Cpd 091 |
| AU-565 | breast cancer | | WT | >10000 | >10000 |
| MCF-7 | | | WT | >10000 | >10000 |
| MDA-MB-231 | | G13D | WT | 156 | 119 |
| MDA-MB-435 | | | V600E | 71 | 50 |
| DU4475 | | | V600E | 52 | 30 |
| SK-MEL-2 | melanoma | Q61R (N-ras) | WT | 135 | 113 |
| SK-MEL-3 | | | V600E | 24 | 19 |
| SK-MEL-24 | | | V600E | 66 | 53 |
| SK-MEL-31 | | | V600E | 225 | 209 |
| HCT-116 | colon cancer | G13D | WT | 423 | 257 |
| DLD-1 | | G13D | WT | 649 | 466 |
| LoVo | | G13D | WT | 132 | 90 |
| SW-620 | | G12V | WT | 282 | 207 |
| COLO205 | | WT | V600E | 43 | 27 |
| HT-29 | | WT | V600E | 84 | 65 |
| Mia PaCa-2 | pancreatic cancer | G12C | WT | 734 | 582 |
| PANC-1 | | G12D | WT | >10000 | >10000 |
| BxPC-3 | | WT | WT | - | 412 |
| MES-SA | sarcoma | | WT | 7490 | 5780 |
| MES-SA/Dx5-Rx1 | sarcoma (P-glycoprotein overex.) | | WT | 1590 | 908 |
| IMR-90 | Non-dividing fibroblast | | | - | >10000 |

"WT" = wild type; "-" = not tested

**Fig. 3B**

## $IC_{50}$ Values for Compound 106 in a Panel of Melanoma Cell Lines

| Cell Line No. | $IC_{50}$ (nmol/L) | *BRAF* | Type (*BRAF*) | *PTEN* | pAKT |
|---|---|---|---|---|---|
| 1 | 7 | V600R | homo | wt | 0 |
| 2 | 8 | V600E | hetero | wt | 0 |
| 3 | 8 | V600E | hetero | wt | 0 |
| 4 | 13 | V600E | hetero | F56I | 2 |
| 5 | 21 | V600E | hetero | wt | 0 |
| 6 | 26 | V600E | hetero | wt | 0 |
| 7 | 29 | L597S | homo | wt | 1 |
| 8 | 46 | V600E | hetero | wt | 1 |
| 9 | 46 | V600E | hetero | wt | 3 |
| 10 | 53 | V600E | hetero | wt | 0 |
| 11 | 64 | V600E | hetero | L139X | 2 |
| 12 | 66 | V600E | hetero | wt | 0 |
| 13 | 68 | V600E | hetero | Q298X | 1 |
| 14 | 69 | V600E | hetero | wt | 0 |
| 15 | 75 | V600E | hetero | wt | 2 |
| 16 | 201 | V600E | hetero | Y76X | 1 |
| 17 | 318 | V600E | hetero | wt | 0 |
| 18 | 894 | V600E | hetero | Splice Mutation | 3 |
| 19 | 1081 | V600E | hetero | HomoDeln | 3 |
| 20 | 3378 | L597S | homo | wt | 0 |
| 21 | 24 | wt | wt | wt | 0 |
| 22 | 43 | wt | wt | wt | 0 |
| 23 | 84 | wt | wt | wt | 0 |
| 24 | 120 | wt | wt | wt | 0 |
| 25 | 138 | wt | wt | wt | 0 |
| 26 | 201 | wt | wt | wt | 0 |
| 27 | 396 | wt | wt | wt | 0 |
| 28 | 520 | wt | wt | wt | 1 |
| 29 | 612 | wt | wt | wt | 3 |
| 30 | 739 | wt | wt | HomoDeln | 0 |
| 31 | 1669 | wt | wt | I50N | 1 |

***Fig. 3C***

## $IC_{50}$ Values for Compound 106 in a Panel of Cell Lines

| Cell Line | $IC_{50}$ (nmol/L) | BRAF | PTEN |
|---|---|---|---|
| Malme-3M | 13 | V600E | wt |
| A375 | 25 | V600E | wt |
| SK-MEL-28 | 28 | V600E | wt |
| WM-266-4 | 31 | V600E | wt |
| SH-4 | 40 | V600E | wt |
| SK-MEL-3 | 40 | V600E | wt |
| M14 | 44 | V600E | wt |
| C32 | 53 | V600E | Del-FS |
| UACC-62 | 53 | V600E | wt |
| SK-MEL-24 | 80 | V600E | Del-FS |
| A101D | 85 | V600E | wt |
| Colo-829 | 124 | V600E | wt |
| G-361 | 133 | V600E | wt |
| HT-144 | 182 | V600E | Del-In-fr |
| LOX | 300 | V600E | wt |
| A2058 | 417 | V600E | L112Q, V175Fs 2 |
| RPMI-7951 | 1566 | V600E | Del-FS |
| SK-MEL-31 | 225 | V600E | wt |

*Fig. 3D*

## Prognostic and Response Biomarkers

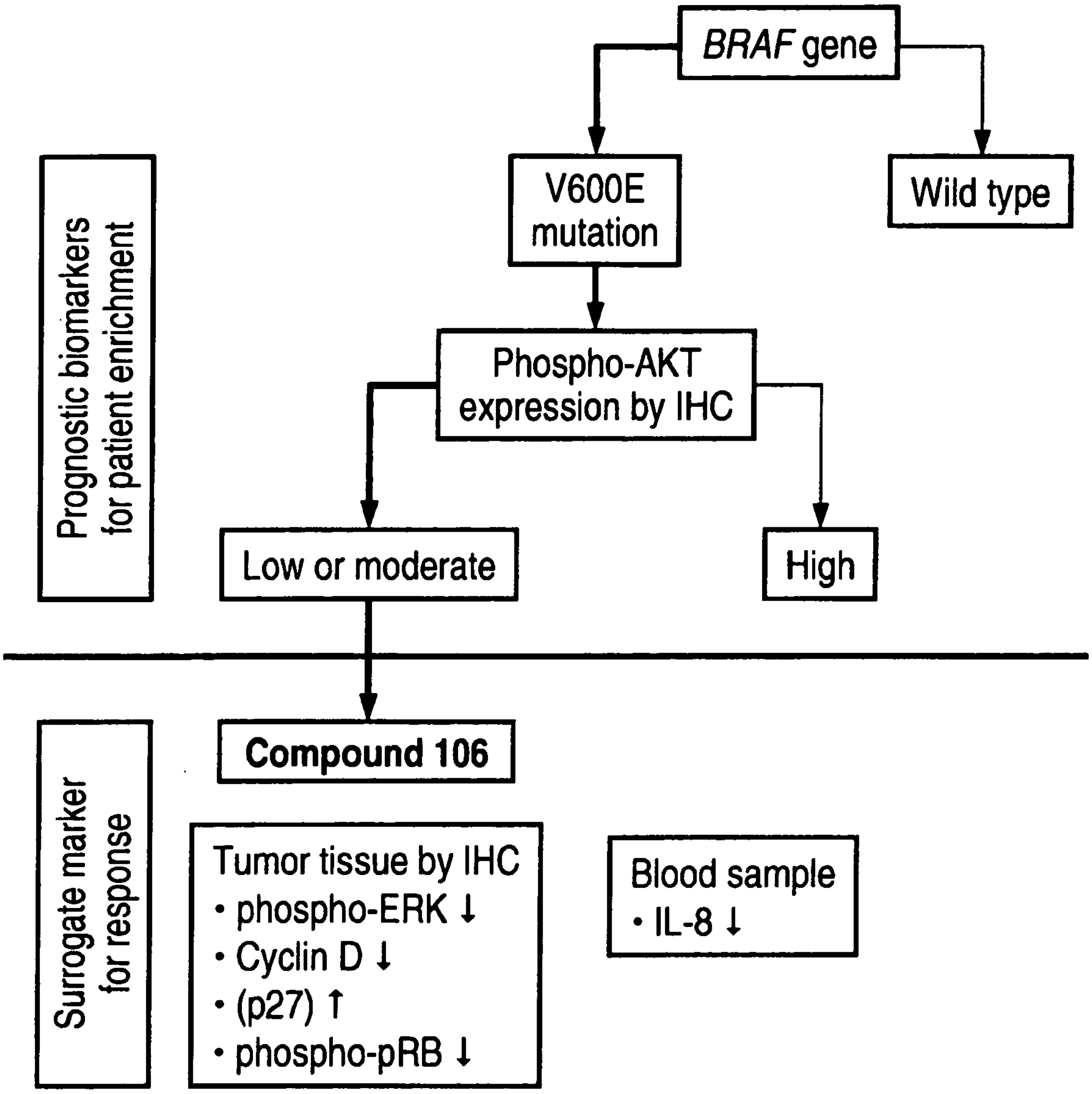


*Fig. 4*

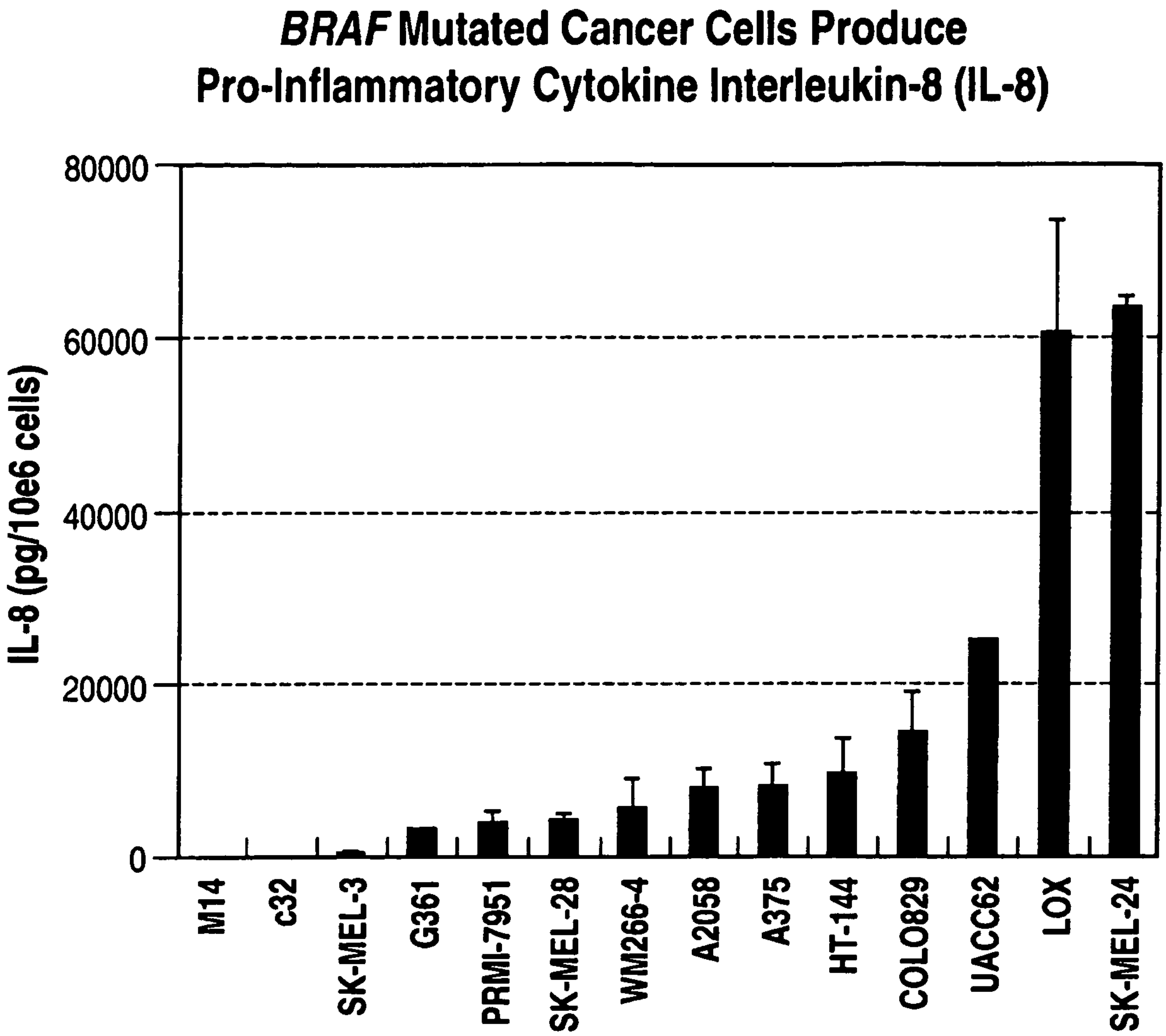
*BRAF* Mutated Cancer Cells Produce
Pro-Inflammatory Cytokine Interleukin-8 (IL-8)
80000
60000
40000
20000
0
IL-8 (pg/10e6 cells)
M14
c32
SK-MEL-3
G361
PRMI-7951
SK-MEL-28
WM266-4
A2058
A375
HT-144
COLO829
UACC62
LOX
SK-MEL-24

*Fig. 5*

## IL-6 and IL-8 Protein Production is Inhibited by Compound 106 in LOX Melanoma Cells *in vitro*

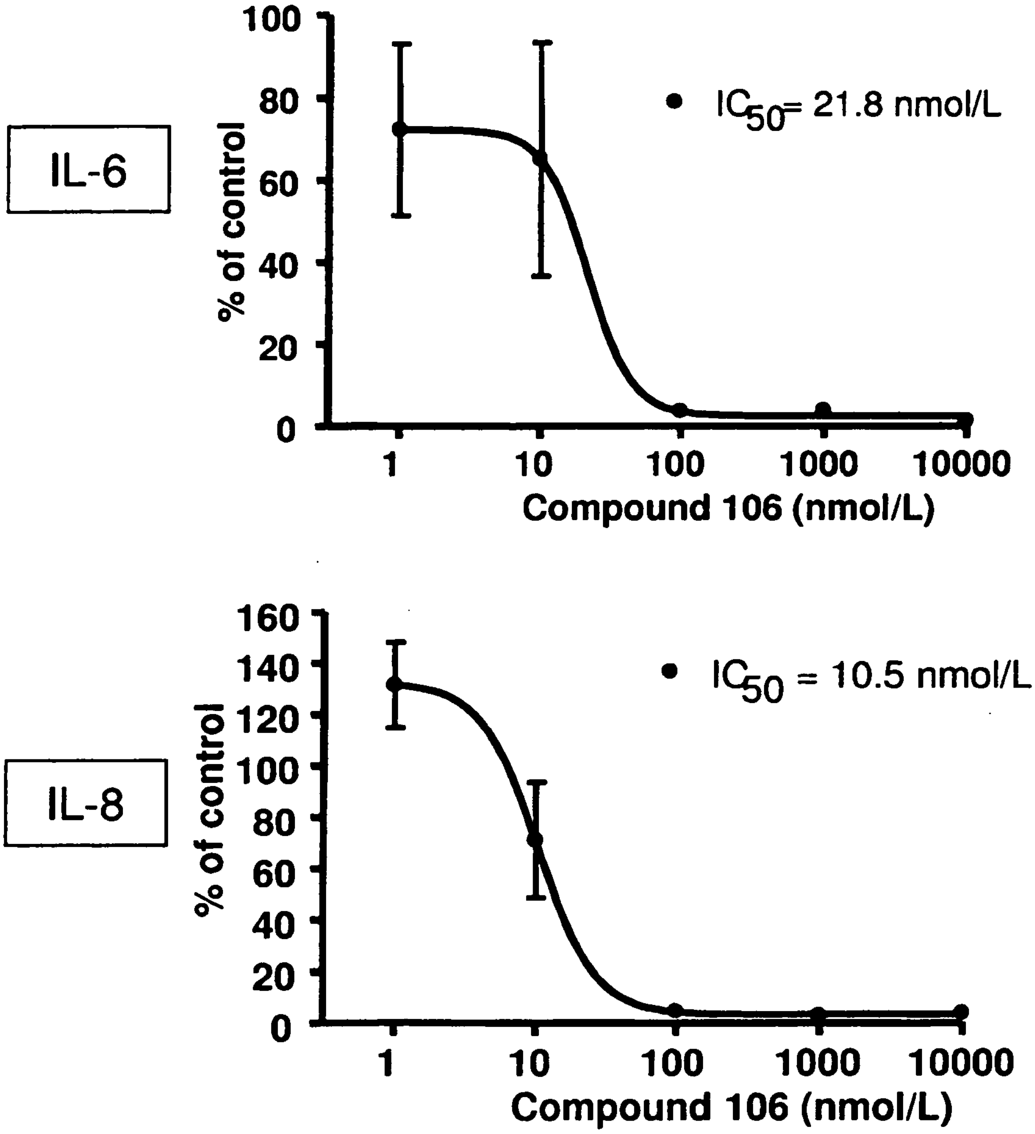


*Fig. 6*

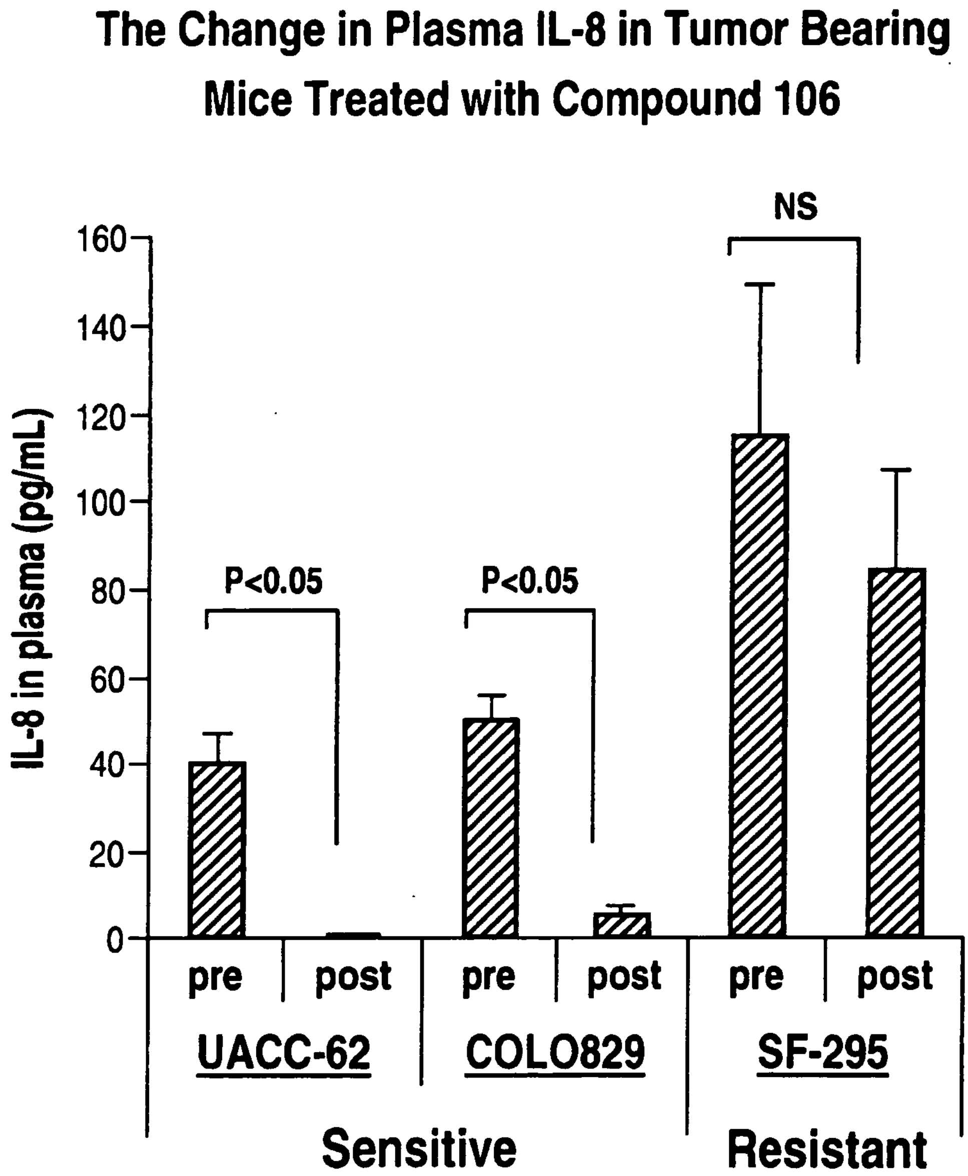
The Change in Plasma IL-8 in Tumor Bearing Mice Treated with Compound 106
IL-8 in plasma (pg/mL)
160
140
120
100
80
60
40
20
0
P<0.05
P<0.05
NS
pre
post
pre
post
pre
post
UACC-62
COLO829
SF-295
Sensitive
Resistant

*Fig. 7*

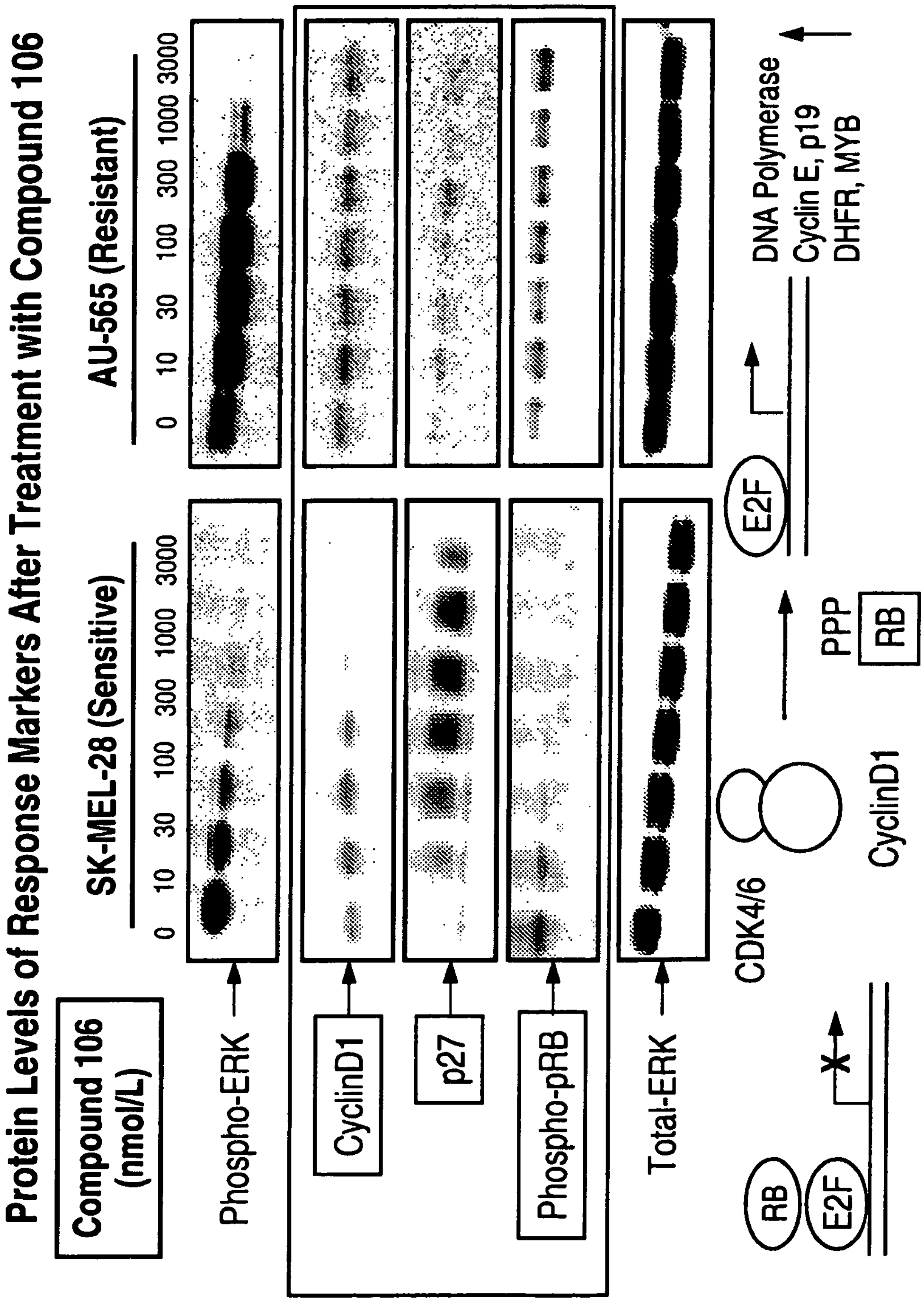
Protein Levels of Response Markers After Treatment with Compound 106
Compound 106 (nmol/L)
SK-MEL-28 (Sensitive)
AU-565 (Resistant)
0 10 30 100 300 1000 3000
0 10 30 100 300 1000 3000
Phospho-ERK
CyclinD1
p27
Phospho-pRB
Total-ERK
RB
E2F
CDK4/6
CyclinD1
PPP
RB
E2F
DNA Polymerase
Cyclin E, p19
DHFR, MYB

*Fig. 8*

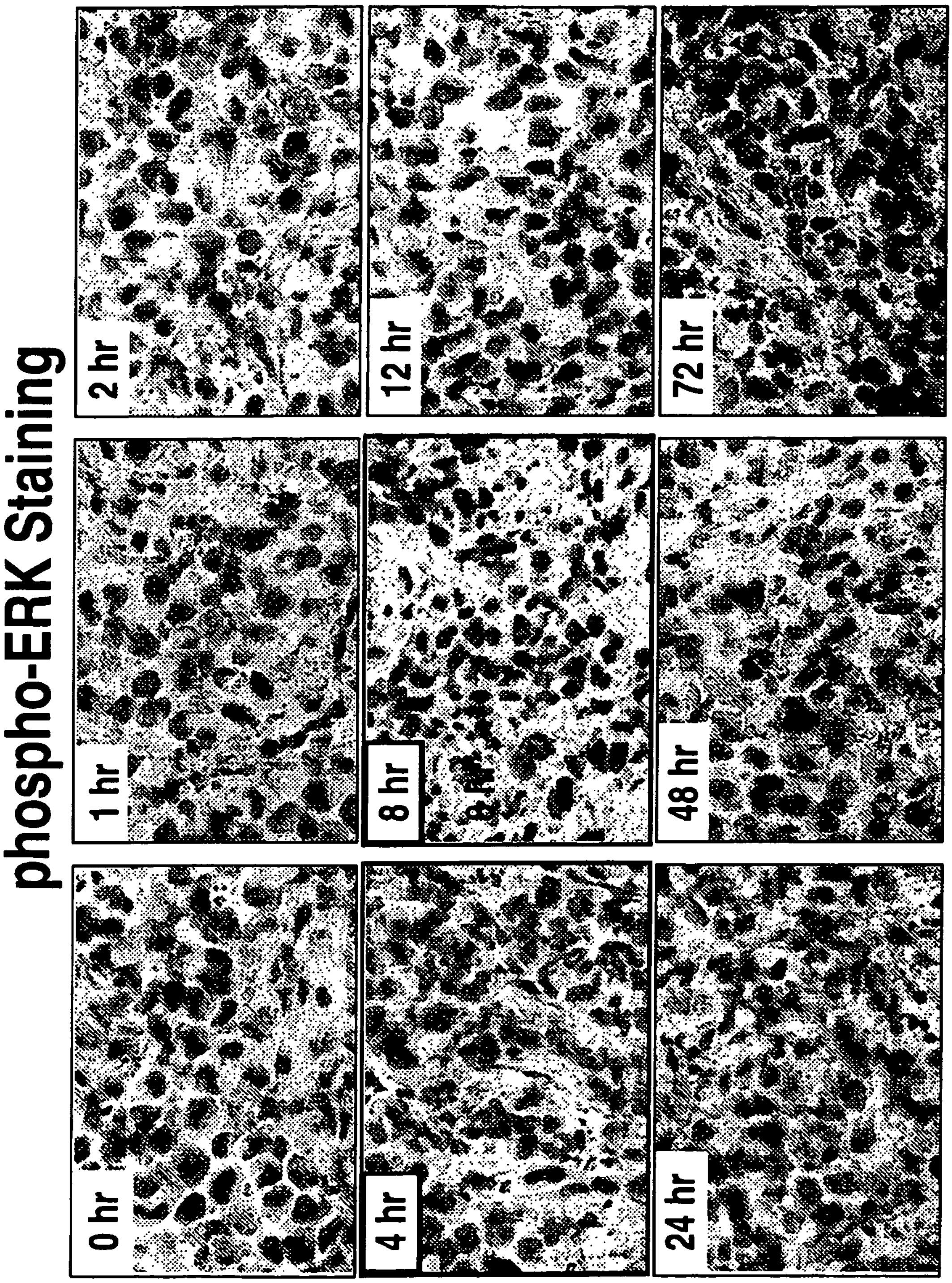


*Fig. 9A*

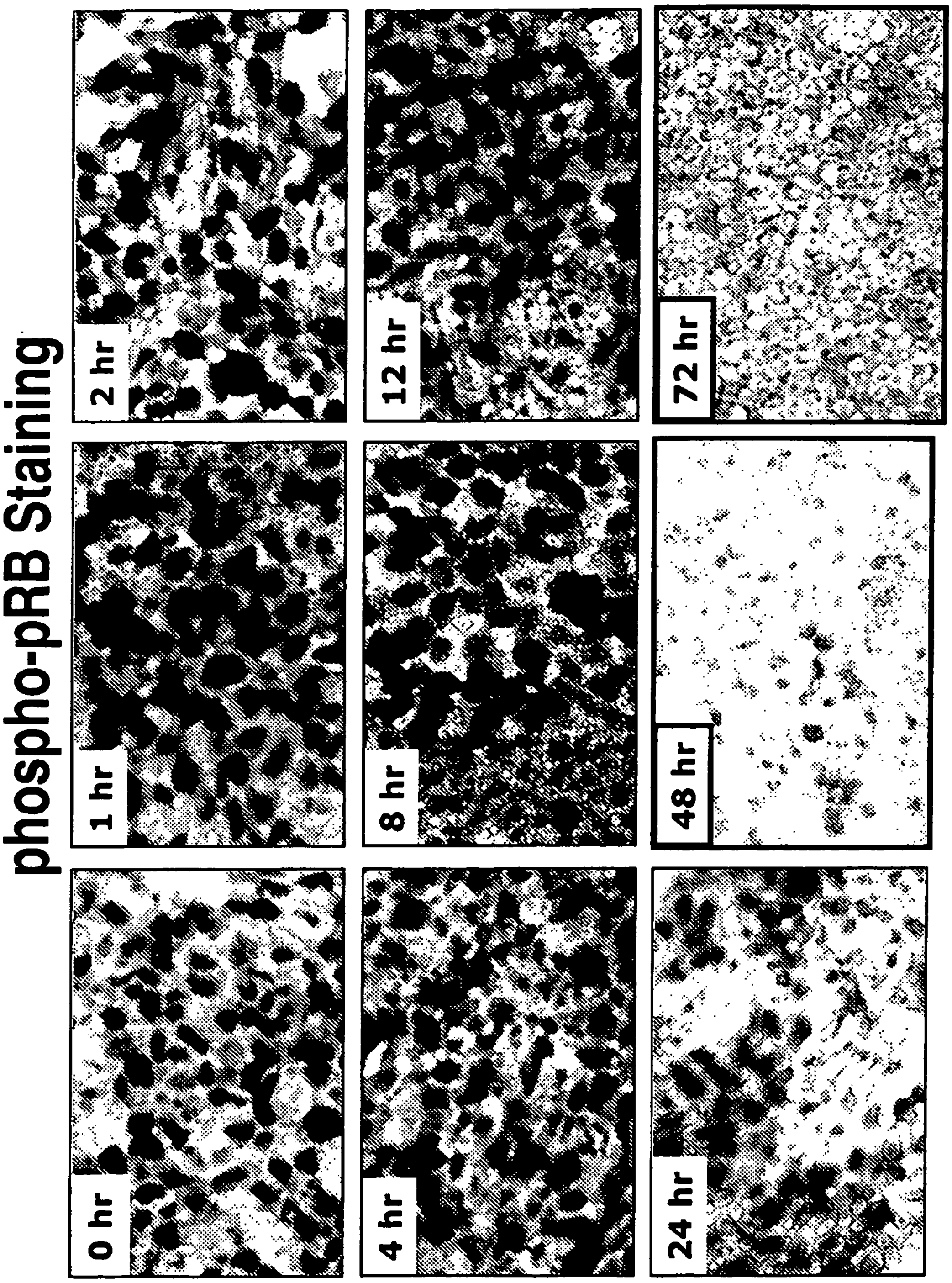


Fig. 9B

Response of s.c. -DBTRG-05MG Glioblastoma
Tumor to Treatment with Compound 106
Mean Tumor Volumes (mm3)

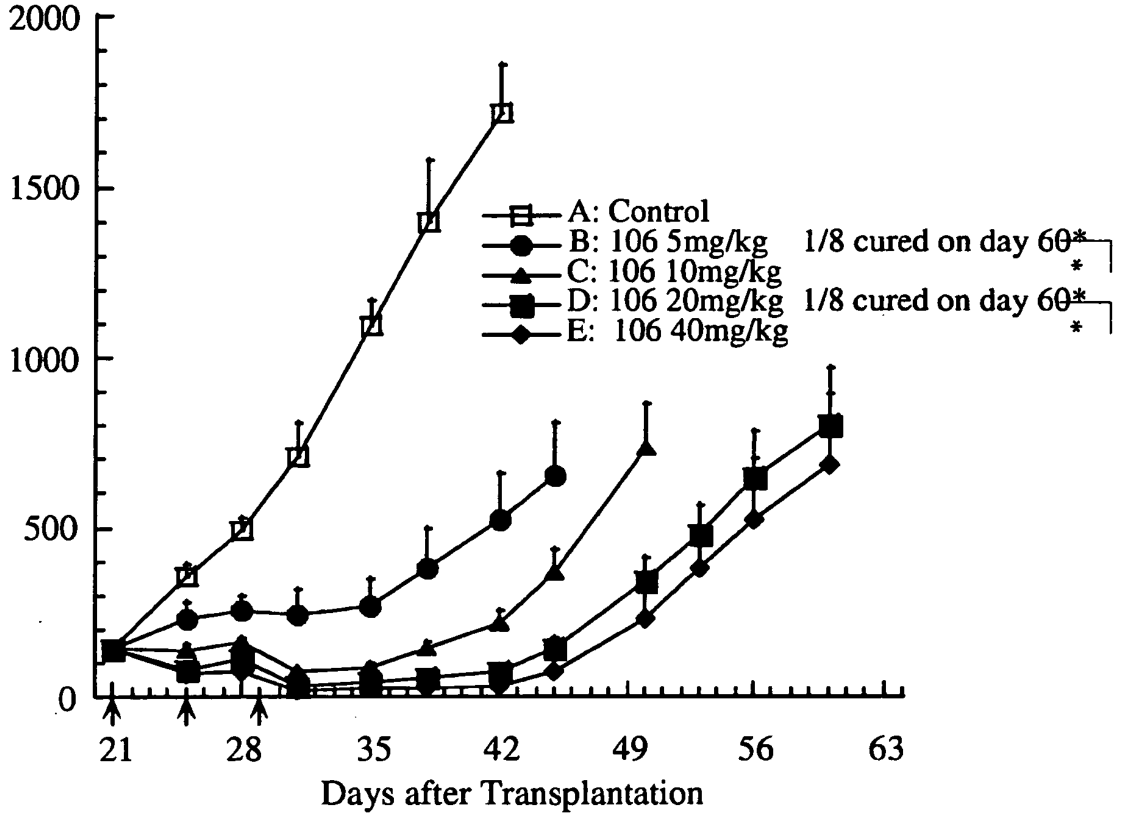


*Fig. 10*

**Response of s.c.-LOX Melanoma Tumor to Treatment with Compound 106**

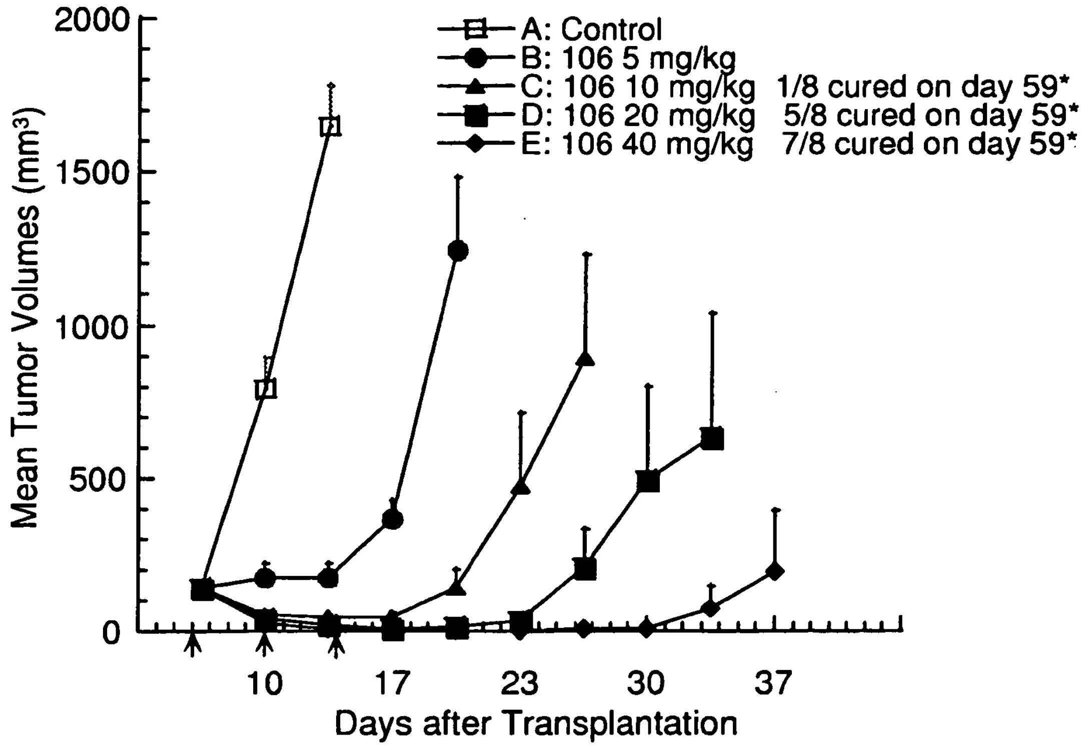


*Fig. 11*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61000796 A
- US 60951906 B
- US 12180408 B
- WO 9504136 A
- EP 0730740 B1
- US 5599681 A
- US 6942987 B
- US 6982318 B
- US 20020150954 A
- US 20070020232 A
- US 5631169 A, Lakowicz
- US 4868103 A, Stavrianopoulos
- US 4843155 A, Chomczynski
- US 4683202 A, Mullis
- US 5854033 A, Lizardi
- US 60951901 B
- US 60951892 B
- US 12180423 B
- US 507067 A
- US 20040224936 A
- GB 323845 A
- EP 606044 A
- WO 0038674 A
- JP 840893 A
- WO 9613259 A
- US 5728726 A
- US 5674892 A
- US 5795910 A
- WO 05023792 A
- WO 03076424 A


### Non-patent literature cited in the description


- **Davies et al.** *Nature,* 2002, vol. 417, 949-954
- **Roberts ; Der.** *Oncogene,* 2007, vol. 26, 3291-3310
- **Guanti et al.** *Human Mol. Gen.,* 2000, vol. 9 (2), 283-287
- **Carnero et al.** *Curr. Cancer Drug Targets,* 2008, vol. 8 (3), 187-98
- **Mirmohammadsadegh et al.** *Cancer Res.,* 2006, vol. 66 (13), 6546-52
- **Hou et al.** *Cancer,* 2008, vol. 113 (9), 2440-7
- **Wallace et al.** *Current Topics in Medicinal Chemistry,* 2005, vol. 5, 215-219
- **Sambrook, J. et al.** Molecular Cloning: A Laboratory Manual. Cold Spring Harbor Laboratory Press, 1989
- **Rodriguez-Viciana et al.** *Science,* 2006, vol. 311, 1287-1290
- **Guanti et al.** *Human Molecular Genetics,* 2000, vol. 9 (2), 283-287
- **Davies et al.** *Nature,* 2002, vol. 417, 949
- *Nature Reviews of Cancer,* 2004, 4
- **Sambrook, J. et al.** Molecular Cloning: A Laboratory Manua. Cold Spring Harbor Laboratory Press, 1989
- **Stokoe et al.** *Science,* 1994, vol. 264, 1463-1467
- **Yoon et al.** *Am. J. Physiol. Renal Physiol.,* 2004, vol. 286, F417-F424
- **Sjolander, S. ; Urbaniczky, C.** *Anal. Chem.,* 1991, vol. 63, 2338-2345
- **Szabo et al.** *Curr. Opin. Struct. Biol.,* 1995, vol. 5, 699-705
- **Rivas, G. ; Minton, A.P.** *Trends Biochem Sci.,* 1993, vol. 18 (8), 284-7
- **Heegaard, N.H.** *J. Mol. Recognit. Winter,* 1998, vol. 11 (1-6), 141-8
- **Hage, D.S. ; Tweed, S.A.** *J Chromatogr B Biomed Sci Appl,* 10 October 1997, vol. 699 (1-2), 499-525
- Current Protocols in Molecular Biology. John Wiley & Sons, 1987
- **Barany.** *Proc. Natl. Acad. Sci. USA,* 1991, vol. 88, 189-193
- **Guatelli et al.** *Proc. Natl. Acad. Sci. USA,* 1990, vol. 87, 1874-1878
- **Kwoh et al.** *Proc. Natl. Acad. Sci. USA,* 1989, vol. 86, 1173-1177
- **Lizardi et al.** *Bio/Technology,* 1988, vol. 6, 1197
- **Harlow ; Lane.** Antibodies: A Laboratory Manual. Cold Spring Harbor Laboratory Press, 1988
- **S.M. Berge et al.** *J. Pharmaceutical Sciences,* 1977, vol. 66, 1-19
- Remington's Pharmaceutical Sciences. Mack Publishing Co, 1980
- **Davies H. et al.** Mutations of the BRAF gene in human cancer. *Nature,* 2002, vol. 417, 949-954